# EUROPEAN PATENT APPLICATION

(11) **EP 3 006 942 A1**
(43) Date of publication of application: **13.04.2016**
(21) Application number: 14804893.7
(22) Date of filing: 29.05.2014
(51) Int. Cl.: G01N 33/574, A61K 39/395, A61K 49/00, C07K 16/28, C12N 15/09

(54) **REAGENT INCLUDING ANTI-LGR6 ANTIBODIES FOR DETECTION AND DIAGNOSIS OF CANCER**

(30) Priority: 30.05.2013 JP 2013114150
(71) Applicant: Order-Made Medical Research Inc., Kashiwa-shi, Chiba 277-0882 (JP)
(72) Inventor: SATOFUKA, Hiroyuki, Chiba 277-0882 (JP); MURAKAMI, Yasufumi, Chiba 277-0882 (JP)
(74) Representative: Tuxworth, Pamela M.
(86) International application number: PCT/JP2014/064902
(87) International publication number: WO 2014/192974

(57) **Abstract**

The object of the present invention is to provide a reagent for detection or diagnosis of cancer.

The present invention provides a reagent for detection or diagnosis of cancer, which comprises a monoclonal antibody against LGR6 or a fragment of the antibody.

## Description

### TECHNICAL FIELD

The present invention relates to a reagent for detection or diagnosis of cancer, which comprises an anti-LGR6 antibody.

### BACKGROUND ART

Cancer ranks high in the causes of death in the world. Above all, colorectal cancer is at a higher position in the mortality of cancer in Europe and North America. The number of colorectal cancer patients has been drastically increasing in recent years, and about 60,000 patients suffer from colorectal cancer every year in Japan. In the number of deaths classified by internal organ, colorectal cancer ranks third after gastric cancer and lung cancer. Likewise, breast cancer is a woman's cancer most commonly seen in the United Kingdom, the United States and Japan. The number of women suffering from breast cancer in Japan has now exceeded 40,000 and still tends to increase.

Colorectal cancer and breast cancer can be cured by surgery before reaching advanced stages. On the other hand, even in unresectable progressive or recurrent cases, treatment outcomes have been greatly improved by advances in drug therapy. However, the average survival period is about two years for progressive or recurrent colorectal cancer cases and is about one and a half years for breast cancer cases.

For cancer diagnosis, it is useful to conduct detection on the basis of body fluid components prior to tissue staining. In the case of colorectal cancer, the diagnostic method based on fecal occult blood reaction is now used widely. This technique is designed to check the presence of human hemoglobin to thereby diagnose the presence or absence of hemorrhage in the intestines and indirectly predict the onset of colorectal cancer.

The fecal occult blood reaction is a test easy to perform and is therefore widely used, although there is a problem in the usefulness of the test. For example, to make a positive diagnosis in the test using Hemoccult II (Astellas Pharma Inc., Japan), this test requires 20 mg hemorrhage per day in the colon and rectum. However, hemorrhage actually caused in colorectal cancer patients is considered to be 10 mg or less. For this reason, the sensitivity of the fecal occult blood reaction is around 26%, so that only approximately 1/4 of actual colorectal cancer patients can be detected and therefore 3/4 of the patients will be missed, as reported (Non-patent Document 1: Jama, Vol. 269, 1262-7, 1993). Further, only 8.3% of the subjects diagnosed as a positive in the test actually have colorectal cancer. Thus, the fecal occult blood reaction is a test method involving many false positive results and therefore still has problems.

In the case of breast cancer, breast X-ray examination (mammography) or ultrasonic diagnosis is conducted. It has been reported that the mortality of breast cancer is 15% to 20% lower in a group continuously receiving mammographic examination than in a group not receiving this examination. On the other hand, recent reports have shown that mammography has no effect in reducing the mortality in women in their forties where women suffering from breast cancer increase in number (Non-patent Document 2: Annals of Internal Medicine, Vol. 137, 305-312, 2002). This is because breast cancer cannot be detected due to high mammary gland density, and it has also been reported that young Japanese women are particularly unsuited to mammographic examination. For this reason, there is a demand for the development of a new diagnostic method.

In cancer diagnosis, when a subject is suspected to have cancer as a result of physical examination, urine analysis, biochemical analysis of blood, blood counting, chest X-ray examination and/or fecal occult blood testing, such a subject will undergo histological examination and immunohistochemical examination using a biopsy sample which is a portion taken from a lump-containing tissue, followed by morphological diagnosis on the stained images. In general, from the obtained tissue, thin sections are prepared and fixed, and then subjected to hematoxylin-eosin staining (HE staining).

In histological observation, disappearance of tissue-constituting basal cells and other events serve as important factors for diagnosing a subject as having cancer. However, basal cells may also be altered in benign lesions, and diagnosis based solely on HE staining involves many difficulties, so that basal cells in atypical lesions are diagnosed mainly by immunohistochemical staining. In immunohistochemical staining, cancer is evaluated by detection of epidermal growth factor receptor (EGFR) for colorectal cancer and epidermal growth factor receptor 2 (Her2/erbB2) for breast cancer (Patent Document 1: JP 2006-519376 A).

However, the frequency of occurrence evaluated by immunohistochemical staining varies among reports from 25% to 77% in colorectal cancer upon detection of EGFR (Non-patent Document 3: Modern Media, Vol. 55, No. 7, 2009 (in Japanese))., In breast cancer, the frequency of occurrence is 15% to 25% upon detection of Her2, which means that a limited percentage of patients can be diagnosed as being positive (Non-patent Document 4: Endocr Relat Cancer, Vol. 9, p. 75-85, 2002). Moreover, both EGFR and Her2 may be stained even in normal tissues, depending on the type of staining; and therefore a strictly normalized method is required to make a positive diagnosis.

Under these circumstances, there is a demand for the development of a new target molecule which is expressed at high rate in cancer patients and allows effect prediction for antibody drugs, and the development of a monoclonal antibody required to detect such a new target molecule.

Leucine-rich repeat containing G protein-coupled receptor 6 (LGR6) is a membrane protein composed of 967 amino acids and has a structure consisting of a seven-transmembrane region and an N-terminal long extracellular region.

LGR6 gene is disclosed as one of the genes whose expression is increased in gastric cancer patients when compared to normal tissues (Non-patent Document 5: Virchows Arch., Vol. 461, p. 355-365, 2012). Moreover, the results are also shown that when 481 cases of gastric cancer were analyzed by tissue arrays using LGR6-recognizing rabbit-derived anti-LGR6 polyclonal antibody (antibodies-online), 153 cases (32%) were found to be LGR6-positive (ibid).

On the other hand, among LGR family proteins, LGR4 and LGR5 are known to have homology with LGR6, although there are many differences between LGR6 and these molecules. Thus, they are recognized to be distinct proteins when used as target molecules for detection purposes, as discussed below.

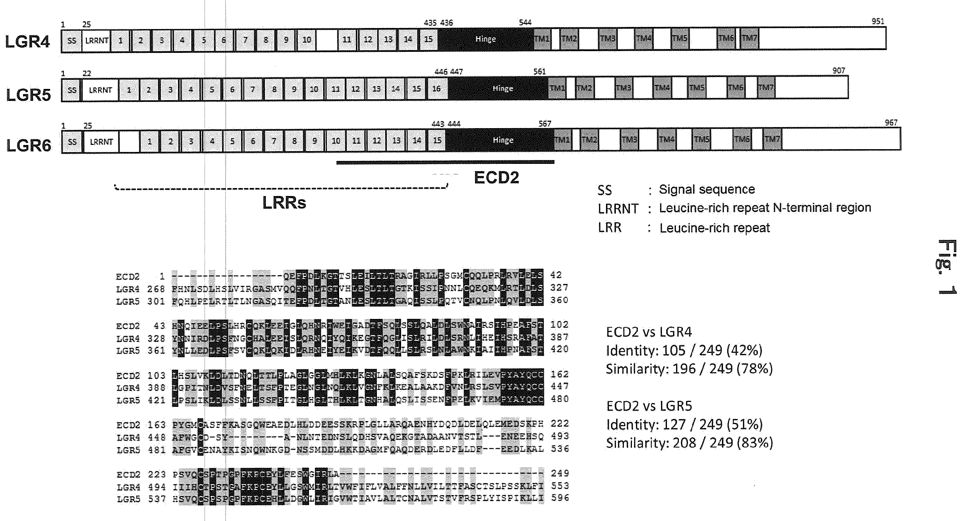

First, the number of leucine-rich repeats (LRRs) is 15 in LGR4 and LGR6, whereas it is 16 in LGR5, so that they differ in their protein structure (Figure 1).

In addition, LGR4, LGR5 and LGR6 bind to R-spondin 1, 2, 3 and 4 to thereby enhance Wnt/β-catenin signals (Non-patent Document 6: PLoS One, Vol. 7, e37137, 2012), but the respective molecules LGR4, LGR5 and LGR6 have different affinities for each of R-spondin 1, 2, 3 and 4. For example, LGR6 is reported to have the highest affinity for R-spondin 2, followed by R-spondin 3, 1 and 4 in this order. Namely, the respective LGR molecules are inferred to also differ in their effect due to differences in their affinity for R-spondin members (Non-patent Document 6: PLoS One, Vol. 7, e37137,2012).

Moreover, as a result of analyzing the expression profiles of LGR4, LGR5 and LGR6 in gastric cancer, the positive rates for LGR4 and LGR6 are shown to be 43% and 32%, respectively (Non-patent Document 5: Virchows Arch., Vol. 461, p. 355-365, 2012). In contrast, LGR5 is shown to be positive in normal gastric stem cells and therefore cannot be used as a marker for gastric cancer. On the other hand, LGR5 is suggested to have a potential to be used as a marker for cancer stem cells in colorectal cancer (Non-patent Document 7: Science, Vol. 337, p. 730-735, 2012). Moreover, it is shown that LGR5 is also expressed in areas deemed to be stem cells in gastric cancer, although its strong expression is not seen in any other cancer areas. In contrast, LGR4 and LGR6 are shown to be expressed throughout cancer areas, including stem cells, in gastric cancer.

Further, LGR4, LGR5 and LGR6 are also reported to greatly differ in their intracellular mRNA levels, depending on the type of human cancer cell line (Non-patent Document 6: PLoS One, Vol. 7, e37137, 2012).

Namely, LGR6 differs from LGR4 and LGR5 in terms of expression patterns in cancers.

In addition, the homology (identity) in the full-length amino acid sequence is 55% between LGR6 and LGR4, and is 49% between LGR6 and LGR5. Likewise, the homology in ECD2 including a hinge region is 42% between LGR6 and LGR4, and is 51% between LGR6 and LGR5. Namely, the homology between LGR6 and LGR4 or LGR5 cannot be regarded as being high.

Thus, LGR6 is a protein completely distinct as a target molecule from LGR4 and LGR5.

JP 2010-532169 A (Patent Document 2) discloses a monoclonal antibody specifically binding to the extracellular domain of human LGR protein. This document also shows that the antibody disrupts the binding of R-spondin 1, 2, 3 and 4 proteins to LGR protein and/or disrupts the R-spondin-mediated activation of LGR signaling. Further, this document discloses LGR4, LGR5 and LGR6 as members of the LGR protein.

However, it is only LGR5 and antibodies against LGR5 for which analysis results are shown in the above document, and no analysis results are shown for LGR6, nor is there any description showing that an antibody against LGR6 was obtained. As described above, LGR6 is a protein completely distinct as a target molecule from LGR4 and LGR5; and hence the structure, properties, functions and others of an antibody against LGR6 are not obvious from the descriptions in the above document.

Moreover, evaluations conducted in the above document are limited only to some cancer cases, i.e., analysis using tumorigenic (TG) colon cancer cells isolated from human tumor and expression analysis on 3 cases of colon tumor. Namely, the above document does not mention that LGR6 is overexpressed in other cancers, e.g., in colorectal cancer and breast cancer.

As antibodies against LGR6, polyclonal antibodies are provided from several manufacturers. Commonly known examples include LS-A621 (LifeSpan BioSciences), ABIN122207 (GenWay Biotech) and NBP1-49696 (Novus Biological), each being prepared by using the N-terminal segment of LGR6 as an antigen. Other known examples include H00059352-A01 prepared by using amino acid residues 445-504 of LGR6 as an antigen (Abnova Corporation), an antibody prepared by using amino acid residues 456-548 as an antigen (Novus Biologicalsh), and L4169 prepared by using amino acid residues 516-528 as an antigen (Sigma-Aldrich).

As a monoclonal antibody binding to LGR6, EPR6874 (GenTax) is known as a rabbit monoclonal antibody. However, because of being prepared by using an intracellular domain as an antigen, this antibody is difficult to use for detection of living cancer cells. Moreover, in the case of immunostaining, fixing of cells and permeation treatment of their cell membrane will cause denaturation of the LGR6 protein, so that the detection sensitivity of the antibody will be reduced.

To use an antibody as a tool for target protein detection or disease diagnosis, a monoclonal antibody is desired for this purpose because it is important to ensure continuous and uniform production and provision. In general, a membrane protein such as LGR6, particularly a multi-transmembrane protein is difficult to solubilize, and it is difficult to derive a high titer antibody against such a membrane protein due to its high amino acid sequence homology among mammals. For these reasons, it is difficult to prepare an antibody which allows detection of a membrane protein with high sensitivity. Even when polyclonal antibodies are obtained from, e.g., the peripheral blood of immunized animals, the probability of successfully isolating antibody-producing cells is very low under the present circumstances.

### DISCLOSURE OF THE INVENTION

The present invention has been made under these circumstances, and the problem to be solved by the present invention is to provide an antibody which allows detection of cancer with high sensitivity, and a reagent for detection or diagnosis of cancer, which comprises such an antibody.

As a result of extensive and intensive efforts made to solve the above problem, the inventors of the present invention have succeeded in detecting cancer with higher sensitivity by using an antibody binding to LGR6, particularly the extracellular region of LGR6, when compared to conventional antibodies. This has led to the completion of the present invention.

Namely, the present invention is as follows.
(1) A reagent for detection or diagnosis of cancer, which comprises a monoclonal antibody against leucine-rich repeat containing G protein-coupled receptor 6 (LGR6) or a fragment of the antibody.
(2) The reagent according to (1) above, wherein the antibody or fragment thereof binds to the extracellular region of leucine-rich repeat containing G protein-coupled receptor 6 (LGR6).
(3) The reagent according to (2) above, wherein the extracellular region comprises the amino acid sequence shown in SEQ ID NO: 7, 8, 9 or 10.
(4) The reagent according to (1) above, wherein the cancer is at least one selected from the group consisting of colorectal cancer, breast cancer, uterine cancer, gastric cancer, thyroid cancer, pancreatic cancer, brain tumor, cervical cancer, esophageal cancer, tongue cancer, lung cancer, small intestinal cancer, duodenal cancer, bladder cancer, kidney cancer, liver cancer, prostate cancer, uterine cervical cancer, ovarian cancer, gallbladder cancer, pharyngeal cancer, sarcoma, melanoma, leukemia, lymphoma and multiple myeloma.
(5) The reagent according to (1) above, wherein the cancer is at least one selected from the group consisting of colorectal cancer, breast cancer, uterine cancer, gastric cancer, thyroid cancer, pancreatic cancer and leukemia.
(6) The reagent according to (1) above, wherein the cancer is colorectal cancer or breast cancer.
(7) A method for detection of cancer, which comprises the step of contacting a monoclonal antibody against leucine-rich repeat containing G protein-coupled receptor 6 (LGR6) or a fragment of the antibody with a biological sample taken from a subject to thereby detect LGR6 in the sample.
(8) A kit for detection of cancer, which comprises a monoclonal antibody against leucine-rich repeat containing G protein-coupled receptor 6 (LGR6) or a fragment of the antibody.
(9) A method for diagnosis of cancer, which comprises the step of contacting a monoclonal antibody against leucine-rich repeat containing G protein-coupled receptor 6 (LGR6) or a fragment of the antibody with a biological sample taken from a subject, and detecting LGR6 in the sample.
(10) A monoclonal antibody against leucine-rich repeat containing G protein-coupled receptor 6 (LGR6) or a fragment of the antibody for use in the detection or diagnosis of cancer.

The present invention allows detection of cancer with higher sensitivity when compared to conventional antibodies.

### BRIEF DESCRIPTION OF THE DRAWINGS

Figure 1 shows a structural comparison of LGR4, LGR5 and LGR6, along with an alignment of their partial regions.
Figure 2 shows the results obtained when antibodies reactive to the immunized antigen (ECD2) were evaluated by ELISA.
Figure 3 shows the results obtained when the antibodies produced by the established hybridomas were evaluated by ELISA.
Figure 4 shows the results obtained when the antibodies produced by the established hybridomas were evaluated by Western blotting.
Figure 5 shows the results obtained when HT29 cells were subjected to immunocytological staining with the antibodies produced by the established hybridomas.
Figure 6 shows the results obtained when culture supernatants containing ECD2-reactive antibodies were used and evaluated in a flow cytometer.
Figure 7 shows the results obtained when Mu2C 15 antibody was purified with Protein G and analyzed for its reactivity to HT29 cells in a flow cytometer.
Figure 8A shows the results obtained when human colorectal cancer tissues were immunostained with the antibody produced by hybridoma cells under clone No. Mu2C15.
Figure 8B is a graph showing the results of statistical analysis on the degree of staining in immunohistological staining when ranked as strongly positive (Strong), positive (Moderate), weakly positive (Weak) or negative (Negative).
Figure 9 shows the results obtained when human breast cancer tissues were immunostained with the antibody produced by hybridoma cells under clone No. Mu2C15.
Figure 10A shows the results obtained when breast cancer tissues at different stages were immunostained with clone No. Mu2C15 and HercepTest II.
Figure 10B is a graph showing the results of statistical analysis on the degree of staining in immunohistological staining with HercepTest II. The results of determination are expressed as "3+," "2+," "1+" and "0" in the order from strong to weak signals.
Figure 10C is a graph showing the results of statistical analysis on the degree of staining in immunohistological staining with clone No. Mu2C15. The results of determination are expressed as "3+," "2+," "1+" and "0" in the order from strong to weak signals.
Figure 11 shows an affinity comparison between known monoclonal antibody (clone No. 2A3) binding to the extracellular domain of LGR6 and clone No. Mu2C15.
Figure 12 shows the results analyzed for combinations of antibodies allowing detection of the antigen ECD2 by sandwich ELISA. "-" denotes an absorbance of 0.2 to 0.3, "+" denotes an absorbance of 0.3 to 0.45, "++" denotes an absorbance of 0.45 to 0.8, and "+++" denotes an absorbance of 0.8 or more.
Figure 13 shows a standard curve obtained when ECD2 was detected using Mu2C 15 as an immobilized antibody and Mu2C21 as a detection antibody.
Figure 14A shows the nucleotide sequence and amino acid sequence of the heavy chain variable region (VH) in Mu2C15 antibody, along with the positions of the signal sequence, CDR1, CDR2 and CDR3.
Figure 14B shows the nucleotide sequence and amino acid sequence of the light chain variable region (VL) in Mu2C15 antibody, along with the positions of the signal sequence, CDR1, CDR2 and CDR3.
Figure 15 shows the results compared for the reactivity of Mu2C 15 antibody to partial peptides of LGR4, LGR5 and LGR6.

### BEST MODES FOR CARRYING OUT THE INVENTION

The present invention will be described in more detail below. The following embodiments are illustrated to describe the present invention, and it is not intended to limit the present invention only to these embodiments. The present invention can be implemented in various modes, without departing from the spirit of the present invention. Moreover, this specification incorporates the contents disclosed in the specification and drawings of Japanese Patent Application No. 2013-114150 (filed on May 30, 2013), based on which the present application claims priority.

### 1. Summary

The present invention relates to a reagent for detection or diagnosis of cancer, which comprises a monoclonal antibody against leucine-rich repeat containing G protein-coupled receptor 6 (LGR6) or a fragment of the antibody.

As a result of extensive and intensive efforts made with a focus on LGR6 which is a transmembrane protein, the inventors of the present invention have found that LGR6 is overexpressed in cancer, particularly in colorectal cancer and breast cancer. Moreover, the inventors of the present invention have succeeded in preparing a monoclonal antibody against LGR6, which allows detection of cancer with extremely high sensitivity and high accuracy when compared to conventionally known antibodies. The antibody of the present invention allows detection of cancer with high sensitivity even at an early stage of cancer progression and also allows detection of living cancer cells because of being capable of binding to the extracellular region of LGR6, which in turn allows detection of cancer cells present in blood samples, by way of example. Namely, when using the antibody of the present invention, cancers which have been undetectable by conventional methods can be detected in a simple manner with high sensitivity and high accuracy, so that the antibody of the present invention is very useful for detection or diagnosis of cancer. The present invention has been completed on the basis of such findings.

### 2. Antibody against LGR6 (anti-LGR6 antibody)

### (1) Antigen preparation

LGR6 intended in the present invention may be derived from any mammal, and examples of such a mammal include mice, rats, rabbits, goats, monkeys and humans, with mice, rats and humans being preferred, and with humans being more preferred.

In the present invention, the amino acid sequences of mouse, rat and human LGR6 are shown in SEQ ID NOs: 2, 4 and 6, respectively. Likewise, the nucleotide sequences of DNAs encoding mouse, rat and human LGR6 are shown in SEQ ID NOs: 1, 3 and 5, respectively. These amino acid sequences and nucleotide sequences have been registered in the GenBank database under the accession numbers (Accession Nos.) given to the respective sequences.
Amino acid sequence of mouse LGR6: NP_001028581.1 (SEQ ID NO: 2)
Amino acid sequence of rat LGR6: XP_001062538.1 (SEQ ID NO: 4)
Amino acid sequence of human LGR6: NP_001017403.1 (SEQ ID NO: 6)
Nucleotide sequence of DNA encoding mouse LGR6: NM_001033409.3 (SEQ ID NO: 1)
Nucleotide sequence of DNA encoding rat LGR6: XM_001062538.3 (SEQ ID NO: 3)
Nucleotide sequence of DNA encoding human LGR6: NM_001017403.1 (SEQ ID NO: 5)

As an antigen, it is possible to use a polypeptide or peptide (also collectively referred to as a peptide) comprising at least a part (whole or a part) of the amino acid sequence of LGR6, preferably a peptide comprising at least a part (whole or a part) of the amino acid sequence of the extracellular region of LGR6.

The extracellular region of LGR6 refers to a region covering leucine-rich repeats (LRRs) and a hinge region connecting LRRs to transmembrane domains. Leucine-rich repeats refer to a structure in which a region composed of about 25 amino acid residues rich in leucine is located repeatedly. In the case of LGR6, the number of repeats is 15.

For example, in the case of human LGR6, the extracellular region of LGR6 corresponds to a region (SEQ ID NO: 7) consisting of amino acids at positions 25 to 567 in the amino acid sequence shown in SEQ ID NO: 6, LRRs correspond to a region (SEQ ID NO: 8) consisting of amino acids at positions 91 to 443 in the amino acid sequence shown in SEQ ID NO: 6, and the hinge region corresponds to a region (SEQ ID NO: 10) consisting of amino acids at positions 444 to 567 in the amino acid sequence shown in SEQ ID NO: 6 (Figure 1). In the case of LGR6 derived from other animals, regions corresponding to these regions are intended.

The peptide to be used as an antigen is not limited in any way as long as it is at least a part of LGR6. Preferred is at least a part of the extracellular region of LGR6, e.g., at least a part of a region consisting of amino acids at positions 25 to 567 (SEQ ID NO: 7), a region consisting of amino acids at positions 91 to 567 (SEQ ID NO: 8), a region consisting of amino acids at positions 319 to 567 (SEQ ID NO: 9) or a region consisting of amino acids at positions 444 to 567 (SEQ ID NO: 10) in the amino acid sequence of LGR6 shown in SEQ ID NO: 6. Among them, more preferred is at least a part of the region consisting of amino acids at positions 319 to 567 or the region consisting of amino acids at positions 444 to 567, and particularly preferred is at least a part of the region consisting of amino acids at positions 319 to 567. In the present invention, a protein consisting of amino acids at positions 319 to 567 in the amino acid sequence of LGR6 shown in SEQ ID NO: 6 is also referred to as "ECD2."

Moreover, LGR6 has mutants which are derived from the same mRNA but differ in their translation initiation site. For example, human LGR6 has mutants such as a peptide (SEQ ID NO: 11) lacking N-terminal 43 amino acids and having different amino acids at positions 44 to 71 from those of the wild-type (SEQ ID NO: 6), a peptide (SEQ ID NO: 12) lacking N-terminal 52 amino acids and having different amino acids at positions 53 to 70 from those of the wild-type (SEQ ID NO: 6), etc.

LGR6 intended in the present invention also includes these mutants.

LGR6 may be naturally occurring LGR6 purified from mouse, rat, human or other animal tissues or cells, or alternatively, may be genetically engineered LGR6. For example, a biological sample known to contain LGR6 may be fractionated into a soluble fraction and an insoluble fraction by using any type of surfactant such as Triton-X, Sarkosyl, etc. The insoluble fraction may further be dissolved in, e.g., urea or guanidine hydrochloride and then bound to any type of column such as a heparin column or crosslinked resin to thereby obtain LGR6.

Alternatively, the peptide to be used as an antigen may be prepared by chemical synthesis or by synthesis through genetic engineering procedures using *E. coli* or the like. Techniques well known to those skilled in the art may be used for this purpose.

For chemical synthesis, the peptide may be synthesized by well-known peptide synthesis techniques. Moreover, the synthesis may be accomplished by applying either solid phase synthesis or liquid phase synthesis. A commercially available peptide synthesizer (e.g., PSSM-8, Shimadzu Corporation, Japan) may also be used for this purpose.

For peptide synthesis through genetic engineering procedures, DNA encoding the peptide is first designed and synthesized. The design and synthesis may be accomplished, for example, by PCR techniques using a vector or the like containing the full-length LGR6 gene as a template and using primers which have been designed to allow synthesis of a desired DNA region. Then, the above DNA may be ligated to an appropriate vector to obtain a recombinant vector for protein expression, and this recombinant vector may be introduced into a host, such that a desired gene can be expressed therein, thereby obtaining a transformant (Sambrook J. et al., Molecular Cloning, A Laboratory Manual, 3rd edition, Cold Spring Harbor Laboratory Press, 2001).

As a vector, a phage or plasmid which is autonomously replicable in host microorganisms is used. Further, it is also possible to use an animal virus or insect virus vector. To prepare a recombinant vector, purified DNA may be cleaved with an appropriate restriction enzyme and ligated to a vector by being inserted into, e.g., an appropriate restriction enzyme site in the vector DNA. There is no particular limitation on the host for use in transformation as long as it is capable of expressing a desired gene. Examples include bacteria (e.g., *E. coli, Bacillus subtilis*), yeast, animal cells (e.g., COS cells, CHO cells), insect cells or insects. It is also possible to use a mammal (e.g., goat) as a host. Procedures for introduction of a recombinant vector into a host are known.

Moreover, the above transformant may be cultured, and a peptide for use as an antigen may be collected from the cultured product. The term "cultured product" is intended to mean either (a) a culture supernatant or (b) cultured cells or cultured microorganisms or a homogenate thereof.

After culture, when the desired peptide is produced within microorganisms or cells, the microorganisms or cells may be homogenized to thereby extract the peptide. Alternatively, when the desired peptide is produced outside microorganisms or cells, the cultured solution may be used directly or treated by centrifugation or other techniques to remove the microorganisms or cells. Then, the desired peptide may be isolated and purified by biochemical techniques commonly used for isolation and purification of peptides, as exemplified by ammonium sulfate precipitation, gel filtration, ion exchange chromatography, affinity chromatography and so on, which may be used either alone or in combination as appropriate.

In the present invention, the peptide to be used as an antigen may also be obtained by *in vitro* translation using a cell-free synthesis system. In this case, it is possible to use two methods, i.e., a method in which RNA is used as a template and a method in which DNA is used as a template (transcription/translation). As a cell-free synthesis system, a commercially available system may be used, as exemplified by an Expressway^{™} system (Invitrogen), etc.

The peptide obtained as described above may also be linked to an appropriate carrier protein such as bovine serum albumin (BSA), keyhole limpet hemocyanin (KLH), human thyroglobulin, avian gamma globulin, etc.

Moreover, the antigen intended in the present invention encompasses not only (a) a polypeptide comprising the amino acid sequence shown in SEQ ID NO: 2, 4, 6, 7, 8, 9, 10, 11 or 12, but also (b) a polypeptide comprising an amino acid sequence with deletion, substitution or addition of one or several amino acids in the amino acid sequence shown in SEQ ID NO: 2, 4, 6, 7, 8, 9, 10, 11 or 12 and (c) a polypeptide comprising an amino acid sequence sharing a homology of 70% or more with the amino acid sequence shown in SEQ ID NO: 2, 4, 6, 7, 8, 9, 10, 11 or 12.

In the context of the present invention, the "polypeptide comprising the amino acid sequence shown in SEQ ID NO: 2, 4, 6, 7, 8, 9, 10, 11 or 12" includes a polypeptide consisting of the amino acid sequence shown in SEQ ID NO: 2, 4, 6, 7, 8, 9, 10, 11 or 12.

Moreover, the "amino acid sequence with deletion, substitution or addition of one or several amino acids in the amino acid sequence shown in SEQ ID NO: 2, 4, 6, 7, 8, 9, 10, 11 or 12" includes, for example:
(i) an amino acid sequence with deletion of 1 to 10 amino acids (e.g., 1 to 5 amino acids, preferably 1 to 3 amino acids, more preferably 1 or 2 amino acids, even more preferably a single amino acid) in the amino acid sequence shown in SEQ ID NO: 2, 4, 6, 7, 8, 9, 10, 11 or 12;
(ii) an amino acid sequence with substitution of other amino acids for 1 to 10 amino acids (e.g., 1 to 5 amino acids, preferably 1 to 3 amino acids, more preferably 1 or 2 amino acids, even more preferably a single amino acid) in the amino acid sequence shown in SEQ ID NO: 2, 4, 6, 7, 8, 9, 10, 11 or 12;
(iii) an amino acid sequence with addition of 1 to 10 amino acids (e.g., 1 to 5 amino acids, preferably 1 to 3 amino acids, more preferably 1 or 2 amino acids, even more preferably a single amino acid) to the amino acid sequence shown in SEQ ID NO: 2, 4, 6, 7, 8, 9, 10, 11 or 12; and
(iv) an amino acid sequence mutated by any combination of (i) to (iii) above.

In addition, LGR6 intended in the present invention encompasses not only a polypeptide comprising the amino acid sequence shown in SEQ ID NO: 2, 4, 6, 7, 8, 9, 10, 11 or 12, but also a polypeptide comprising an amino acid sequence sharing a homology (identity) of 70% or more with the amino acid sequence shown in SEQ ID NO: 2, 4, 6, 7, 8, 9, 10, 11 or 12. Such a polypeptide also includes those comprising amino acid sequences sharing a homology of about 70% or more, 75% or more, about 80% or more, 85% or more, 90% or more, 95% or more, 96% or more, 97% or more, 98% or more, or 99% or more with the amino acid sequence shown in SEQ ID NO: 2, 4, 6, 7, 8, 9, 10, 11 or 12 (i.e., amino acid sequences substantially equivalent to the amino acid sequence shown in SEQ ID NO: 2, 4, 6, 7, 8, 9, 10, 11 or 12). For homology determination, it is possible to use homology search tools such as FASTA, BLAST, PSI-BLAST and so on in a homology search site on the Internet (e.g., DNA Data Bank of Japan (DDBJ)). Alternatively, it is also possible to conduct a BLAST search in the National Center for Biotechnology Information (NCBI).

To prepare the above mutation-carrying proteins, mutations may be introduced into a gene (DNA) encoding the protein by using a kit for mutation introduction based on site-directed mutagenesis (e.g., Kunkel method or Gapped duplex method), as exemplified by a QuikChange^{™} Site-Directed Mutagenesis Kit (Stratagene), GeneTailor^{™} Site-Directed Mutagenesis Systems (Invitrogen), TaKaRa Site-Directed Mutagenesis Systems (e.g., Mutan-K, Mutan-Super Express Km; Takara Bio Inc., Japan). Alternatively, it is also possible to use techniques for site-directed mutagenesis as described in "Molecular Cloning, A Laboratory Manual (4th edition)" (Cold Spring Harbor Laboratory Press (2012)), etc.

In the present invention, the gene to be introduced into cells or the like may be a gene encoding LGR6 or a partial fragment thereof or a mutated peptide thereof. As such a gene, it is possible to use a gene comprising the nucleotide sequence shown in SEQ ID NO: 1, 3 or 5, by way of example.

Alternatively, the gene to be introduced into cells or the like may also be a gene comprising a nucleotide sequence or a partial sequence thereof, which is hybridizable under stringent conditions with a sequence complementary to the nucleotide sequence shown in SEQ ID NO: 1, 3 or 5.

In the context of the present invention, the term "stringent conditions" may be any of low stringent conditions, moderately stringent conditions and high stringent conditions. "Low stringent conditions" refer to, for example, conditions of 5 × SSC, 5 × Denhardt's solution, 0.5% SDS, 50% formamide and 32°C. Likewise, "moderately stringent conditions" refer to, for example, conditions of 5 × SSC, 5 × Denhardt's solution, 0.5% SDS, 50% formamide and 42°C. "High stringent conditions" refer to, for example, conditions of 5 × SSC, 5 × Denhardt's solution, 0.5% SDS, 50% formamide and 50°C. Under these conditions, it can be expected that DNA having a higher homology is more efficiently obtained at a higher temperature. However, the stringency of hybridization would be affected by a plurality of factors, including temperature, probe concentration, probe length, ionic strength, reaction time, salt concentration and so on. Those skilled in the art would be able to achieve the same stringency by selecting these factors as appropriate.

Moreover, the gene encoding LGR6 for use in the present invention also encompasses a gene comprising a nucleotide sequence sharing a homology of 60% or more with the nucleotide sequence shown in SEQ ID NO: 1, 3 or 5. Such a gene may be exemplified by a gene sharing a homology of 60% or more, 70% or more, 80% or more, 85% or more, 90% or more, 95% or more, 96% or more, 97% or more, 98% or more, or 99% or more with the nucleotide sequence shown in SEQ ID NO: 1, 3 or 5, as calculated by homology search software such as FASTA or BLAST using default parameters.

The above gene encoding LGR6 or a mutant thereof can be obtained, for example, on the basis of the nucleotide sequences found in the NCBI GenBank database by using known genetic engineering procedures as described in "Molecular Cloning, A Laboratory Manual (4th edition)" (Cold Spring Harbor Laboratory Press (2012)), etc.

### (2) Preparation of polyclonal antibodies

The thus prepared LGR6 or partial peptide thereof is administered directly or together with a carrier or diluent to immunize non-human mammals (e.g., rabbits, dogs, guinea pigs, mice, rats, goats). The amount of an antigen to be administered per animal is 1 µg to 10 mg in the case of using an adjuvant. Examples of an adjuvant include Freund's complete adjuvant (FCA), Freund's incomplete adjuvant (FIA), aluminum hydroxide adjuvant and so on. Immunization is accomplished primarily by injection via the intravenous, subcutaneous or intraperitoneal route, etc. Moreover, the interval between immunizations is not limited in any way, and immunization may be repeated twice to 20 times, preferably 5 to 15 times, at intervals of several days to several weeks, preferably at intervals of 1 or 2 weeks. Those skilled in the art would be able to determine the interval between immunizations in consideration of the resulting antibody titers. Preferably, at the time of repeating subcutaneous immunization 3 to 4 times, blood is sampled and measured for antibody titers. Antibody titers in serum may be measured by ELISA (enzyme-linked immunosorbent assay), EIA (enzyme immunoassay), radioimmunoassay (RIA), etc. After antibody titers have been confirmed to rise sufficiently, blood may be collected completely and treated in a commonly used manner to separate and purify antibodies. For separation and purification, known techniques such as salting out with ammonium sulfate, ion exchange chromatography, gel filtration chromatography, affinity chromatography and so on may be selected as appropriate or used in combination. More specifically, serum containing desired antibodies may be passed through a column on which proteins other than LGR6 have been immobilized, and fractions passing through the column may be collected to thereby obtain polyclonal antibodies with improved specificity to LGR6.

### (3) Preparation of monoclonal antibodies

### (i) Collection of antibody-producing cells

As in the case of polyclonal antibody preparation, LGR6 or a partial peptide thereof (e.g., ECD2) is administered directly or together with a carrier or diluent to immunize non-human mammals. The amount of an antigen to be administered per animal, the type of adjuvant to be used, the method of immunization and the interval between immunizations are the same as those used for preparation of polyclonal antibodies. After 1 to 30 days, preferably 2 to 5 days, from the final immunization date, animals showing antibody titers may be selected to collect antibody-producing cells. Antibody-producing cells may be exemplified by spleen cells, lymph node cells, peripheral blood cells and so on, with spleen cells or lymph node cells being preferred.

### (ii) Cell fusion

To obtain hybridomas, cell fusion is conducted between antibody-producing cells and myeloma cells. Operations for cell fusion may be accomplished in a known manner, for example, according to the method of Kohler et al. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells. Examples of myeloma cells include mouse myeloma cell lines (e.g., P3X63-Ag8, P3X63-Ag8U.1, SP2/O-Ag14, PAI, P3U1, NSI/1-Ag4-1, NSO/1) and rat myeloma cell lines (e.g., YB2/0), etc.

Cell fusion between the above myeloma cells and antibody-producing cells may be accomplished as follows: in a serum-free medium for animal cell culture (e.g., DMEM or RPMI-1640 medium), 1 × 10⁸ to 5 × 10⁸ antibody-producing cells may be mixed with 2 × 10⁷ to 10 × 10⁷ myeloma cells (the ratio of antibody-producing cells to myeloma cells is 10:1 to 1:1) to cause fusion reaction in the presence of a cell fusion promoter. As a cell fusion promoter, it is possible to use polyethylene glycol having an average molecular weight of 1000 to 6000 daltons or Sendai virus, etc. Alternatively, a commercially available cell fusion apparatus using electrical stimulation (e.g., electroporation) may also be used to cause cell fusion between antibody-producing cells and myeloma cells.

### (iii) Screening and cloning of hybridomas

After cell fusion, the cells are screened to select desired hybridomas. For screening, a cell suspension may be diluted as appropriate with, e.g., RPMI-1640 medium containing 10% to 20% fetal bovine serum and then seeded by limiting dilution in microtiter plates at a density of about 0.3 cells/well by calculation, and a selective medium (e.g., HAT medium) may be added to each well, followed by culture while replacing the selective medium as appropriate. As a result, cells growing at around 10 days after the initiation of culture in the selective medium may be obtained as hybridomas.

Subsequently, the growing hybridomas are further screened. Screening of these hybridomas is not limited in any way and may be conducted in accordance with commonly used procedures. For example, aliquots of the culture supernatants contained in the wells where hybridomas have been cultured may be sampled and screened by enzyme immunoassay, radioimmunoassay, etc. More specifically, an antigen is adsorbed to 96-well plates, and the plates are then blocked with calf serum, skimmed milk, etc. The culture supernatants of hybridoma cells are reacted with the immobilized antigen at 37°C for 1 hour and then reacted with peroxidase labeled anti-mouse IgG at 37°C for 1 hour, followed by color development using orthophenylenediamine as a substrate. After the reaction is stopped with an acid, the plates are measured for absorbance at a wavelength of 490 nm for screening purposes. Monoclonal antibody-producing hybridomas found to be positive when measured in the above manner are cloned by limiting dilution or other techniques to thereby finally establish hybridomas which are cells producing monoclonal antibodies specifically binding to LGR6.

Cell lines (hybridomas) producing the monoclonal antibodies of the present invention may be exemplified by "Mouse-Mouse hybridoma Mu2C15" (hereinafter referred to as "Mu2C15"), "Mouse-Mouse hybridoma Mu2C21" (hereinafter referred to as "Mu2C21") and "Mouse-Mouse hybridoma Mu2C22" (hereinafter referred to as "Mu2C22"). Upon culture of these hybridomas, homogeneous monoclonal antibodies can be prepared.

### (iv) Collection of monoclonal antibodies

For collection of monoclonal antibodies from the establish hybridomas, commonly used procedures, e.g., cell culture-based procedures or ascites formation procedures may be used for this purpose. In cell culture-based procedures, hybridomas are cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days, and antibodies are obtained from their culture supernatants. In the case of ascites formation procedures, the hybridomas are intraperitoneally administered at about 5 × 10⁶ to 2 × 10⁷ cells to animals of the same species as the mammal from which myeloma cells are derived, e.g., mice (BALB/c), whereby the hybridomas are allowed to grow in abundance. Then, their ascites are collected after 1 to 2 weeks. In cases where antibodies are required to be purified in the above antibody collection procedures, known techniques such as salting out with ammonium sulfate, ion exchange chromatography, gel filtration, affinity chromatography and so on may be selected as appropriate or used in combination for purification purposes.

A preferred example of the anti-LGR6 antibody of the present invention is an antibody in which the amino acid sequences of complementarity determining regions (CDRs) 1 to 3 in the heavy chain variable region (VH) comprise or consist of the amino acid sequences shown in SEQ ID NOs: 27, 29 and 31, respectively, and/or the amino acid sequences of complementarity determining regions (CDRs) 1 to 3 in the light chain variable region (VL) comprise or consist of the amino acid sequences shown in SEQ ID NOs: 33, 35 and 37, respectively. In another embodiment, a preferred example of the anti-LGR6 antibody of the present invention is an antibody in which the amino acid sequence of the heavy chain variable region (VH) comprises or consists of the amino acid sequence shown in SEQ ID NO: 23, and/or the amino acid sequence of the light chain variable region (VL) comprises or consists of the amino acid sequence shown in SEQ ID NO: 25.

### (v) Epitope for anti-LGR6 antibody

The epitope (antigenic determinant) for the anti-LGR6 antibody of the present invention is not limited in any way as long as it is at least a part of the antigen LGR6, but it is preferably at least a part of the extracellular region of LGR6, for example, at least a part of a region consisting of amino acids at positions 25 to 567 (SEQ ID NO: 7), a region consisting of amino acids at positions 91 to 567 (SEQ ID NO: 8), a region consisting of amino acids at positions 319 to 567 (SEQ ID NO: 9) or a region consisting of amino acids at positions 444 to 567 (SEQ ID NO: 10) in the amino acid sequence of LGR6 shown in SEQ ID NO: 6. Among them, preferred is at least a part of the region consisting of amino acids at positions 319 to 567 or the region consisting of amino acids at positions 444 to 567, and particularly preferred is at least a part of the region consisting of amino acids at positions 319 to 567. Anti-LGR6 antibody recognizing such a region (binding to such a region) is, for example, very useful for use in detection and diagnosis of cancer as described later because of ensuring high detection sensitivity for LGR6 in biological samples.

Moreover, as described above, LGR has two deletion mutants. More specifically, human LGR6 has deletion mutants such as a peptide (SEQ ID NO: 11) lacking N-terminal 43 amino acids and having different amino acids at positions 44 to 71 from those of the wild-type (SEQ ID NO: 6), a peptide (SEQ ID NO: 12) lacking N-terminal 52 amino acids and having different amino acids at positions 53 to 70 from those of the wild-type (SEQ ID NO: 6), etc.

These peptides are identical with wild-type LGR6 in a region consisting of amino acids at positions 91 to 567, a region consisting of amino acids at positions 319 to 567 or a region consisting of amino acids at positions 444 to 567 in the amino acid sequence of LGR6 shown in SEQ ID NO: 6. Thus, the antibody of the present invention allows detection of these deletion mutants.

Moreover, the epitope (antigenic determinant) for the anti-LGR6 antibody of the present invention also includes at least a part of the corresponding region in LGR6 of other animal origin.

The antibody against LGR6 of the present invention includes an antibody binding to a site (e.g., epitope) to which the antibody binds, as exemplified by an antibody binding to a site to which an antibody produced by the hybridoma of the present invention binds.

### (4) Preparation of genetically recombinant antibodies

A preferred embodiment of the antibody of the present invention may be a genetically recombinant antibody. Examples of a genetically recombinant antibody include, but are not limited to, a chimeric antibody, a humanized antibody and a reconstituted human antibody, etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855, (1984)). For preparation of a chimeric antibody, gene recombination technology may be used for its construction such that the thus linked antibody is obtained. In this regard, the antibody variable regions of mouse origin are preferably composed of a heavy chain variable region which comprises or consists of, for example, the amino acid sequence shown in SEQ ID NO: 23 and a light chain variable region which comprises or consists of, for example, the amino acid sequence shown in SEQ ID NO: 25.

For preparation of a humanized antibody, it is possible to use a process referred to as so-called CDR grafting (CDR transplantation). CDR grafting is a technique to prepare reconstituted variable regions whose framework regions (FRs) are of human origin and whose CDRs are of mouse origin by transplanting complementarity determining regions (CDRs) from mouse antibody variable regions to human variable regions. Next, these humanized reconstituted human variable regions are linked to human constant regions. Procedures for preparation of such a humanized antibody are well known in the art (see, e.g., Nature, 321, 522-525 (1986); J. Mol. Biol., 196, 901-917 (1987); Queen C et al., Proc. Natl. Acad. Sci. USA, 86: 10029-10033 (1989); Japanese Patent No. 2828340). In this regard, the amino acid sequences of CDRs of mouse origin which can be used for the humanized anti-LGR6 antibody of the present invention are preferably, but not limited to, the amino acid sequences shown in SEQ ID NOs: 27, 29 and 31 for heavy chain variable region CDRs 1 to 3, respectively, and the amino acid sequences shown in SEQ ID NOs: 33, 35 and 37 for light chain variable region CDRs 1 to 3, respectively, by way of example.

A reconstituted human antibody (complete human antibody) is generally an antibody in which hyper variable regions serving as antigen-binding sites in its variable regions (V regions), the other regions in its V regions and its constant regions have the same structures as those of human antibody. Techniques for reconstituted human antibody preparation are also known, and in the case of gene sequences common to humans, an approach has been established for their preparation by genetic engineering procedures. Such a reconstituted human antibody may be obtained, for example, by using human antibody-producing mice which have human chromosome fragments comprising genes for human antibody heavy chain (H chain) and light chain (L chain) (see, e.g., Tomizuka, K. et al., Nature Genetics, (1977) 16, 133-143; Kuroiwa, Y. et al., Nuc. Acids Res., (1998) 26, 3447-3448; Yoshida, H. et al., Animal Cell Technology: Basic and Applied Aspects, (1999) 10, 69-73 (Kitagawa, Y, Matuda, T. and Iijima, S. eds.), Kluwer Academic Publishers; Tomizuka, K. et al., Proc. Natl. Acad. Sci. USA, (2000) 97, 722-727) or by obtaining a phage display-derived human antibody selected from human antibody libraries (see, e.g., Wormstone, I. M. et al, Investigative Ophthalmology & Visual Science., (2002) 43 (7), 2301-8; Carmen, S. et al., Briefings in Functional Genomics and Proteomics, (2002) 1 (2), 189-203; Siriwardena, D. et al., Opthalmology, (2002) 109 (3), 427-431).

Alternatively, in the present invention, a hybridoma or DNA or RNA extracted from this hybridoma may be used as a starting material to prepare a chimeric antibody, a humanized antibody or a reconstituted human antibody in accordance with the well-known approaches mentioned above.

Further, a protein fused with the antibody of the present invention may be prepared from the antibody variable regions and any other protein by known gene recombination techniques. Alternatively, such a fusion protein may be prepared by crosslinking the monoclonal antibody with any other protein using a crosslinker.

### (5) Preparation of antibody fragments

A fragment of the antibody against LGR6 for use in the present invention specifically binds to LGR6.

A fragment of the antibody is intended to mean a partial region of the antibody of the present invention, and examples include Fab, Fab', F(ab')₂, Fv, diabody (dibodies), dsFv, scFv (single chain Fv) and so on. The above antibody fragments may be obtained by cleaving the antibody of the present invention with various proteases depending on the intended purpose.

For example, Fab may be obtained by treating an antibody molecule with papain, while F(ab')₂ may be obtained by treating an antibody molecule with pepsin. Likewise, Fab' may be obtained by cleaving the disulfide bonds in the hinge region of the above F(ab')₂.

In the case of scFv, cDNAs encoding antibody heavy chain variable region (H chain V region) and light chain variable region (L chain V region) may be obtained to construct DNA encoding scFv. This DNA may be inserted into an expression vector, and the resulting expression vector may be introduced into a host organism to cause expression, whereby scFv may be prepared.

In the case of diabody, cDNAs encoding antibody H chain V region and L chain V region may be obtained to construct DNA encoding scFv such that the amino acid sequence of a peptide linker has a length of 8 residues or less. This DNA may be inserted into an expression vector, and the resulting expression vector may be introduced into a host organism to cause expression, whereby diabody may be prepared.

In the case of dsFv, cDNAs encoding antibody H chain V region and L chain V region may be obtained to construct DNA encoding dsFv. This DNA may be inserted into an expression vector, and the resulting expression vector may be introduced into a host organism to cause expression, whereby dsFv may be prepared.

In the present invention, the nucleotide sequence of DNA encoding the heavy chain variable region may be exemplified by those comprising or consisting of the nucleotide sequence shown in SEQ ID NO: 22, while the nucleotide sequence of DNA encoding the light chain variable region may be exemplified by those comprising or consisting of the nucleotide sequence shown in SEQ ID NO: 24.

Moreover, specific examples of the antibody fragment of the present invention include, but are not limited to, antibody fragments comprising the amino acid sequences shown in SEQ ID NOs: 27, 29 and 31 for the amino acid sequences of VH CDRs 1 to 3, respectively, and/or comprising the amino acid sequences shown in SEQ ID NOs: 33, 35 and 37 for the amino acid sequences of VL CDRs 1 to 3, respectively. Further examples include antibody fragments comprising the amino acid sequence shown in SEQ ID NO: 23 for VH, and/or comprising the amino acid sequence shown in SEQ ID NO: 25 for VL.

A CDR-containing antibody fragment (peptide) is configured to comprise at least one or more regions of VH or VL CDRs (CDRs 1 to 3). In the case of an antibody fragment containing a plurality of CDRs, these CDRs may be linked directly or via an appropriate peptide linker. For preparation of a CDR-containing antibody fragment, DNA encoding antibody VH and VL CDRs may be constructed, and this DNA may be inserted into an expression vector for prokaryotic organisms or an expression vector for eukaryotic organisms, and the resulting expression vector may be introduced into a prokaryotic organism or a eukaryotic organism to cause expression. Alternatively, a CDR-containing peptide may also be prepared by chemical synthesis such as Fmoc method (fluorenylmethyloxycarbonyl method) and tBoc method (t-butyloxycarbonyl method).

The nucleotide sequences encoding VH CDRs 1 to 3 are preferably the nucleotide sequences shown in SEQ ID NOs: 26, 28 and 29, respectively, by way of example, while the nucleotide sequences encoding VL CDRs 1 to 3 are preferably the nucleotide sequences shown in SEQ ID NOs: 32, 34 and 36, respectively, by way of example.

### (6) Binding affinity

The binding affinity can be determined from the binding constant (KA) and the dissociation constant (KD). The affinity equilibrium constant (K) is expressed as the KA/KD ratio. The binding affinity may be detected in the following manner.

The antibody of the present invention has a dissociation constant (KD) of at least 1 × 10⁻¹⁰ M and may have an affinity which is, for example, 2- to 5-fold, 5- to 10-fold, 10- to 100-fold, 100- to 1000-fold or 1000- to 10,000-fold higher than this dissociation constant. More specifically, the dissociation constant (KD) of the antibody of the present invention in relation to LGR6 binding affinity is 1 × 10⁻¹⁰ M, 5 × 10⁻¹¹ M, 1 × 10⁻¹¹ M, 5 × 10⁻¹² M, 1 × 10⁻¹² M, 5 × 10⁻¹³ M, 1 × 10⁻¹³ M, 5 × 10⁻¹⁴ M, 1 × 10⁻¹⁴ M, 5 × 10⁻¹⁵ M or 1 × 10⁻¹⁵ M, and is preferably 1 × 10⁻¹⁰ M to 1 × 10⁻¹³ M. Alternatively, the antibody of the present invention may have a value lower than these KD values and a higher affinity.

In this regard, if the dissociation constant (KD) of an antibody to be measured for its affinity is within about 1- to 100-fold of the KD of the antibody of the present invention, this antibody is regarded as being substantially the same as the antibody of the present invention and therefore falls within the present invention.

The binding constant (KA) and the dissociation constant (KD) may be measured by surface plasmon resonance (SPR), and any known instrument and method which allow real-time detection and monitoring of the binding rate may be used for this purpose (e.g., Biacore^{®} T200 (GE Healthcare), ProteON XPR36 (Bio-Rad)).

### 3. Reagent for detection or diagnosis of cancer (agent for detection or diagnosis of cancer)

The reagent for detection or diagnosis of cancer according to the present invention comprises the antibody or fragment thereof described above in the section "2. Antibody against LGR6 (anti-LGR6 antibody)." Since the antibody of the present invention specifically binds to LGR6 expressed in cancer, the reagent of the present invention comprising this antibody can be used for detection or diagnosis of cancer.

Examples of cancer to be detected or diagnosed in the present invention include colorectal cancer, breast cancer, uterine cancer, gastric cancer, thyroid cancer, pancreatic cancer, brain tumor, cervical cancer, esophageal cancer, tongue cancer, lung cancer, small intestinal cancer, duodenal cancer, bladder cancer, kidney cancer, liver cancer, prostate cancer, uterine cervical cancer, ovarian cancer, gallbladder cancer, pharyngeal cancer, sarcoma, melanoma, leukemia, lymphoma, multiple myeloma and so on, although preferred are cancers where LGR6 is significantly highly expressed when compared to normal cells or tissues. Examples of such cancers include colorectal cancer, breast cancer, uterine cancer, gastric cancer, thyroid cancer, pancreatic cancer and so on, with colorectal cancer, breast cancer, uterine cancer and gastric cancer being preferred, and with colorectal cancer and breast cancer being more preferred.

The reagent of the present invention may further comprise a pharmaceutically acceptable carrier, in addition to the antibody of the present invention. The term "pharmaceutically acceptable carrier" refers to any type of carrier (e.g., liposomes, lipid vesicles, micelles), diluent, excipient, wetting agent, buffering agent, suspending agent, lubricant, adjuvant, emulsifier, disintegrant, absorbent, preservative, surfactant, coloring agent, flavoring agent or the like, which is suitable for the reagent of the present invention.

In another embodiment, the present invention provides the use of a monoclonal antibody against LGR6 or a fragment thereof for the manufacture of a reagent for detection or diagnosis of cancer. Moreover, the present invention also provides a monoclonal antibody against LGR6 or a fragment thereof for use in the detection or diagnosis of cancer. Further, the present invention provides a monoclonal antibody against LGR6 or a fragment thereof for use as a reagent for detection or diagnosis of cancer. Such a monoclonal antibody against LGR6 or a fragment thereof is as described above in the section "2. Antibody against LGR6 (anti-LGR6 antibody)."

### 4. Method for detection or diagnosis of cancer

The method for detection or diagnosis of cancer according to the present invention comprises the step of contacting the antibody or fragment thereof described above in the section "2. Antibody against LGR6 (anti-LGR6 antibody)" with a biological sample taken from a subject (hereinafter also simply referred to as a "biological sample") and detecting LGR6 in the sample. In this case, it is preferred that the detection results of LGR6 are connected with the possibility of cancer.

In the context of the present invention, the term "subject" is intended to include, for example, humans, rabbits, guinea pigs, rats, mice, hamsters, cats, dogs, goats, pigs, sheep, cows, horses, monkeys and other mammals, with humans being preferred.

Likewise, the biological sample is not limited in any way and refers to, for example, mammalian tissue or cells, a homogenate or extract thereof, body fluid or excrement per se or a sample containing any of them. Moreover, the biological sample also includes samples obtained from mammalian tissue or cells, a homogenate or extract thereof, body fluid and excrement through any type of treatment, e.g., dilution, separation, nucleic acid or protein extraction, protein denaturation, etc. A biological sample preferred for screening of colorectal cancer or gastric cancer is body fluid or excrement, particularly feces. For screening of bladder cancer or kidney cancer, it is possible to use tissue or urine. Likewise, for screening of breast cancer or uterine cancer, it is possible to use mother's milk, sanitary goods or the like. Any biological samples may be applied by being pretreated appropriately.

When feces is used as a sample, the sample is preferably prepared by the method described in JP 2005-46065 A. This method is designed to efficiently collect cancer cells from feces. More specifically, feces and a buffer are introduced into a stomacher bag to prepare a suspension of feces. This suspension is filtered through a multi-filter apparatus to capture cells on the final filter whose bore size is set to 10 µm or less, from which the cells are then collected using Ber-EP4 antibody-bound magnetic beads (Dynabeads Epithelial Enrich, Dynal Biotech). In addition to this, efficient cell collection techniques (e.g., Percoll centrifugation) commonly used by those skilled in the art may be used for this purpose.

In various cancers including colorectal cancer and breast cancer, any tumor marker has not yet been established which allows early detection using body fluid. Commonly used markers may show false negative results even in advanced stages of cancer. In contrast, when using the antibody of the present invention, a trace amount of cells, which express LGR6 serving as a marker, contained in body fluid or excrement can be detected and/or measured before cancer has progressed.

If the "subject" is a human, the subject includes cancer patients (e.g., early cancer patients, advanced cancer patients) and normal subjects. For example, in the case of colorectal cancer, gastric cancer or the like, early cancer refers to a state where cancer cells remain in the mucosa or submucosa, while advanced cancer refers to a state where cancer cells have reached the muscularis propria or deeper layers. Based on these criteria, patients may be categorized and provided for the detection of the present invention. Moreover, as a blood sample, preferred is peripheral blood and more preferred is peripheral blood-derived serum. Further, tissues include cancer tissues and normal tissues which may develop cancer.

In the context of the present invention, the term "contact" is intended to mean that the antibody of the present invention and a biological sample taken from a subject are allowed to exist in the same reaction system, as exemplified by mixing the antibody of the present invention and the biological sample in a reaction well, adding the antibody of the present invention to the biological sample, adding either the antibody of the present invention or the biological sample to a carrier on which the other is immobilized, etc.

In the present invention, for LGR6 detection or for measurement or evaluation of LGR6 expression levels, it is possible to use, for example, enzyme immunoassay (EIA), fluorescence immunoassay (FIA), radioimmunoassay (RIA), chemiluminescence immunoassay (CIA), turbidimetric immunoassay, immunonephelometry, latex agglutination, latex turbidimetry, hemagglutination reaction, particle agglutination reaction, Western blotting, immunostaining, immunoprecipitation, immunochromatography and so on.

Any device may be used for such detection and other purposes, and examples include a micro-well plate, an array, a chip, a flow cytometer, a surface plasmon resonance apparatus, an immunochromatographic strip and so on. In the case of using such a device, signals (e.g., color development, fluorescence intensity, luminescence intensity) can be detected, measured and/or evaluated, and the results of detection, measurement or evaluation can be connected with the possibility of cancer, etc.

In the present invention, based on the results of LGR6 detection or on the results of measurement or evaluation of LGR6 expression levels, a subject can be diagnosed for the presence or absence of the possibility of suffering from cancer.

In cases where the detection or diagnostic method of the present invention is accomplished by enzyme immunoassay or fluorescence immunoassay, an appropriately pretreated sample as described above (e.g., cells including cancer cells collected from feces or sections prepared from tissue taken from a patient) may be immobilized on a solid phase (e.g., micro-well plates or slide glasses) and provided for immunological reaction. Alternatively, a cell suspension may be reacted with the antibody in a tube. Moreover, it is also possible to use procedures in which the monoclonal antibody of the present invention is immobilized on beads or micro-well plates and then reacted with cells.

In this case, the monoclonal antibody of the present invention may be labeled with an enzyme or a fluorescent substance to thereby directly detect the reaction as fluorescence signals, or alternatively, a labeled secondary antibody which binds to the monoclonal antibody of the present invention may be used to indirectly detect signals. As a labelling substance, any substance commonly used by those skilled in the art may be used, as exemplified by peroxidase (POD), alkaline phosphatase, β-galactosidase, a biotin-avidin complex, etc. Likewise, the detection method used for this purpose may be any of competitive assay, sandwich assay or direct adsorption assay, etc. Moreover, the strength of the reaction or the ratio of cells bound to the antibody among all cells in the reacted sample may be measured and compared with the predetermined reference value or index to thereby determine or diagnose the possibility of suffering from cancer.

In the present invention, when LGR6 detection or measurement or evaluation of LGR6 expression levels is accomplished by using immunostaining, for example, the degree of staining in immunohistological staining (i.e., the detection results of LGR6) may be ranked as strongly positive (Strong), positive (Moderate), weakly positive (Weak), negative (Negative), etc., and the degree of staining is connected with the possibility of cancer to thereby detect cancer or diagnose a subject for the presence or absence of the possibility of cancer (the possibility of suffering from cancer).

Moreover, under the assumption that the extracellular region of LGR6 is released into blood, the expression level of LGR6 in a blood sample may be measured to thereby detect cancer or diagnose a subject for the presence or absence of the possibility of cancer. In this case, for detection of cancer by connecting the results measured for the expression level of LGR6 with the possibility of cancer, it is desired to define a critical value (cut-off value) for the expression level of LGR6 in a biological sample.

The cut-off value for the expression level of LGR6 may be determined as follows, by way of example. First, biological samples derived from cancer patients are measured for their expression levels of LGR6. The number of patients intended here is two or more, for example, 5 or more, 10 or more, 50 or more, or 100 or more. Preferably, biological samples derived from two or more normal subjects have also been measured for their expression levels of LGR6, and the number of normal subjects intended here is two or more, for example, 5 or more, 10 or more, 50 or more, or 100 or more. Then, from all the cases including both the group of biological samples derived from cancer patients and the group of biological samples derived from normal subjects, the cut-off value for the expression level of LGR6 is determined by statistical processing. For statistical processing, 4-parameter logistic fitting may be used to prepare a standard curve. On the basis of the signal value in a LGR6-free sample, the double of this numerical value may be defined to be the cut-off value. Cases provided for statistical analysis may also be classified by the type of cancer (e.g., colorectal cancer, breast cancer, uterine cancer, gastric cancer, thyroid cancer, pancreatic cancer, leukemia), the stage of cancer, the presence or absence of recurrence, the presence or absence of metastasis, before or after surgery, etc. Furthermore, statistical processing may also be accomplished by combining, as appropriate, the measured values of LGR6 expression levels in normal subjects and the measured values of LGR6 expression levels in cancer patients when classified by the type of cancer, the stage of cancer, the presence or absence of recurrence, the presence or absence of metastasis, before or after surgery, etc.

In the present invention, the critical value (cut-off value) for the expression levels of LGR6 (extracellular region) in blood samples is, for example, about 10 ng/ml, 11 ng/ml, 12 ng/ml, 13 ng/ml, 14 ng/ml, 15 ng/ml, 16 ng/ml, 17 ng/ml, 18 ng/ml, 19 ng/ml, 20 ng/ml, 21 ng/ml, 22 ng/ml, 23 ng/ml, 24 ng/ml, 25 ng/ml, 26 ng/ml, 27 ng/ml, 28 ng/ml, 29 ng/ml, 30 ng/ml, 35 ng/ml, 40 ng/ml, 45 ng/ml, 50 ng/ml, 55 ng/ml, 60 ng/ml or 65 ng/ml when expressed as a concentration in serum, with about 20 ng/ml being preferred.

When a sample is measured for its expression level of LGR6 under the above conditions, if the measured expression level of LGR6 is equal to or higher than the above cut-off value, it is possible to make a determination that cancer was detected or to make a diagnosis that the subject has the possibility of suffering from cancer. In the present invention, an upper limit may be provided for the value of serum concentration required for the above determination or diagnosis. For example, if the measured value is equal to or higher than the above cut-off value and is equal to or lower than the given upper limit value (e.g., 200 ng/ml or less, 190 ng/ml or less, 180 ng/ml or less, 170 ng/ml or less, 160 ng/ml or less, 150 ng/ml or less, 145 ng/ml or less, 140 ng/ml or less, 135 ng/ml or less, 130 ng/ml or less), it is possible to detect cancer or diagnose a subject for the presence or absence of the possibility of cancer.

In the present invention, the probability of the detection results obtained when cancer was detected is 60% or more, 70% or more, 80% or more, 90% or more, or 95% or more, and preferably 99% or more.

Further, in addition to the embodiment where the expression level of LGR6 measured in a sample from a single subject (patient) is compared with the above cut-off value to detect or diagnose the relation to cancer, biological samples derived from a plurality of patients may be used and measured for their expression levels of LGR6 in another embodiment of the present invention. Thus, in the same manner as used to determine the above cut-off value, in a population (primary population) including a given number of normal subjects and patients, their expression levels of LGR6 may be measured and processed by statistical analysis, whereby these subjects belonging to the population may be divided into a group where cancer was detected and a group where cancer was not detected. Further, the thus obtained measured values are used as master data, and a comparison may be made between these master data and the expression levels of LGR6 in samples derived from individual subjects to be detected or diagnosed in another population (secondary population).

Alternatively, the data of the respective patients may be incorporated into the values of the above population and subjected again to data processing of LGR6 expression levels to increase the number of cases of target patients (population). The increased number of cases can improve the accuracy of the critical value for the expression level of LGR6 to thereby improve the accuracy of detection or diagnosis in a single subject or a plurality of subjects.

In the present invention, a comparison may also be made between (i) the expression level of LGR6 in a biological sample from a subject and (ii) the expression level of LGR6 in a biological sample from a normal subject.

Moreover, if the expression level of LGR6 in (i) above is higher than the expression level of LGR6 in (ii) above, e.g., is about 10% or more, about 20% or more, about 30% or more, about 40% or more, about 50% or more, about 60% or more, about 70% or more, about 80% or more, about 90% or more, or about 100% or more higher than the expression level of LGR6 in a biological sample from a normal subject, it is possible to make a determination that cancer was detected or to make a diagnosis that the subject has the possibility of suffering from cancer.

The above detection results may be used, for example, as main data or supplemental data in screening of cancer or in definitive diagnosis of therapeutic effects.

Since LGR6 expression is observed in patients with various types of cancer, it cannot be identified in some cases what type of cancer a subject has even when the expression level of LGR6 is detected during medical examination, etc. For this reason, such a subject is evaluated as being "suspected to have cancer" in group health screening or the like, and this evaluation result may be used later as supplemental data to identify the type of cancer and/or determine the degree of cancer progression (for detailed examination). Moreover, when LGR6 was detected by using a sample derived from a patient who was suspected to have some type of cancer, such a result may be used as main data on cancer type (e.g., definitive diagnosis). It should be noted that identification of cancer type may be accomplished by using other tumor markers, diagnostic imaging, pathological diagnosis, etc.

Namely, the present invention also provides a method for aiding detection or diagnosis of cancer, which comprises the step of contacting a monoclonal antibody against LGR6 or a fragment thereof with a biological sample taken from a subject and detecting LGR6 in the sample.

### 5. Kit for detection or diagnosis of cancer

The anti-LGR6 antibody of the present invention may be provided in the form of a kit for detection or diagnosis of cancer. The kit of the present invention comprises the antibody and may further comprise a labelling substance, or alternatively, may comprise an immobilized reagent in which the antibody or the antibody labeled with the labelling substance is immobilized. The antibody labeled with the labelling substance is intended to mean the antibody labeled with an enzyme, a radioisotope, a fluorescent compound, a chemiluminescent compound or the like. In addition to the above constituent elements, the kit of the present invention may further comprise other reagents required to accomplish the detection of the present invention, as exemplified by an enzyme and a substrate (e.g., a chromogenic substrate), a substrate diluent, an enzyme reaction stop solution, or an analyte diluent and so on when the labeled product is an enzymatically labeled product. Moreover, the kit of the present invention may also comprise various buffers, sterilized water, various cell culture vessels, various reaction vessels (e.g., Eppendorf tubes), a blocking agent (e.g., bovine serum albumin (BSA), skimmed milk, goat serum or other serum components), a detergent, a surfactant, various plates, an antiseptic (e.g., sodium azide), an instruction manual for experimental operations (manufacturer's instructions) and so on. These reagents are provided, e.g., in an immobilized state, in an aqueous solution state or in a lyophilized state, and may be reconstituted into an appropriate state before use.

The kit of the present invention can be used effectively to accomplish the above detection method of the present invention and is extremely useful. The use of the kit of the present invention allows highly accurate screening of cancer patients during medical examination or the like, early diagnosis of cancer, determination of the degree of cancer progression, monitoring of therapeutic effects during treatment, and acquisition of information useful to predict metastasis and/or recurrence, so that the mortality due to cancer can be reduced.

Details on the anti-LGR antibody, the type of cancer, the detection method and so on are the same as described above.

### EXAMPLES

The present invention will be further described in more detail by way of the following illustrative examples, which are not intended to limit the scope of the invention.

### Example 1. Preparation of anti-LGR6 monoclonal antibodies

### (1) Cell culture

Mouse myeloma cell line P3X63-Ag8 (ATCC Accession No. CRL-1580), human colorectal cancer cell line HT-29 (ATCC Accession No. HTB38), human fetal kidney 293T cells were each cultured and subcultured in RPMI 1640 medium (Sigma) containing 10% (v/v) serum (Hyclone) at 37°C under 5% CO₂ for 48 to 72 hours, such that the confluency did not exceed 80%.

### (2) Cloning of LGR6 gene

293T cells were cultured and their total RNA was extracted with a Qiagen RNeasy Mini kit. From the extracted total RNA (2 µg), cDNA was synthesized by reverse transcription (RT) reaction at 50°C for 1 hour with SuperScript III reverse transcriptase (Invitrogen), followed by heating at 85°C for 5 minutes to stop the reaction. The resulting cDNA was used as a template for PCR reaction with the following primers and with KOD PLUS (TOYOBO) to thereby amplify DNA comprising a nucleotide sequence (SEQ ID NO: 13) encoding amino acids at positions 319 to 567 of LGR6.

### Primer sequences

Forward: TTTGAATTCGATGCAGGAGTTTCCAGATCTCAAAGGC (SEQ ID NO: 14)
Reverse: TTTGCGGCCGCCACGTGTGAAGCAAAGGCCAAG (SEQ ID NO: 15)

The PCR reaction was conducted by preincubation at 95°C for 10 minutes and subsequent 40 cycles of denaturation at 95°C for 15 seconds and annealing/elongation at 58°C for 1 minute to thereby amplify the desired gene fragment.

The resulting amplified fragment was cleaved with restriction enzymes (EcoRI and NotI) at the restriction enzyme sites located on the primers and then integrated into pET32b vector (Invitrogen) to thereby obtain DNA comprising a nucleotide sequence encoding a partial protein of LGR6 having Trx-, S- and His-tags on the N-terminal side and a His-tag on the C-terminal side.

### (3) Preparation of LGR6 partial protein

BL21(DE3) (Invitrogen) was transformed with the vector prepared in (2) above and cultured in LB medium [1% (w/v) tryptone (Sigma), 0.5% (w/v) yeast extract (Sigma), 0.5% (w/v) NaCl (Sigma)] supplemented with 1% (w/v) glucose. After the medium turbidity reached 0.6 at a wavelength of 600 nm, 1 mM IPTG (WAKO) was added and culture was continued for 16 hours. The microbial cells were collected by centrifugation and then homogenized by ultrasonication to obtain a fraction containing the extracellular region of LGR6 as an insoluble protein.

About 10 mg of the sample was dissolved in Buffer A (1 M guanidine hydrochloride (Sigma), 10 mM DTT (Sigma), 10 mM EDTA (Sigma)) and reacted at 37°C for 1 hour. The reaction solution was added gently to 1 L of Buffer B (50 mM Tris, 150 mM NaCl, 5% glycerol, 0.4 mM oxidized glutathione (Sigma), pH 8.5) and stirred at 4°C for 18 hours. The dissolved sample was applied to a Ni-sepharose column (GE) and eluted with Buffer C (50 mM potassium phosphate buffer, 150 mM NaCl, 200 mM imidazole, pH 8.0), followed by dialysis against imidazole-free Buffer C to obtain a partial protein of LGR6 including its hinge region in purified form. This protein is hereinafter referred to as ECD2. The amino acid sequence of ECD2 is shown in SEQ ID NO: 9.

### (4) Immunization

ECD2 prepared above was mixed with an equal amount of Freund's complete adjuvant and intraperitoneally administered to BALB/c mice in a volume of 100 µl (about 40 µg/mouse). After about 3 weeks, ECD2 was also mixed with an equal amount of Freund's incomplete adjuvant and intraperitoneally administered to the mice. This operation was repeated 7 to 12 times, followed by confirmation of an increase in antibody titers. The mice showing increased titers were intravenously administered with ECD2 (about 40 µg) as the final immunization, and after 3 days, their spleens were excised.

### (5) Cell fusion

Mouse spleen lymphocytes were electrically fused with mouse myeloma cell line P3X63-Ag8. For cell fusion, 1 × 10⁸ spleen cells were mixed with 0.25 × 10⁸ cells of the myeloma cell line and suspended in EP Buffer (0.3 M mannitol, 0.1 mM CaCl₂, 0.1 mM MgCl₂) to give a cell density of 0.25 × 10⁸ cells/mL, followed by cell fusion with an electro cell fusion generator LF201 (Nepa Gene Co., Ltd., Japan). Fusion conditions were set in accordance with the manufacturer's recommended protocols.

The fused cells were suspended in HAT medium (Invitrogen) and dispensed into thirty 96-well plates in a volume of 100 µL per well. During culture, 200 µL of HAT medium was added to each well. After culture for 11 to 16 days, the plates were observed under a microscope, indicating that 5 to 12 colonies were formed per well.

### (6) ELISA

Culture supernatants were collected from wells where hybridomas were growing, and screened to select hybridomas producing monoclonal antibodies reactive to ECD2. ECD2 was diluted with PBS(-) and dispensed into 96-well ELISA plates (Nunc) in an amount of 1 µg per well, and immobilized on the plate surface by being allowed to stand overnight at 4°C. Then, after the plates were washed three times with 350 µL of PBS(-) containing 0.05% Tween 20 (hereinafter abbreviated as PBS-T), PBS-T containing 1% skimmed milk was dispensed in a volume of 300 µL per well, followed by blocking for 1 hour at room temperature. After washing with PBS-T, the culture supernatants of hybridomas were dispensed into the ECD2-immobilized ELISA plates and reacted for 1 hour at room temperature. After washing with PBS-T, a 10000-fold dilution of peroxidase (hereinafter abbreviated as POD)-labeled anti-mouse immunoglobulin antibody (BETHYL) was dispensed in a volume of 100 µL per well and reacted for 1 hour at room temperature. After washing in the same manner, a POD substrate prepared at 1 mg/mL POD was added to cause color development at room temperature for 5 minutes. After the reaction was stopped with 1.5 N sulfuric acid, the plates were measured for absorbance at 490 nm with a plate reader (Molecular Devices).

### (7) Obtaining of monoclonal antibodies

From among antibodies produced from the hybridoma cells formed in (5) above, antibodies reactive to ECD2 were selected in the same manner as shown in (6) above. Moreover, to remove antibodies reactive to the Trx-, S- and His-tags added during ECD2 preparation, pET32b vector alone was introduced into *E. coli* and the same procedure as shown in (3) above was repeated to express and purify a protein for use as a negative control. Hereinafter, this protein for use as a negative control is referred to as Tag.

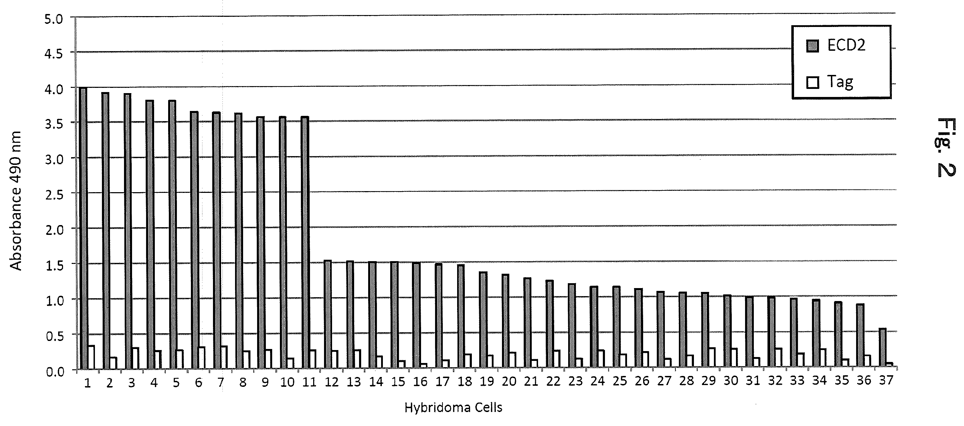

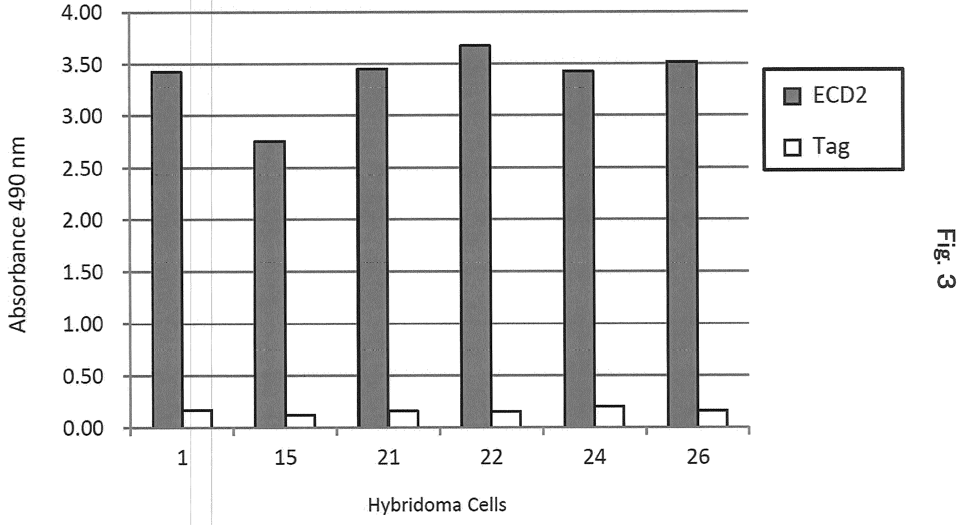

As a result of selecting hybridoma cells producing monoclonal antibodies which were reactive to ECD2 and not reactive to Tag, 37 clones were obtained. The results of ELISA for individual antibodies are shown in Figure 2. From among these hybridoma cells, those producing antibodies which were strongly reactive to ECD2 and not reactive to Tag were selected and cloned singly by limiting dilution, whereby 6 clones of hybridoma cells were established. The reactivity of antibodies produced from these hybridomas was measured by ELISA and the results obtained are shown in Figure 3. Hereinafter, antibodies from the 6 clones are also referred to as "Mu2Cl," "Mu2C 15," "Mu2C21," "Mu2C22," "Mu2C24" and "Mu2C26," respectively.

These hybridoma cells were each cultured in ten 10 cm dishes to reach 90% confluency and cultured for 10 days in a 1:1 mixed medium of HT medium (Invitrogen) and EX CELL Sp2/0 (Nichirei Bioscience Inc., Japan). The culture supernatants were collected and purified with a Protein G column. The Protein G column (GE Healthcare) was used in a volume of 0.5 mL relative to 100 mL culture supernatant. The cultured solutions were each passed at a flow rate of 1 to 3 ml/min through the Protein G column which had been equilibrated with PBS, followed by washing with 6 mL of a washing buffer (25 mM Tris-HCl (pH 7.4), 140 mM NaCl, 10 mM KCl). Then, antibody proteins were eluted with 1 mL of an elution buffer (0.1 M glycine (pH 2.5)) and neutralized with 3 M Tris-HCl (pH 7.4) to be within pH 7.0 to 7.4. The antibodies were concentrated with Amicon Ultra 30 (Millipore) and the buffer was replaced with PBS.

### Example 2. Analysis of anti-LGR6 monoclonal antibodies

### (1) Western blotting

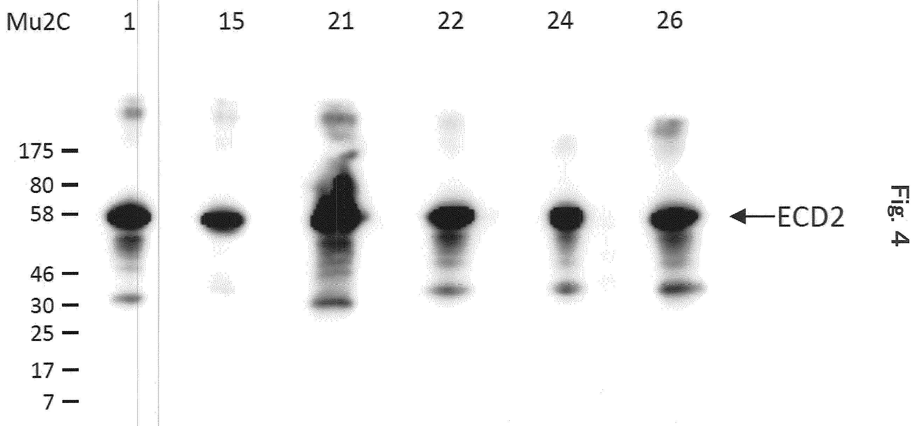

The antibodies obtained in Example 1. (7), which were produced by the six lines of hybridoma cells "Mu2C1," "Mu2C15," "Mu2C21," "Mu2C22," "Mu2C24" and "Mu2C26" were analyzed by Western blotting. First, these antibodies were analyzed for their reactivity to the antigen ECD2. ECD2 prepared in Example 1. (3) was measured for its protein concentration by the Bradford assay, and then adjusted to 1 µg/mL and provided for SDS-PAGE. After electrophoresis, the protein was transferred onto a PVDF membrane (PIERCE) with a Trans-Blot SD cell (BioRad Laboratories) in accordance with the manufacturer's recommended protocols. Skimmed milk dissolved at a concentration of 5% (w/v) in TBS-T was used to block the membrane at room temperature for 30 minutes, and the membrane was washed twice with TBS-T. The six types of monoclonal antibodies prepared in Example 1. (7) were each diluted to 1 µg/mL with TBS-T and reacted with the membrane for 1 hour at room temperature. After washing three times with TBS-T, anti-mouse IgG polyclonal antibody-HRP label (BETHYL) was diluted 10,000-fold with TBS-T for use as a secondary antibody. This dilution was reacted with the membrane at room temperature for 30 minutes, followed by washing three times with TBS-T. The membrane was soaked in Immobilon (Millipore) and then wrapped, followed by signal detection with LAS-3000 (Fuji Photo Film Co., Ltd., Japan). The experimental results obtained are shown in Figure 4. These results indicated that the six types of antibodies were also reactive to denatured ECD2.

### (2) Immunocytological staining

The six types of antibodies obtained in Example 1. (7) were analyzed for their reactivity to colorectal cancer cells (HT-29). Hereinafter, the antibodies produced by the hybridoma cells are also expressed as their respective clone numbers.

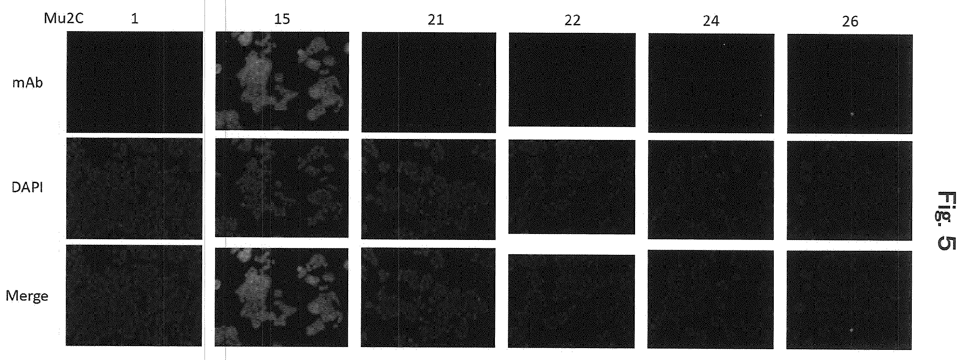

HT-29 cells were cultured to reach 80% confluency and then seeded onto cover slips coated with Cellmatrix Type I-A (Nitta Gelatin Inc., Japan). After culture for 2 days, the cells were fixed with 10% neutral buffered formalin (WAKO). After being treated with 0.3% (v/v) aqueous hydrogen peroxide for 20 minutes, the cover slips were washed three times with TBS-T and treated with TBS-T containing 5% (w/v) skimmed milk, followed by addition of the antibodies purified in (7) above at a concentration of 10 µg/mL and reaction at 4°C for 16 hours. After washing three times with TBS-T, anti-mouse IgG polyclonal antibody-Alexa Fluor 488 label or anti-mouse IgM polyclonal antibody-Alexa Fluor 488 label was reacted as a secondary antibody at room temperature for 30 minutes. After washing three times with TBS-T, the cover slips were sealed with Mounting Medium (Vector Shield). Figure 5 shows the results analyzed for fluorescence intensity under the same conditions. Strong signals were observed only in Mu2C15, thus suggesting that the cell membrane and cytoplasm were stained. Namely, Mu2C15 was confirmed to recognize LGR6 on the cell surface. Further, since LGR6 is a membrane protein with seven transmembrane domains, signals observed in the cytoplasm are indicative of the finding that LGR6 on the cell membrane was taken up into cells through internalization. A phenomenon where LGR6 causes internalization has not so far been reported, and hence this is the first report of this phenomenon.

### (3) FACS

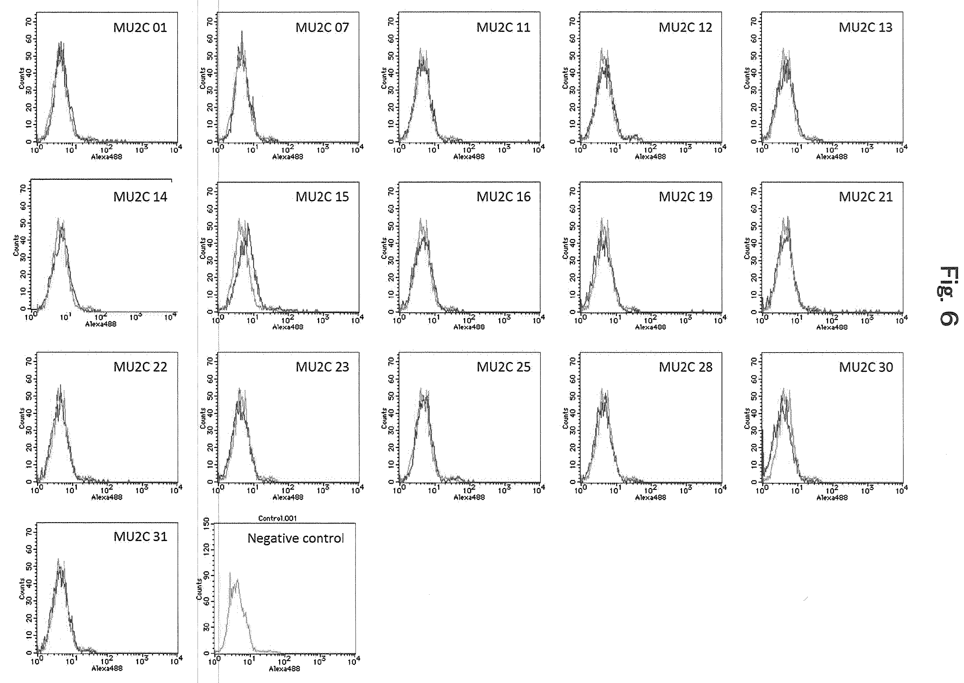

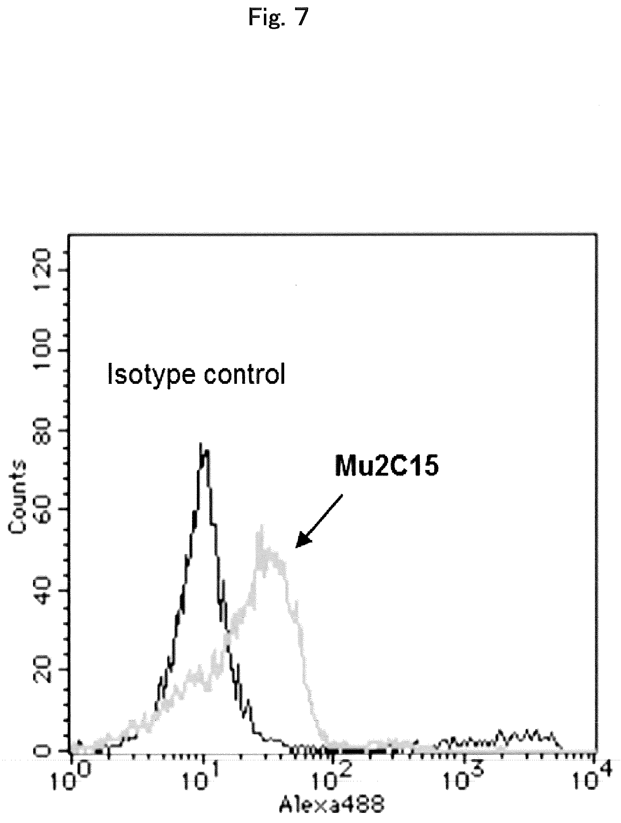

HT-29 cells, which are human colorectal cancer cells, were cultured to reach 90% confluency. After washing twice with PBS, the cells were detached non-enzymatically from the plates with Cell Dissociation Buffer (Invitrogen) and collected into 1.5 mL tubes. From 16 lines among the hybridoma cells obtained in Example 1. (7), i.e., Mu2C1, 7, 11, 12, 13, 14, 15, 16, 19, 21, 22, 23, 25, 28, 30 and 31, culture supernatants were each collected and added in a volume of 100 µL, and then reacted for 60 minutes. As a control for comparison purposes, an antibody-free medium was prepared and provided for reaction. After washing twice with PBS + 2% FBS, Alexa Fluor 488-labeled goat anti-mouse IgG (Invitrogen) was added as a 1/1000 dilution in PBS + 2% FBS, and then reacted for 30 minutes. After washing twice with PBS + 2% FBS, the cells were analyzed by Guava Flow Cytometry (Millipore). The results obtained are shown in Figure 6. The antibodies except for Mu2C15 and Mu2C30 showed signals similar to those of the negative control and did not react with LGR6 in its native structure, whereas Mu2C 15 and Mu2C30 were found to show slight changes in signals. Moreover, in the case of Mu2C15, the antibody was purified to increase the antibody concentration to 100 µg/mL and provided for reaction with SW480 cells. The results obtained are shown in Figure 7. In this case, a clear shift in signals was observed when compared to the negative control. Namely, the antibody was found to react with living cells (living cancer cells).

These results indicated that the antibodies of the present invention were useful for detection of living cancer cells present in biological samples, e.g., blood samples.

### (4) Determination of nucleotide sequences and amino acid sequences for antibody variable regions

The hybridoma cells obtained in Example 1. (7) were cultured and their total RNA was extracted with a Qiagen RNeasy Mini kit. From the extracted total RNA (500 ng), cDNA was synthesized by reverse transcription (RT) reaction at 42°C for 1.5 hours with a SMARTer RACE cDNA Amplification Kit (Clontech), followed by heating at 70°C for 10 minutes to stop the reaction. The resulting cDNA was used as a template for PCR reaction with the following primers and with KOD PLUS ver.2 (TOYOBO) to thereby amplify a desired gene. As a representative example of the antibodies produced from the hybridoma cells obtained in Example 1. (7), the antibody produced by hybridoma cells "Mu2C15" (also referred to as "Mu2C15" or "Mu2C 15 antibody") was determined for amino acid sequences of its variable regions in the following manner.

To amplify an H chain variable region fragment of the antibody, the following primers (SEQ ID NOs: 16, 17 and 18) were used in PCR reaction (30 cycles of denaturation at 94°C for 2 minutes, subsequent annealing at 56°C for 15 seconds and elongation at 68°C for 45 seconds) to thereby obtain the desired fragment.
Long: 5'-CTAATACGACTCACTATAGGGCAAGCAGTGGTATCAACGCA GAGT-3' (SEQ ID NO: 16)
Short: 5'-CTAATACGACTCACTATAGGGC-3' (SEQ ID NO: 17)
mIgG1_CH_reverse: 5'-CCAGGGGCCAGTGGATAGACAGATGGGGGTG TCG-3' (SEQ ID NO: 18)

To amplify an L chain leader sequence and an L chain variable region fragment of the antibody, the above primers (Long and Short) and the following primer (SEQ ID NO: 19) were used in PCR reaction (30 cycles of denaturation at 94°C for 2 minutes, subsequent annealing at 56°C for 15 seconds and elongation at 68°C for 45 seconds) to thereby obtain the desired fragment.
mIgkappa_R3: 5'-GCACCTCCAGATGTTAACTGCTCACT-3' (SEQ ID NO: 19)

The purified PCR amplification fragments were each mixed with 10 mM dNTP Mix (Invitrogen) and 2 × GoTaq Maxter Mix (Promega) and reacted at 70°C for 15 minutes, followed by ice cooling at 4°C for 2 minutes to ensure dA addition to the 3'-terminal end. Then, using a pGEM-T-Easy Vector System (Promega), the H chain fragment and the L chain fragment were cloned by the so-called TA cloning method. Then, the following primers (SEQ ID NO: 20 and SEQ ID NO: 21) were used in sequencing reaction to determine the sequences of the H chain and L chain variable regions.
T7 promoter primer v2: GTCACGACGTTGTAAAACGA (SEQ ID NO: 20)
SP6 promoter primer: ATTTAGGTGACACTATAGAA (SEQ ID NO: 21)

The amino acid sequence of the H chain variable region (hereinafter also referred to as "VH") in the Mu2C15 antibody is shown in SEQ ID NO: 23, and the nucleotide sequence encoding this region is shown in SEQ ID NO: 22. Likewise, the amino acid sequence of the L chain variable region (hereinafter also referred to as "VL") in the Mu2C15 antibody is shown in SEQ ID NO: 25, and the nucleotide sequence encoding this region is shown in SEQ ID NO: 24.

Moreover, in the amino acid sequences of the variable regions in the Mu2C 15 antibody, regions showing particularly large changes (i.e., having low homology) when compared to the amino acid sequences of the variable regions in the existing anti-LGR6 antibodies were identified as complementarity determining regions (CDRs) of this antibody.

The amino acid sequences of the identified CDRs in the Mu2C15 antibody are shown below.
VH CDR1: GYTFTTYT (SEQ ID NO: 27)
VH CDR2: INPNNGYT (SEQ ID NO: 29)
VH CDR3: ARRDYYRFSTGFAY (SEQ ID NO: 31)
VL CDR1: QSLVHTNGNTY (SEQ ID NO: 33)
VL CDR2: KVS (SEQ ID NO: 35)
VH CDR3: SQSTHVPYT (SEQ ID NO: 37)

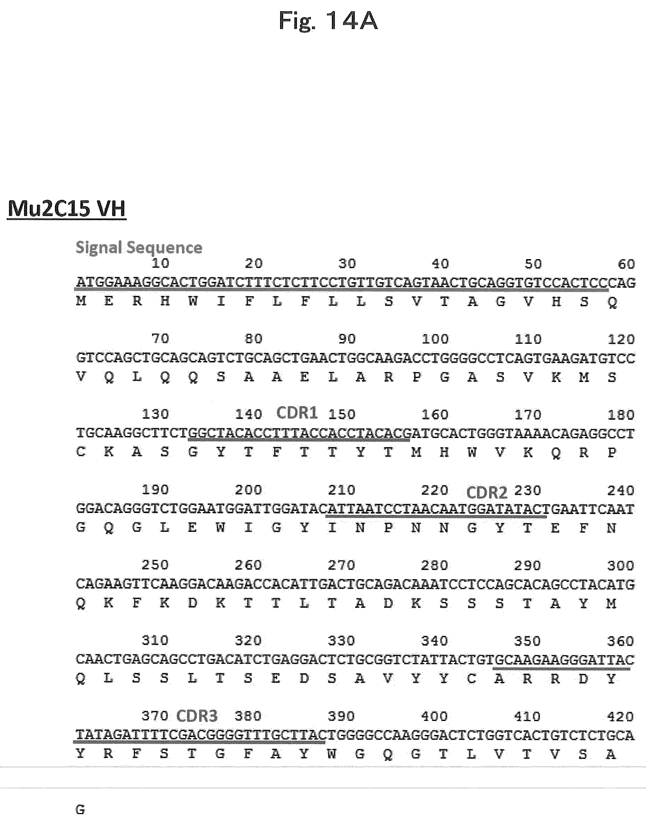

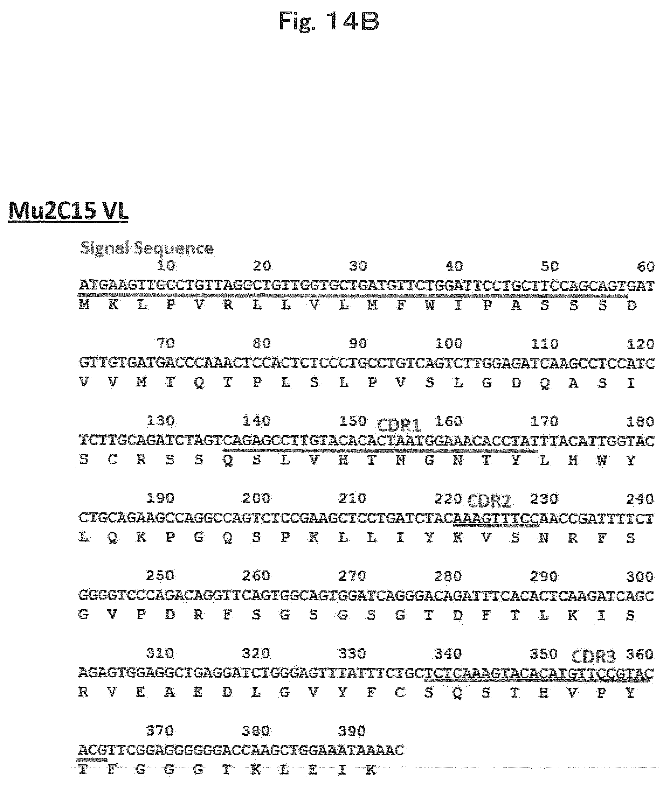

In addition, the nucleotide sequences encoding the above VH CDR1, VH CDR2 and VH CDR3 are show in SEQ ID NOs: 26, 28 and 30, respectively, while the nucleotide sequences encoding the above VL CDR1, VL CDR2 and VL CDR3 are shown in SEQ ID NOs: 32, 34 and 36, respectively. The positions of CDRs in VH and VL of the Mu2C15 antibody are shown in Figure 14A and Figure 14B.

### (5) Study on binding ability to other LGR family proteins

### (5-1) Homology analysis of LGR family proteins

As a representative example of the antibodies produced from the hybridoma cells obtained in Example 1 (7), the Mu2C15 antibody was used and analyzed for its binding ability to molecules belonging to the LGR family other than LGR6. Proteins having homology with the extracellular domain of human LGR6 are exemplified by human LGR4 and human LGR5. The amino acid sequence of human LGR4 is shown in SEQ ID NO: 39 and the amino acid sequence of human LGR5 is shown in SEQ ID NO: 41, each having homology with ECD2 described in Example 1 (3) and (4).

Amino acid sequence consisting of amino acids at positions 311 to 577 of human LGR4: (SEQ ID NO: 39)

Amino acid sequence consisting of amino acids at positions 319 to 563 of human LGR5: (SEQ ID NO: 41)

These amino acid sequences were analyzed for their homology with the amino acid sequence of ECD2 in human LGR6, indicating that the similarity was 78% between LGR6 and LGR4 and was 83% between LGR6 and LGR5, while the identity was 42% between LGR6 and LGR4 and was 51% between LGR6 and LGR5 (Figure 1).

Amino acid sequence consisting of amino acids at positions 311 to 577 of human LGR4: (SEQ ID NO: 39)

QFPNLTGTVHLESLTLTGTKISSIPNNLCQEQKMLRTLDLSYNNIRDLPSFNGCHA
LEEISLQRNQIYQIKEGTFQGLISLRILDLSRNLIHEIHSRAFATLGPITNLDVSFNE
LTSFPTEGLNGLNQLKLVGNFKLKEALAAKDFVNLRSLSVPYAYQCCAFWGCD
SYANLNTEDNSLQDHSVAQEKGTADAANVTSTLENEEHSQIIIHCTPSTGAFKPC
EYLLGSWMIRLTVWFIFLVALFFNLLVILTTFASCTSLPSSKLFI

Amino acid sequence consisting of amino acids at positions 319 to 563 of human LGR5: (SEQ ID NO: 41)

TEFPDLTGTANLESLTLTGAQISSLPQTVCNQLPNLQVLDLSYNLLEDLPSFSVCQ
KLQKIDLRHNEIYEIKVDTFQQLLSLRSLNLAWNKIAIIHPNAFSTLPSLIKLDLSS
NLLSSFPITGLHGLTHLKLTGNHALQSLISSENFPELKVIEMPYAYQCCAFGVCEN
AYKISNQWNKGDNSSMDDLHKKDAGMFQAQDERDLEDFLLDFEEDLKALHSV
QCSPSPGPFKPCEHLLDGWLIRIG

### (5-2) Cloning of genes for partial proteins of LGR family proteins

HT-29 cells were cultured and their RNA was extracted in the same manner as shown in Example 1 (2). After synthesis of cDNA, the following primers were used to amplify DNA comprising a nucleotide sequence (SEQ ID NO: 38) encoding amino acids at positions 311 to 577 of LGR4.

### Primer sequences

LGR4-F: CGTGAATTCGCAGCAGTTCCCCAATCTTAC (SEQ ID NO: 42)
LGR4-R: CGCAAAGCTTAGTAAGACGAATCATCCAGC (SEQ ID NO: 43)

PCR reaction was conducted by preincubation at 95°C for 10 minutes and subsequent 40 cycles of denaturation at 95°C for 15 seconds and annealing/elongation at 58°C for 1 minute to thereby amplify the desired gene fragment.

The resulting amplified fragment was cleaved with restriction enzymes (Hind III and EcoRI) at the restriction enzyme sites located on the primers and then integrated into pET32b vector (Invitrogen) to thereby obtain DNA comprising a nucleotide sequence encoding a partial protein of LGR4 having Trx-, S- and His-tags on the N-terminal side and a His-tag on the C-terminal side.

A nucleotide sequence (SEQ ID NO: 40) encoding amino acids at positions 319 to 563 of LGR5 was prepared by chemical synthesis (Medical & Biological Laboratories Co., Ltd., Japan), and then cleaved with restriction enzymes (BamHI and EcoRI) at the restriction enzyme sites designed outside of this nucleotide sequence and integrated into pET32a vector (Invitrogen) to thereby obtain DNA comprising a nucleotide sequence encoding a partial protein of LGR5 having Trx-, S- and His-tags on the N-terminal side and a His-tag on the C-terminal side.

### (5-3) Study on binding ability to LGR4 and LGR5 partial proteins

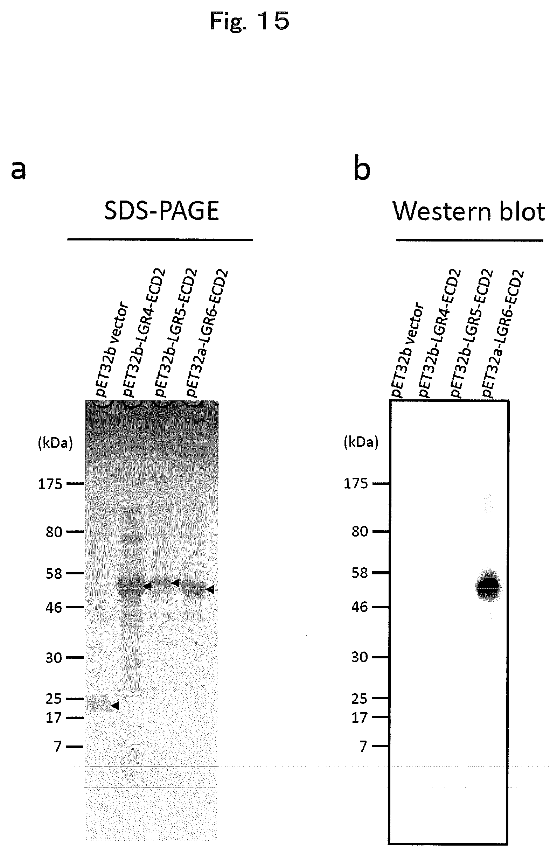

The vector comprising the nucleotide sequence encoding the partial protein of LGR6 described in Example 1. (2) and the vectors comprising the nucleotide sequences encoding the partial proteins of LGR4 and LGR5 prepared in (5-2) above were each used to transform BL21(DE3). Likewise, pET32b vector was also used to transform BL21(DE). After expression was induced in the same manner as shown in Example 1. (3), 5 x Loading Buffer (10% SDS, 10 mM dithiotheritol, 20% glycorol, 200 mM Tris-HCl (pH 6.8), 0.05% bromophenol blue) was added in a volume of 5 µl relative to 20 µl of each cell culture solution, followed by heating at 95°C for 5 minutes. Each sample was then provided for SDS-PAGE (Figure 15a).

After electrophoresis, the proteins were transferred onto a PVDF membrane. Skimmed milk dissolved at a concentration of 5% (w/v) in TBS-T was used to block the membrane at room temperature for 30 minutes, and the membrane was washed twice with TBS-T. Among the monoclonal antibodies obtained in Example 1. (7), Mu2C15 was diluted to 1 µg/mL with TBS-T and reacted with the membrane for 1 hour at room temperature. After washing three times with TBS-T, anti-mouse IgG polyclonal antibody-HRP label was diluted 100,000-fold with TBS-T for use as a secondary antibody. This dilution was reacted with the membrane at room temperature for 30 minutes, followed by washing three times with TBS-T. The membrane was soaked in Immobilon^{®} and then wrapped, followed by signal detection with LAS-3000 (Figure 15b)

These results indicated that the Mu2C 15 antibody was reactive to the partial protein of LGR6, but was not reactive to the partial proteins of LGR4 and LGR5.

### Example 3. Analysis of clinical tissue samples

### (1) Immunohistological staining using colorectal cancer tissues

The rate of LGR6 expression in colorectal cancer was analyzed by analysis of the reactivity between Mu2C15 and colorectal cancer tissue. In this example, human colorectal cancer tissue array slides (CDA3; SuperBiochips) were used for analysis.

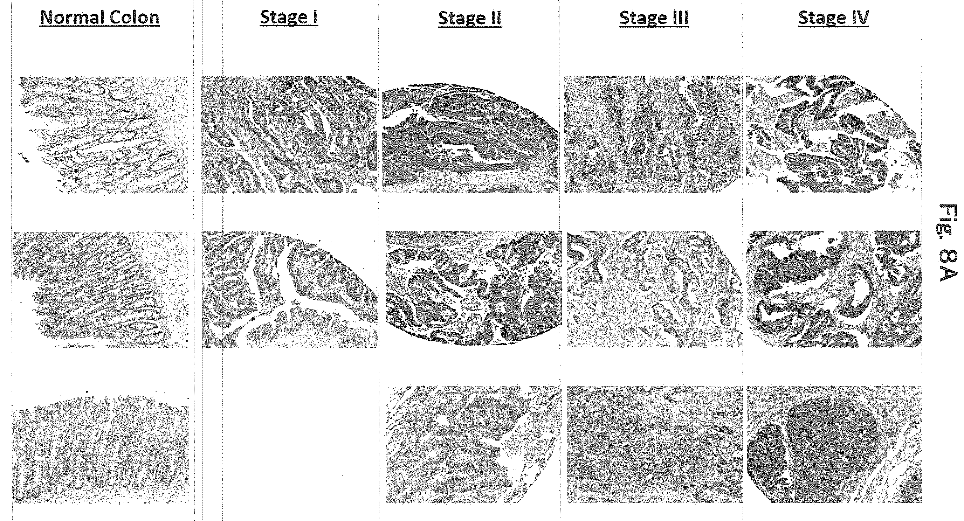

The colorectal cancer tissue array slides were deparaffinized by being soaked three times in xylene for 5 minutes. The slides were then soaked twice in 100% ethanol for 5 minutes and further soaked sequentially in 90% ethanol and 80% ethanol for 5 minutes each, and then soaked under running water for 2 minutes to hydrate the samples. The slides were soaked in 10 mM citrate buffer (pH 6.0) and microwaved at 600 W for 5 minutes. This operation was repeated three times in total to activate the antigen. The slides were treated with 3% hydrogen peroxide solution diluted in methanol for 10 minutes to thereby deactivate endogenous peroxidase activity. For the subsequent detection, a Histofine (M) kit (Nichirei Corporation, Japan) was used to conduct blocking, secondary antibody reaction, washing and detection reaction in accordance with the protocols attached to the kit. For primary antibody reaction, Mu2C15 was diluted to 5 or 10 µg/mL with TBS-T and reacted at room temperature for 1 hour. For color development, Impact DAB (Vector Laboratories) was used and reacted at room temperature for 5 minutes. The reacted slides were washed under running water and then counter stained with hematoxylin (Merck) and then sealed. The results of tissue staining are shown in Figure 8A.

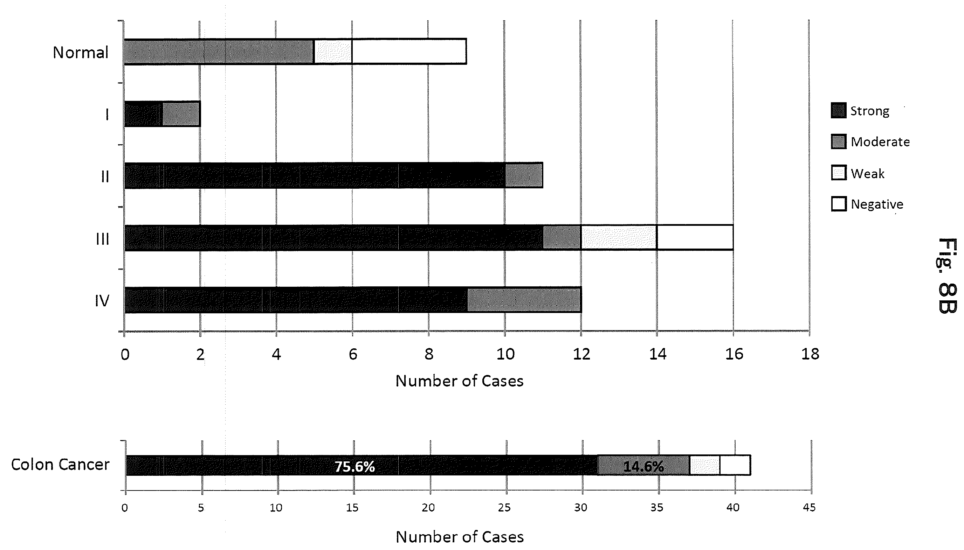

The degree of staining in immunohistological staining was ranked as strongly positive (Strong), positive (Moderate), weakly positive (Weak) or negative (Negative), and the results of immunohistological staining with Mu2C 15 were statistically analyzed. The results obtained are shown in Figure 8B.

In the case of normal colorectal mucosa, about half of the tissue samples used for measurement were almost not stained, and the remainder showed only weak staining. In contrast, in the case of colorectal cancer tissues, strongly positively stained tissues were already seen in stage I of cancer progression, and 90% or more of the samples in stage II used for measurement were found to show strongly positive results. This indicated that LGR6 was already expressed in early stages of colorectal cancer and 90% or more of the colorectal cancer tissue samples used for measurement showed positive or strongly positive results, and that LGR6 expression was able to be detected with high sensitivity when using the Mu2C 15 antibody.

### (2) Immunohistological staining using breast cancer tissues

The rate of LGR6 expression in breast cancer was analyzed by analysis of the reactivity between Mu2C15 and breast cancer tissue. For analysis, human breast cancer tissue array slides (FDA808b; USBiomax, BC081120; USBiomax, and CBA4; SuperBiochips) were used.

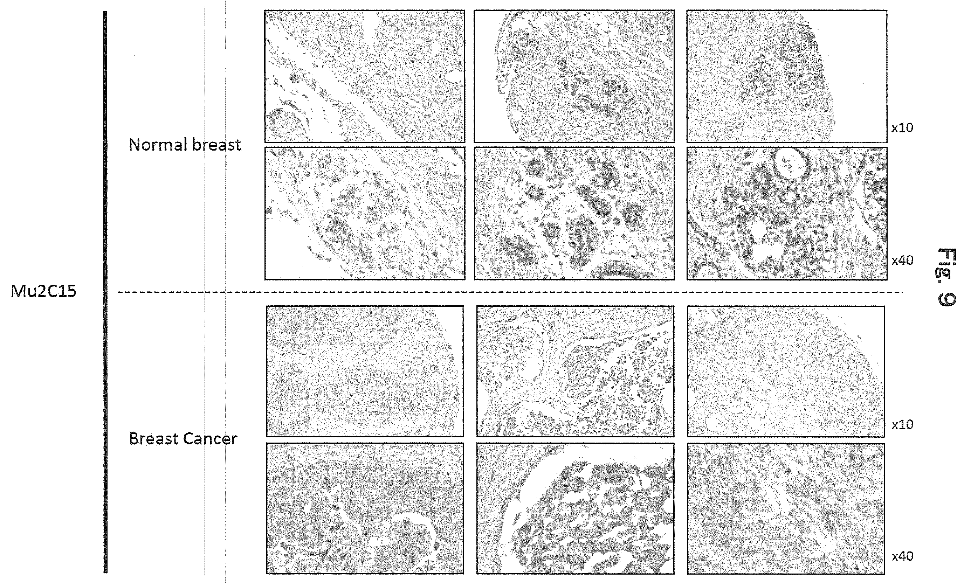

Immunostaining was conducted in the same manner as shown in Example 3. (1) above. The results of the experiment are shown in Figure 9.

In the case of normal tissue, no staining was observed or glandular cells were positive in some samples. In contrast, in the case of breast cancer tissues, strongly positively stained tissues were already seen in stage I of cancer progression, and 80% or more of the samples used for measurement were found to show strongly positive or positive results. This indicated that LGR6 was already expressed in early stages of breast cancer and 90% or more of the breast cancer tissue samples used for measurement showed positive or strongly positive results, and that LGR6 expression was able to be detected with high sensitivity when using the Mu2C15 antibody.

These results indicated that LGR6 was a marker available for use in cancer diagnosis at least for both colorectal cancer and breast cancer. Moreover, it was also indicated that the antibody of the present invention was useful for detection and diagnosis of at least colorectal cancer and breast cancer.

### (3) Comparison with HercepTest II

Her2 expression is used as an index to determine whether a patient should receive treatment with Herceptin, which is an existing molecular targeted drug. It is said that Her2-positive patients account for only 15% to 20% of all breast cancer patients. Her2 testing is conducted in the case of invasive breast cancer. For identification of Her2 *expression, in vitro* diagnostic agents are commercially available from several manufacturers. Among them, HercepTest II (DAKO) was used to analyze the positive rate of Her2 in the same tissue array slides as used in Example 3. (2) above, and a comparison was made with the positive rate of LGR6.

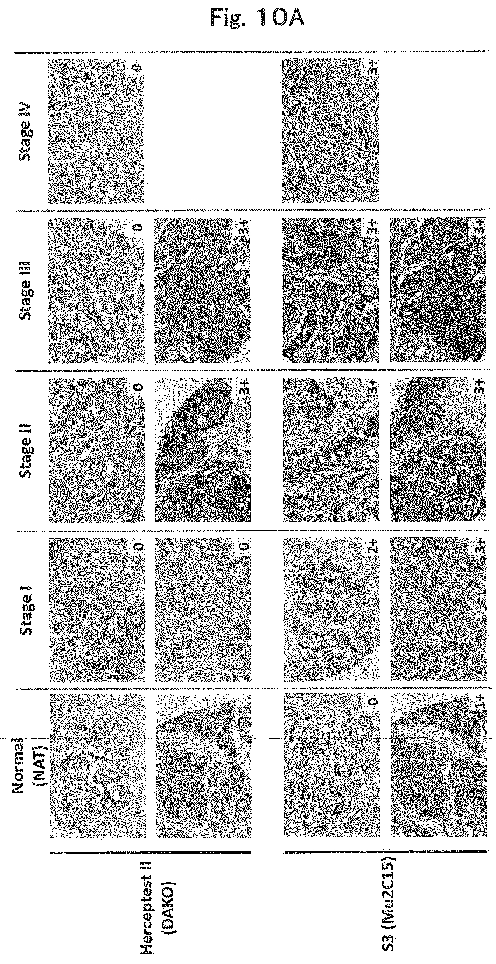

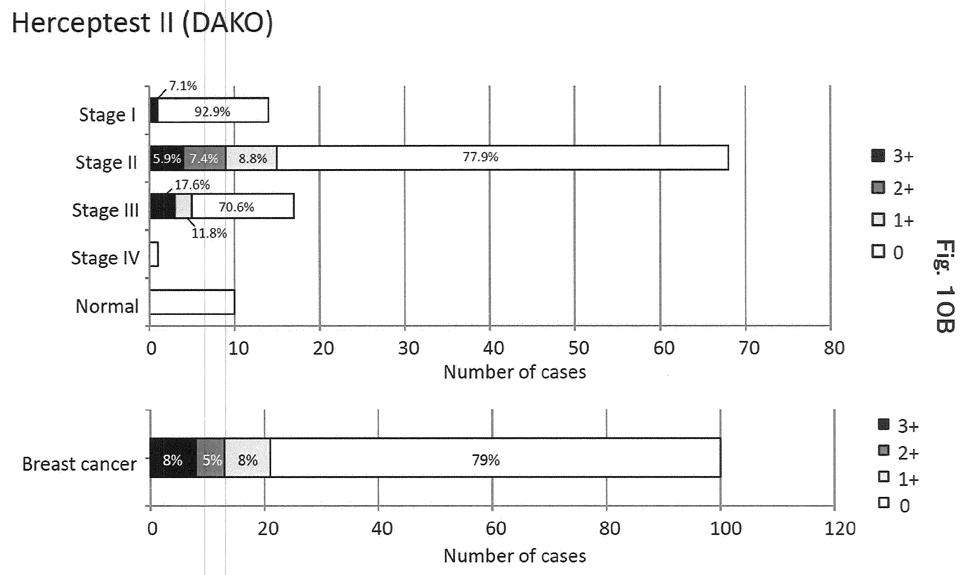

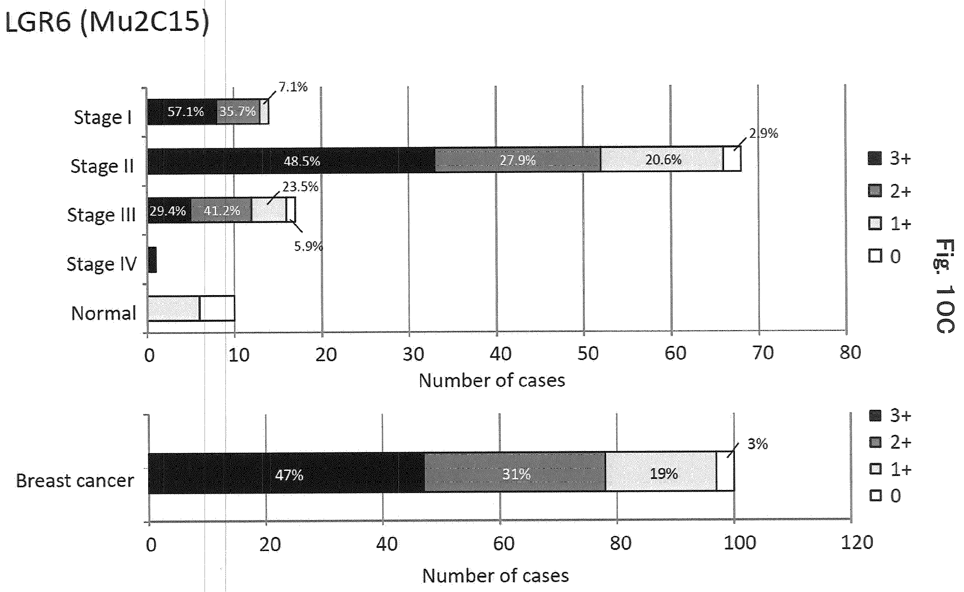

The results of immunostaining in breast cancer tissues at different stages are shown in Figure 10A. Moreover, the results analyzed for the positive rate of Her2 and the positive rate of LGR6 are shown in Figure 10B and Figure 10C, respectively.

In the case of HercepTest II, among the samples used for measurement, those detected as being strongly positive (3+) and positive (2+) accounted for 13% (Figure 10B, lower panel), whereas such samples accounted for 78% upon detection with the anti-LGR6 antibody (Figure 10C, lower panel). Further, expression was analyzed for each stage. In stage I cases, only 7.1% were able to be detected by HercepTest II (Figure 10B, upper panel), whereas 90% or more were able to be detected when using the anti-LGR6 antibody (Figure 10C, upper panel).

These results indicated that breast cancer was able to be detected at stage I with high sensitivity when using the antibody of the present invention, and that the reagent of the present invention comprising this antibody was very effective for detection of breast cancer at early stages.

Moreover, it was also indicated that the grade of malignancy of breast cancer was able to be evaluated when using the antibody of the present invention.

As a monoclonal antibody binding to the extracellular domain of LGR6, the antibody under clone No. 2A3 (Abcam) is known. The titers of Mu2C15 and 2A3 were compared using colorectal cancer tissues.

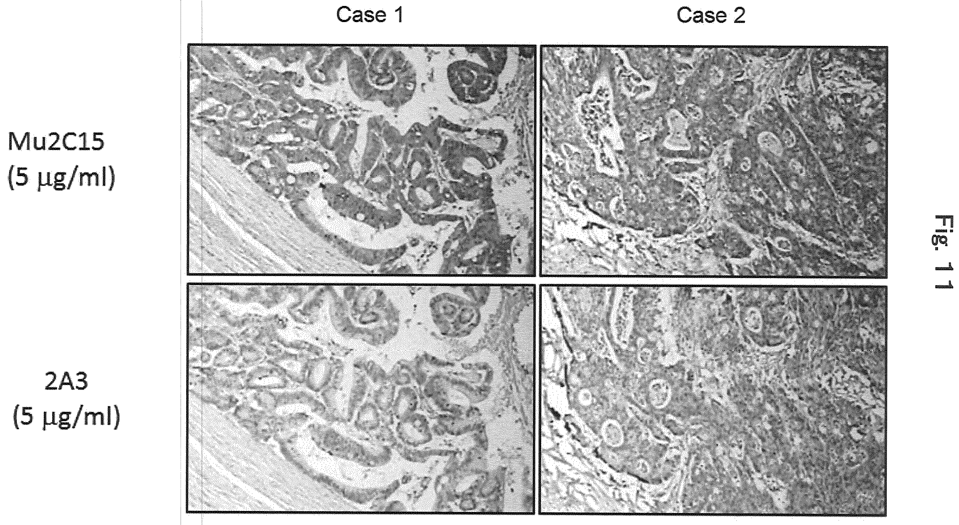

Immunostaining was conducted in the same manner as shown in Example 3. (1) above, such that the antibody concentration was adjusted to 5 µg/mL. The results of the experiment are shown in Figure 11.

In colorectal cancer tissues from two cases, clear staining was observed when using Mu2C15, whereas no staining was observed when using 2A3.

This result indicated that the antibody of the present invention had a higher affinity and hence allowed cancer detection with higher sensitivity than the existing monoclonal antibody binding to the extracellular domain of LGR6.

### Example 4. Construction of LGR6 detection system

In some membrane proteins such as EGFR, it is known that their extracellular domains are released into blood (Molecular Cancer, Vol. 9, 166, 2010). Likewise, in the case of LGR6, it is also expected that cancer diagnosis is achieved by detection of an LGR6 extracellular domain segment in blood. Moreover, it is also assumed that the LGR6 protein is extracted and detected in a denatured state from feces. Thus, the monoclonal antibodies produced by the six lines of hybridoma cells obtained in Example 1. (7) were used to construct a method for detecting the extracellular domain of LGR6 with high sensitivity by sandwich ELISA.

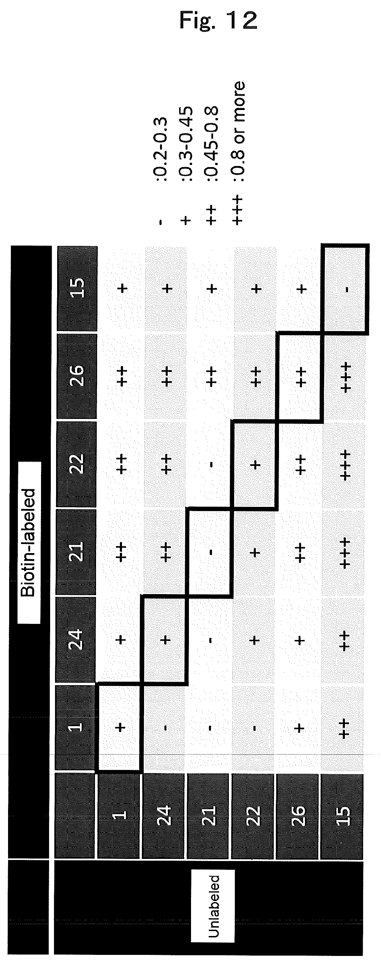

First, the monoclonal antibodies were labeled with biotin using a Biotin Labeling Kit-NH2 (Dojindo Laboratories, Japan) in accordance with the protocols attached thereto. Then, the monoclonal antibodies in an unlabeled state were dispensed into 96-well ELISA plates (Nunc) in an amount of 500 ng per well and immobilized on the plate surface by being allowed to stand overnight at 4°C. Then, after the plates were washed three times with 350 µL of PBS(-) containing 0.05% Tween 20 (hereinafter abbreviated as PBS-T), PBS-T containing 1% skimmed milk was dispensed in a volume of 300 µL per well, followed by blocking for 1 hour at room temperature. After washing with PBS-T, ECD2 was added at a concentration of 50 ng/mL and reacted for 1 hour at room temperature. After washing three times with 350 µL of PBS-T, the biotinylated antibodies prepared at a concentration of 0.5 mg/mL were each added and reacted for 1 hour at room temperature. After washing three times with 350 µL of PBS-T, a 5000-fold dilution of peroxidase-labeled streptavidin (Vector Labs) in TBS-T was added in 100 µL volumes and reacted for 1 hour at room temperature. The subsequent detection was conducted in the same manner as shown in Example 1. (6). The experimental results obtained are shown in Figure 12.

The experimental results indicated that when Mu2C 15 was immobilized and Mu2C21 or Mu2C22 was labeled with biotin for detection, ECD2 was able to be detected with low background and with high sensitivity.

Further, the combination of these antibodies was used to analyze the detection limit (critical value) for ECD2.

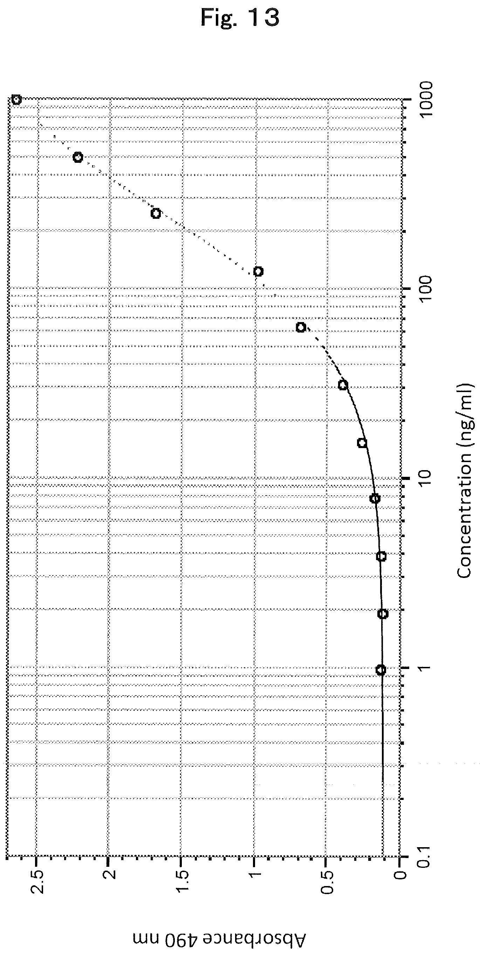

The experimental results obtained are shown in Figure 13. As a result, it is indicated that a concentration greater than approximately 20 ng/mL would be sufficient for detection.

This result indicated that cancer was able to be detected with high accuracy and high sensitivity from biological samples (e.g., blood samples, feces) when using the antibody of the present invention.

### INDUSTRIAL APPLICABILITY

The present invention enables the detection of cancer with higher sensitivity than conventional antibodies.

### Sequence Listing Free Text

SEQ ID NOs: 7 to 12: synthetic peptide
SEQ ID NOs: 13 to 21: synthetic DNA
SEQ ID NO: 22: synthetic DNA
SEQ ID NO: 23: synthetic peptide
SEQ ID NO: 24: synthetic DNA
SEQ ID NO: 25: synthetic peptide
SEQ ID NO: 26: synthetic DNA
SEQ ID NO: 27: synthetic peptide
SEQ ID NO: 28: synthetic DNA
SEQ ID NO: 29: synthetic peptide
SEQ ID NO: 30: synthetic DNA
SEQ ID NO: 31: synthetic peptide
SEQ ID NO: 32: synthetic DNA
SEQ ID NO: 33: synthetic peptide
SEQ ID NO: 34: synthetic DNA
SEQ ID NO: 35: synthetic peptide
SEQ ID NO: 36: synthetic DNA
SEQ ID NO: 37: synthetic peptide
SEQ ID NO: 38: synthetic DNA
SEQ ID NO: 39: synthetic peptide
SEQ ID NO: 40: synthetic DNA
SEQ ID NO: 41: synthetic peptide
SEQ ID NOs: 42 and 43: synthetic DNA

## Claims

1. A reagent for detection or diagnosis of cancer, which comprises a monoclonal antibody against leucine-rich repeat containing G protein-coupled receptor 6 (LGR6) or a fragment of the antibody.

2. The reagent according to claim 1, wherein the antibody or fragment thereof binds to the extracellular region of leucine-rich repeat containing G protein-coupled receptor 6 (LGR6).

3. The reagent according to claim 2, wherein the extracellular region comprises the amino acid sequence shown in SEQ ID NO: 7, 8, 9 or 10.

4. The reagent according to claim 1, wherein the cancer is at least one selected from the group consisting of colorectal cancer, breast cancer, uterine cancer, gastric cancer, thyroid cancer, pancreatic cancer, brain tumor, cervical cancer, esophageal cancer, tongue cancer, lung cancer, small intestinal cancer, duodenal cancer, bladder cancer, kidney cancer, liver cancer, prostate cancer, uterine cervical cancer, ovarian cancer, gallbladder cancer, pharyngeal cancer, sarcoma, melanoma, leukemia, lymphoma and multiple myeloma.

5. The reagent according to claim 1, wherein the cancer is at least one selected from the group consisting of colorectal cancer, breast cancer, uterine cancer, gastric cancer, thyroid cancer, pancreatic cancer and leukemia.

6. The reagent according to claim 1, wherein the cancer is colorectal cancer or breast cancer.

7. A method for detection of cancer, which comprises the step of contacting a monoclonal antibody against leucine-rich repeat containing G protein-coupled receptor 6 (LGR6) or a fragment of the antibody with a biological sample taken from a subject to thereby detect LGR6 in the sample.

8. A kit for detection of cancer, which comprises a monoclonal antibody against leucine-rich repeat containing G protein-coupled receptor 6 (LGR6) or a fragment of the antibody.

9. A method for diagnosis of cancer, which comprises the step of contacting a monoclonal antibody against leucine-rich repeat containing G protein-coupled receptor 6 (LGR6) or a fragment of the antibody with a biological sample taken from a subject, and detecting LGR6 in the sample.

10. A monoclonal antibody against leucine-rich repeat containing G protein-coupled receptor 6 (LGR6) or a fragment of the antibody for use in the detection or diagnosis of cancer.


SEQUENCE LISTING

<110> Order-made Medical Research Inc.

<120> A reagent for detecting or diagnosing cancer comprising an
      anti-LGR6 antibody

<130> G1114WO

<150> JP 2013-114150
<151> 2013-05-30

<160> 43

<170> PatentIn version 3.5

<210> 1
<211> 3623
<212> DNA
<213> Mus musculus


<220>
<221> CDS
<222> (137)..(3040)

<400> 1
aagtcgagcg ggggcgttgc ccaccgacgg cgcagccctc gggcccgccc gggaccagga      60

ggtgagccgc gcgcgcacaa ctccgtgcgc tcgcccgtct gagcgcccgc caggtgcccc     120

gcagcccgcc gccgag atg cac agc ccg cct ggg ctc ctg gcg ctg tgg ctt     172
                  Met His Ser Pro Pro Gly Leu Leu Ala Leu Trp Leu
                  1               5                   10

tgc gct gtg ctg tgc gca tcg gcg cgc gcg ggc agc gac ccc cag cct       220
Cys Ala Val Leu Cys Ala Ser Ala Arg Ala Gly Ser Asp Pro Gln Pro
        15                  20                  25

ggc ccg ggg cgt ccc gcc tgc ccg gct ccc tgc cac tgc cag gag gac       268
Gly Pro Gly Arg Pro Ala Cys Pro Ala Pro Cys His Cys Gln Glu Asp
    30                  35                  40

ggc atc atg ctg tcc gct gac tgc tcc gag ctc ggg ctc tca gtg gtg       316
Gly Ile Met Leu Ser Ala Asp Cys Ser Glu Leu Gly Leu Ser Val Val
45                  50                  55                  60

cct gcg gac ctg gac ccc ctg acg gct tac cta gac ctc agt atg aac       364
Pro Ala Asp Leu Asp Pro Leu Thr Ala Tyr Leu Asp Leu Ser Met Asn

                65                  70                  75

aac ctc acg gag ctt cag ccg ggt ctc ttc cac cac ctg cgc ttc ctg 412
Asn Leu Thr Glu Leu Gln Pro Gly Leu Phe His His Leu Arg Phe Leu
            80                  85                  90

gag gag ctg cgg ctc tca ggg aac cac ctc tca cac atc ccg gga cag 460
Glu Glu Leu Arg Leu Ser Gly Asn His Leu Ser His Ile Pro Gly Gln
        95                  100                 105

gca ttc tct ggc ctc cac agc ctc aaa att cta atg ctg cag agc aac 508
Ala Phe Ser Gly Leu His Ser Leu Lys Ile Leu Met Leu Gln Ser Asn
    110                 115                 120

cag ctc cgt ggg atc cca gca gag gca cta tgg gag ctg ccc agc ctg 556
Gln Leu Arg Gly Ile Pro Ala Glu Ala Leu Trp Glu Leu Pro Ser Leu
125                 130                 135                 140

cag tcg ctg cgc cta gat gct aat ctc atc tcc ctg gtc cct gag aga 604
Gln Ser Leu Arg Leu Asp Ala Asn Leu Ile Ser Leu Val Pro Glu Arg
                145                 150                 155

agc ttt gag ggg ctc tcc tcc ctc cgc cac ctc tgg ctg gat gac aat 652
Ser Phe Glu Gly Leu Ser Ser Leu Arg His Leu Trp Leu Asp Asp Asn
            160                 165                 170

gca ctc acc gag atc ccc gtc aga gct ctc aac aac ctt cct gcc cta 700
Ala Leu Thr Glu Ile Pro Val Arg Ala Leu Asn Asn Leu Pro Ala Leu
        175                 180                 185

caa gcc atg acc ttg gct ctc aac cat atc cgc cac atc cct gac tat 748
Gln Ala Met Thr Leu Ala Leu Asn His Ile Arg His Ile Pro Asp Tyr
    190                 195                 200

gcc ttc cag aac ctc acc agt ctt gtg gtg ctg cat cta cat aac aac 796
Ala Phe Gln Asn Leu Thr Ser Leu Val Val Leu His Leu His Asn Asn
205                 210                 215                 220

cgc atc cag cat gtg ggg acc cac agc ttc gag ggg ctg cac aat ctg 844
Arg Ile Gln His Val Gly Thr His Ser Phe Glu Gly Leu His Asn Leu
                225                 230                 235

gag aca cta gac ctg aac tat aat gag ctg cag gag ttc ccc ttg gct 892
Glu Thr Leu Asp Leu Asn Tyr Asn Glu Leu Gln Glu Phe Pro Leu Ala
            240                 245                 250

atc cgg acc ctg ggc agg ctg cag gaa ttg ggt ttc cat aac aac aac 940
Ile Arg Thr Leu Gly Arg Leu Gln Glu Leu Gly Phe His Asn Asn Asn
        255                 260                 265

atc aag gct atc cca gag aaa gcc ttc atg ggc agc cct ctc ctg cag 988
Ile Lys Ala Ile Pro Glu Lys Ala Phe Met Gly Ser Pro Leu Leu Gln
    270                 275                 280

aca ata cat ttt tat gac aac cca atc cag ttt gtg gga agg tca gca 1036
Thr Ile His Phe Tyr Asp Asn Pro Ile Gln Phe Val Gly Arg Ser Ala
285                 290                 295                 300

ttc cag tac ctg tct aaa ctg cat acg cta tct ttg aat ggt gcc act 1084
Phe Gln Tyr Leu Ser Lys Leu His Thr Leu Ser Leu Asn Gly Ala Thr
                305                 310                 315

gat atc caa gag ttc cca gac ctc aaa ggc acc act agc ctg gag atc 1132
Asp Ile Gln Glu Phe Pro Asp Leu Lys Gly Thr Thr Ser Leu Glu Ile
            320                 325                 330

ctg acc ctg acc cgt gcg ggc atc aga ctg ctc cca ccg gga gtg tgc 1180
Leu Thr Leu Thr Arg Ala Gly Ile Arg Leu Leu Pro Pro Gly Val Cys
        335                 340                 345

caa cag ctg cct agg ctc cga atc ctg gag ctg tct cat aat cag atc 1228
Gln Gln Leu Pro Arg Leu Arg Ile Leu Glu Leu Ser His Asn Gln Ile
    350                 355                 360

gag gag tta ccc agc ctg cac agg tgt cag aag ctg gag gaa att ggc 1276
Glu Glu Leu Pro Ser Leu His Arg Cys Gln Lys Leu Glu Glu Ile Gly
365                 370                 375                 380

ctc cga cat aac cgg atc aag gaa att ggt gca gat acc ttc agc cag 1324
Leu Arg His Asn Arg Ile Lys Glu Ile Gly Ala Asp Thr Phe Ser Gln
                385                 390                 395

ctg ggc tcc ttg caa gct tta gac ctg agt tgg aat gcc atc cgt gcc 1372
Leu Gly Ser Leu Gln Ala Leu Asp Leu Ser Trp Asn Ala Ile Arg Ala
            400                 405                 410

atc cac cct gag gct ttc tca acc ctt cga tcc ttg gtt aag ctg gac 1420
Ile His Pro Glu Ala Phe Ser Thr Leu Arg Ser Leu Val Lys Leu Asp
        415                 420                 425

ctg act gac aac cag ctg acc aca ctg ccc ctg gct ggg ctg gga ggc 1468
Leu Thr Asp Asn Gln Leu Thr Thr Leu Pro Leu Ala Gly Leu Gly Gly
    430                 435                 440

ctg atg cac ctg aag ctc aaa ggg aac ttg gcc ctg tct cag gcc ttc 1516
Leu Met His Leu Lys Leu Lys Gly Asn Leu Ala Leu Ser Gln Ala Phe
445                 450                 455                 460

tcc aag gac agt ttc cca aaa ctg agg atc ctg gag gtg ccc tac gcc 1564
Ser Lys Asp Ser Phe Pro Lys Leu Arg Ile Leu Glu Val Pro Tyr Ala
                465                 470                 475

tac cag tgc tgt gcc tac ggc atc tgt gcc agc ttc ttc aag acc tct 1612
Tyr Gln Cys Cys Ala Tyr Gly Ile Cys Ala Ser Phe Phe Lys Thr Ser
            480                 485                 490

ggg cag tgg cag gcc gag gac ttt cat cca gag gaa gag gag gca cca 1660
Gly Gln Trp Gln Ala Glu Asp Phe His Pro Glu Glu Glu Glu Ala Pro
        495                 500                 505

aag agg ccc ctg ggt ctc ctt gct gga caa gct gag aac cac tat gac 1708
Lys Arg Pro Leu Gly Leu Leu Ala Gly Gln Ala Glu Asn His Tyr Asp
    510                 515                 520

cta gac ctg gat gag ctc cag atg ggg aca gag gac tca aag cca cac 1756
Leu Asp Leu Asp Glu Leu Gln Met Gly Thr Glu Asp Ser Lys Pro His
525                 530                 535                 540

ccc agt gtc cag tgc agc cct gtt cca ggc ccc ttc aag ccc tgc gag 1804
Pro Ser Val Gln Cys Ser Pro Val Pro Gly Pro Phe Lys Pro Cys Glu
                545                 550                 555

cac ctc ttt gag agc tgg ggc atc cgc ctt gct gtg tgg gcc atc gtg 1852
His Leu Phe Glu Ser Trp Gly Ile Arg Leu Ala Val Trp Ala Ile Val
            560                 565                 570

tta ctc tcc gta ctc tgt aac ggg ctg gtg ctg ctg aca gtc ttt gcc 1900
Leu Leu Ser Val Leu Cys Asn Gly Leu Val Leu Leu Thr Val Phe Ala
        575                 580                 585

agc gga ccc agc ccg ctg tcc ccc gtc aag ctt gtg gtg ggt gcg atg 1948
Ser Gly Pro Ser Pro Leu Ser Pro Val Lys Leu Val Val Gly Ala Met
    590                 595                 600

gca ggc gcc aac gcc ctg tcg ggc att tcc tgt ggt ctc ctg gcc tcg 1996
Ala Gly Ala Asn Ala Leu Ser Gly Ile Ser Cys Gly Leu Leu Ala Ser
605                 610                 615                 620

gtg gac gcc ttg acc tat ggt cag ttc gct gag tat gga gcc cgc tgg 2044
Val Asp Ala Leu Thr Tyr Gly Gln Phe Ala Glu Tyr Gly Ala Arg Trp
                625                 630                 635

gag agc ggt ctg ggc tgc cag gct acg ggc ttc ctg gct gtc ctg ggt 2092
Glu Ser Gly Leu Gly Cys Gln Ala Thr Gly Phe Leu Ala Val Leu Gly
            640                 645                 650

tca gag gcg tcg gtg ctg ctg ctc aca ctg gcg gcc gtg cag tgc agc 2140

Ser Glu Ala Ser Val Leu Leu Leu Thr Leu Ala Ala Val Gln Cys Ser
        655                 660                 665

atc tcc gtg acc tgc gtc cga gcc tac ggg aag gcg ccg tcg cct ggc 2188
Ile Ser Val Thr Cys Val Arg Ala Tyr Gly Lys Ala Pro Ser Pro Gly
    670                 675                 680

agc gtc cgc gca ggc gca ctg gga tgc ctg gcg ctg gcc ggg ctg gcc 2236
Ser Val Arg Ala Gly Ala Leu Gly Cys Leu Ala Leu Ala Gly Leu Ala
685                 690                 695                 700

gca gca ctg ccg ctg gcc tcg gtg gga gag tat ggc gcc tcc cca ctc 2284
Ala Ala Leu Pro Leu Ala Ser Val Gly Glu Tyr Gly Ala Ser Pro Leu
                705                 710                 715

tgc ctg ccc tac gcc cca ccc gag ggc cgg ccg gct gcc ctg ggc ttc 2332
Cys Leu Pro Tyr Ala Pro Pro Glu Gly Arg Pro Ala Ala Leu Gly Phe
            720                 725                 730

gct gta gcc ctg gtg atg atg aac tcg ctc tgc ttc ctg gtg gtg gcc 2380
Ala Val Ala Leu Val Met Met Asn Ser Leu Cys Phe Leu Val Val Ala
        735                 740                 745

ggc gcc tac atc aag ctc tac tgt gac ctg cca cgg ggt gac ttt gag 2428
Gly Ala Tyr Ile Lys Leu Tyr Cys Asp Leu Pro Arg Gly Asp Phe Glu
    750                 755                 760

gcc gtg tgg gac tgc gcc atg gtg cgc cac gtg gcc tgg ctc atc ttt 2476
Ala Val Trp Asp Cys Ala Met Val Arg His Val Ala Trp Leu Ile Phe
765                 770                 775                 780

gca gat ggc ctc ctc tac tgc ccc gtg gcc ttc ctc agc ttt gcc tct 2524
Ala Asp Gly Leu Leu Tyr Cys Pro Val Ala Phe Leu Ser Phe Ala Ser
                785                 790                 795

atg ctg ggc ctc ttc cct gtc acc ccc gag gct gtc aag tca gtc ctt 2572
Met Leu Gly Leu Phe Pro Val Thr Pro Glu Ala Val Lys Ser Val Leu
            800                 805                 810

ctg gtg gtg ctg cct ctg cct gcc tgc ctc aac cca ctg ctc tac ctg 2620
Leu Val Val Leu Pro Leu Pro Ala Cys Leu Asn Pro Leu Leu Tyr Leu
        815                 820                 825

ctc ttc aac cct cac ttc cgg gat gac ctt cgg cgg ctc tgg cca agc 2668
Leu Phe Asn Pro His Phe Arg Asp Asp Leu Arg Arg Leu Trp Pro Ser
    830                 835                 840

cct cgg tcc cca ggg ccc cta gcc tac gct gca gcc ggt gag ctg gag 2716
Pro Arg Ser Pro Gly Pro Leu Ala Tyr Ala Ala Ala Gly Glu Leu Glu

845                 850                 855                 860

aag agc tcc tgc gac tcc acc caa gcg ctg gtg gct ttc tca gat gtg          2764
Lys Ser Ser Cys Asp Ser Thr Gln Ala Leu Val Ala Phe Ser Asp Val
                865                 870                 875

gat ctt att ctg gaa gct tct gag gct ggg cag cct cct ggg cta gag          2812
Asp Leu Ile Leu Glu Ala Ser Glu Ala Gly Gln Pro Pro Gly Leu Glu
            880                 885                 890

acc tat ggc ttc cct tca gtg acc ctc atc tcc cga cat cag ccg ggg          2860
Thr Tyr Gly Phe Pro Ser Val Thr Leu Ile Ser Arg His Gln Pro Gly
        895                 900                 905

gct acc agg ctg gag gga aac cat ttt gta gag tct gat gga acc aag          2908
Ala Thr Arg Leu Glu Gly Asn His Phe Val Glu Ser Asp Gly Thr Lys
    910                 915                 920

ttt ggg aac cca caa cct ccc atg aag gga gaa ctg ctg ctg aag gca          2956
Phe Gly Asn Pro Gln Pro Pro Met Lys Gly Glu Leu Leu Leu Lys Ala
925                 930                 935                 940

gag gga gcc act ttg gca ggc tgt ggc tct tcc gtg ggt gga gcc ctc          3004
Glu Gly Ala Thr Leu Ala Gly Cys Gly Ser Ser Val Gly Gly Ala Leu
                945                 950                 955

tgg ccc tct ggc tct ctc ttt gcc tct cac ttg taa atatccctct               3050
Trp Pro Ser Gly Ser Leu Phe Ala Ser His Leu
            960                 965

ctgtttgtcc tcttcccgtc caatgatggc tgcttataaa agaaagacaa ctccaactcc        3110

atagcaagat ggccaacacc tctgactcca ttgttctctc tccacgaccc ctaaccaatg        3170

agtgcttcca agtcttgctt tgtcttggcc ttcagcttca ctttcaccct gggccttctc        3230

tgtccaatcc aatacttctg acagaggcct gggaaatttg cataggagaa aggagaaaag        3290

caaaagacag tgaaggttat ggggccctga cagagccatg atcagtaagt gcagagtgat        3350

ggggaggtct cacagagcat gacactggaa gacaactacc aaagacattg gagagtctcc        3410

cctgtgacat atagaatata aaatgtgttt tgtgttccat taatcttgac ctatgctgtg        3470

ccaaagtgct tcctgttaaa atacactttg gaagacattg catattatgt ccctttctta        3530

gagtcatggg gtcatgttag tgtctaaaag gtcctgagaa ttaggacttt ttggcttaat        3590

tactacagca attaaactta attatttgat gat                                     3623

<210> 2
<211> 967
<212> PRT
<213> Mus musculus

<400> 2
   Met His Ser Pro Pro Gly Leu Leu Ala Leu Trp Leu Cys Ala Val Leu
   1               5                   10                  15
   Cys Ala Ser Ala Arg Ala Gly Ser Asp Pro Gln Pro Gly Pro Gly Arg
               20                  25                  30
   Pro Ala Cys Pro Ala Pro Cys His Cys Gln Glu Asp Gly Ile Met Leu
           35                  40                  45
   Ser Ala Asp Cys Ser Glu Leu Gly Leu Ser Val Val Pro Ala Asp Leu
       50                  55                  60
   Asp Pro Leu Thr Ala Tyr Leu Asp Leu Ser Met Asn Asn Leu Thr Glu
   65                  70                  75                  80
   Leu Gln Pro Gly Leu Phe His His Leu Arg Phe Leu Glu Glu Leu Arg
                   85                  90                  95
   Leu Ser Gly Asn His Leu Ser His Ile Pro Gly Gln Ala Phe Ser Gly
               100                 105                 110
   Leu His Ser Leu Lys Ile Leu Met Leu Gln Ser Asn Gln Leu Arg Gly
           115                 120                 125
   Ile Pro Ala Glu Ala Leu Trp Glu Leu Pro Ser Leu Gln Ser Leu Arg
       130                 135                 140
   Leu Asp Ala Asn Leu Ile Ser Leu Val Pro Glu Arg Ser Phe Glu Gly
   145                 150                 155                 160
   Leu Ser Ser Leu Arg His Leu Trp Leu Asp Asp Asn Ala Leu Thr Glu
   165 170 175
   Ile Pro Val Arg Ala Leu Asn Asn Leu Pro Ala Leu Gln Ala Met Thr
   180 185 190
   Leu Ala Leu Asn His Ile Arg His Ile Pro Asp Tyr Ala Phe Gln Asn
   195 200 205
   Leu Thr Ser Leu Val Val Leu His Leu His Asn Asn Arg Ile Gln His
   210 215 220
   Val Gly Thr His Ser Phe Glu Gly Leu His Asn Leu Glu Thr Leu Asp
   225 230 235 240
   Leu Asn Tyr Asn Glu Leu Gln Glu Phe Pro Leu Ala Ile Arg Thr Leu
   245 250 255
   Gly Arg Leu Gln Glu Leu Gly Phe His Asn Asn Asn Ile Lys Ala Ile
   260 265 270
   Pro Glu Lys Ala Phe Met Gly Ser Pro Leu Leu Gln Thr Ile His Phe
   275 280 285
   Tyr Asp Asn Pro Ile Gln Phe Val Gly Arg Ser Ala Phe Gln Tyr Leu
   290 295 300
   Ser Lys Leu His Thr Leu Ser Leu Asn Gly Ala Thr Asp Ile Gln Glu
   305 310 315 320
   Phe Pro Asp Leu Lys Gly Thr Thr Ser Leu Glu Ile Leu Thr Leu Thr
   325 330 335
   Arg Ala Gly Ile Arg Leu Leu Pro Pro Gly Val Cys Gln Gln Leu Pro
   340 345 350
   Arg Leu Arg Ile Leu Glu Leu Ser His Asn Gln Ile Glu Glu Leu Pro
   355 360 365
   Ser Leu His Arg Cys Gln Lys Leu Glu Glu Ile Gly Leu Arg His Asn
   370 375 380
   Arg Ile Lys Glu Ile Gly Ala Asp Thr Phe Ser Gln Leu Gly Ser Leu
   385 390 395 400
   Gln Ala Leu Asp Leu Ser Trp Asn Ala Ile Arg Ala Ile His Pro Glu
   405 410 415
   Ala Phe Ser Thr Leu Arg Ser Leu Val Lys Leu Asp Leu Thr Asp Asn
   420 425 430
   Gln Leu Thr Thr Leu Pro Leu Ala Gly Leu Gly Gly Leu Met His Leu
   435 440 445
   Lys Leu Lys Gly Asn Leu Ala Leu Ser Gln Ala Phe Ser Lys Asp Ser
   450 455 460
   Phe Pro Lys Leu Arg Ile Leu Glu Val Pro Tyr Ala Tyr Gln Cys Cys
   465 470 475 480
   Ala Tyr Gly Ile Cys Ala Ser Phe Phe Lys Thr Ser Gly Gln Trp Gln
   485 490 495
   Ala Glu Asp Phe His Pro Glu Glu Glu Glu Ala Pro Lys Arg Pro Leu
   500 505 510
   Gly Leu Leu Ala Gly Gln Ala Glu Asn His Tyr Asp Leu Asp Leu Asp
   515 520 525
   Glu Leu Gln Met Gly Thr Glu Asp Ser Lys Pro His Pro Ser Val Gln
   530 535 540
   Cys Ser Pro Val Pro Gly Pro Phe Lys Pro Cys Glu His Leu Phe Glu
   545 550 555 560
   Ser Trp Gly Ile Arg Leu Ala Val Trp Ala Ile Val Leu Leu Ser Val
   565 570 575
   Leu Cys Asn Gly Leu Val Leu Leu Thr Val Phe Ala Ser Gly Pro Ser
   580 585 590
   Pro Leu Ser Pro Val Lys Leu Val Val Gly Ala Met Ala Gly Ala Asn
   595 600 605
   Ala Leu Ser Gly Ile Ser Cys Gly Leu Leu Ala Ser Val Asp Ala Leu
   610 615 620
   Thr Tyr Gly Gln Phe Ala Glu Tyr Gly Ala Arg Trp Glu Ser Gly Leu
   625 630 635 640
   Gly Cys Gln Ala Thr Gly Phe Leu Ala Val Leu Gly Ser Glu Ala Ser
   645 650 655
   Val Leu Leu Leu Thr Leu Ala Ala Val Gln Cys Ser Ile Ser Val Thr
   660 665 670
   Cys Val Arg Ala Tyr Gly Lys Ala Pro Ser Pro Gly Ser Val Arg Ala
   675 680 685
   Gly Ala Leu Gly Cys Leu Ala Leu Ala Gly Leu Ala Ala Ala Leu Pro
   690 695 700
   Leu Ala Ser Val Gly Glu Tyr Gly Ala Ser Pro Leu Cys Leu Pro Tyr
   705 710 715 720
   Ala Pro Pro Glu Gly Arg Pro Ala Ala Leu Gly Phe Ala Val Ala Leu
   725 730 735
   Val Met Met Asn Ser Leu Cys Phe Leu Val Val Ala Gly Ala Tyr Ile
   740 745 750
   Lys Leu Tyr Cys Asp Leu Pro Arg Gly Asp Phe Glu Ala Val Trp Asp
   755 760 765
   Cys Ala Met Val Arg His Val Ala Trp Leu Ile Phe Ala Asp Gly Leu
   770 775 780
   Leu Tyr Cys Pro Val Ala Phe Leu Ser Phe Ala Ser Met Leu Gly Leu
   785 790 795 800
   Phe Pro Val Thr Pro Glu Ala Val Lys Ser Val Leu Leu Val Val Leu
   805 810 815
   Pro Leu Pro Ala Cys Leu Asn Pro Leu Leu Tyr Leu Leu Phe Asn Pro
   820 825 830
   His Phe Arg Asp Asp Leu Arg Arg Leu Trp Pro Ser Pro Arg Ser Pro
   835 840 845
   Gly Pro Leu Ala Tyr Ala Ala Ala Gly Glu Leu Glu Lys Ser Ser Cys
   850 855 860
   Asp Ser Thr Gln Ala Leu Val Ala Phe Ser Asp Val Asp Leu Ile Leu
   865 870 875 880
   Glu Ala Ser Glu Ala Gly Gln Pro Pro Gly Leu Glu Thr Tyr Gly Phe
   885 890 895
   Pro Ser Val Thr Leu Ile Ser Arg His Gln Pro Gly Ala Thr Arg Leu
   900 905 910
   Glu Gly Asn His Phe Val Glu Ser Asp Gly Thr Lys Phe Gly Asn Pro
   915 920 925
   Gln Pro Pro Met Lys Gly Glu Leu Leu Leu Lys Ala Glu Gly Ala Thr
   930 935 940
   Leu Ala Gly Cys Gly Ser Ser Val Gly Gly Ala Leu Trp Pro Ser Gly
   945                 950                 955                 960
   Ser Leu Phe Ala Ser His Leu
                   965

<210>  3
<211>  3673
<212>  DNA
<213>  Rattus norvegicus

<220>
<221>  CDS
<222>  (131)..(3028)

<400>  3
   agcgggggcg gtccccaccg acggcgcagc cctcgggccc gcccggaacc aggaggtgag      60
   ccgcgcgctc acagctccgt gcgcccgccc gtcccagcgc ccgccaggtg ccccaaagcc     120
   tgccgccgag atg cac agc tcg ccg ttg ctc ctg gcg ctg tgt ctt tgc       169
              Met His Ser Ser Pro Leu Leu Leu Ala Leu Cys Leu Cys
              1               5                   10
   gtt gtg ctg tgc gca ttg gcg cgc gcg ggc agc gac ccc cag cct ggc      217
   Val Val Leu Cys Ala Leu Ala Arg Ala Gly Ser Asp Pro Gln Pro Gly
       15                  20                  25
   ccg ggg cgt ccc gcc tgc ccc gct ccc tgc cac tgc cag gag gat ggc      265
   Pro Gly Arg Pro Ala Cys Pro Ala Pro Cys His Cys Gln Glu Asp Gly
   30                  35                  40                  45
   atc atg ctg tca gct gac tgc tcc gag ctc ggt ctc tca gag gtg cca      313
   Ile Met Leu Ser Ala Asp Cys Ser Glu Leu Gly Leu Ser Glu Val Pro
                   50                  55                  60
   gcg gac ctg gac ccc ctg acg gct tac cta gac ctc agt atg aac aac      361
   Ala Asp Leu Asp Pro Leu Thr Ala Tyr Leu Asp Leu Ser Met Asn Asn
               65                  70                  75
   ctc acg gag ctt cag ccg ggt ctc ttc cac cac ctg cgc ttc ctg gag      409
   Leu Thr Glu Leu Gln Pro Gly Leu Phe His His Leu Arg Phe Leu Glu
           80                  85                  90
   gag ctg cgg ctc tcg ggg aac cac ctc tca cac atc cca aga cag gca      457
   Glu Leu Arg Leu Ser Gly Asn His Leu Ser His Ile Pro Arg Gln Ala
   95                  100                  105
   ttc tct ggc ctc cac agc ctc aaa att ctg atg ctg cag agc aac cag          505
   Phe Ser Gly Leu His Ser Leu Lys Ile Leu Met Leu Gln Ser Asn Gln
   110                 115                 120                 125
   ctc cga ggg atc cca gca gag gcg ctg tgg gag ctg ccc agc ctg cag          553
   Leu Arg Gly Ile Pro Ala Glu Ala Leu Trp Glu Leu Pro Ser Leu Gln
                   130                 135                 140
   tcg ctg cgc cta gat gct aac ctc atc tcc ctg gtc cct gag aga agc          601
   Ser Leu Arg Leu Asp Ala Asn Leu Ile Ser Leu Val Pro Glu Arg Ser
               145                 150                 155
   ttt gag ggg ctg tcc tcc ctc cgc cac ctc tgg ttg gat gac aat gca          649
   Phe Glu Gly Leu Ser Ser Leu Arg His Leu Trp Leu Asp Asp Asn Ala
           160                 165                 170
   ctc acc gag atc cct gtc aga gct ctc aac aac ctt cct gcc cta cag          697
   Leu Thr Glu Ile Pro Val Arg Ala Leu Asn Asn Leu Pro Ala Leu Gln
       175                 180                 185
   gcc atg acc ttg gct ctc aac cgc atc cgc cac atc cct gac tat gcc          745
   Ala Met Thr Leu Ala Leu Asn Arg Ile Arg His Ile Pro Asp Tyr Ala
   190                 195                 200                 205
   ttc cag aac ctc acc agt ctt gtg gtg ctg cat cta cat aac aac cgc          793
   Phe Gln Asn Leu Thr Ser Leu Val Val Leu His Leu His Asn Asn Arg
                   210                 215                 220
   atc cag cat gtg ggg acc cac agc ttc gag ggg ctg cac aat ctg gag          841
   Ile Gln His Val Gly Thr His Ser Phe Glu Gly Leu His Asn Leu Glu
               225                 230                 235
   aca cta gac ttg aac tat aat gag ctg cag gag ttc ccc gtg gct atc          889
   Thr Leu Asp Leu Asn Tyr Asn Glu Leu Gln Glu Phe Pro Val Ala Ile
           240                 245                 250
   cgg acc ctg ggc agg ctg cag gaa ttg ggt ttc cac aac aac aac atc          937
   Arg Thr Leu Gly Arg Leu Gln Glu Leu Gly Phe His Asn Asn Asn Ile
       255                 260                 265
   aag gct atc cca gag aag gcc ttc atg ggc aac cct ctc ctg cag aca          985
   Lys Ala Ile Pro Glu Lys Ala Phe Met Gly Asn Pro Leu Leu Gln Thr
   270                 275                 280                 285
   ata cac ttt tat gac aac ccg atc cag ttt gtg gga agg tca gca ttc          1033
   Ile His Phe Tyr Asp Asn Pro Ile Gln Phe Val Gly Arg Ser Ala Phe
                   290                 295                 300
   cag tac ctg tct aaa ctg cat acg cta tcc ttg aat ggt gcc act gac 1081
   Gln Tyr Leu Ser Lys Leu His Thr Leu Ser Leu Asn Gly Ala Thr Asp
   305 310 315
   atc cag gag ttc cca gac ctc aaa ggc acc act agc ctg gag att ctg 1129
   Ile Gln Glu Phe Pro Asp Leu Lys Gly Thr Thr Ser Leu Glu Ile Leu
   320 325 330
   acc ctg acc cgt gca ggc atc aga ctg ctc cca gcg gga atg tgt caa 1177
   Thr Leu Thr Arg Ala Gly Ile Arg Leu Leu Pro Ala Gly Met Cys Gln
   335 340 345
   cag ctg cct agg ctc cga atc ctg gag ctg tct cat aat cag att gag 1225
   Gln Leu Pro Arg Leu Arg Ile Leu Glu Leu Ser His Asn Gln Ile Glu
   350 355 360 365
   gag cta ccc agc ctg cac agg tgt cag aag ttg gag gaa att ggc ctc 1273
   Glu Leu Pro Ser Leu His Arg Cys Gln Lys Leu Glu Glu Ile Gly Leu
   370 375 380
   cag cat aac cgg atc tgg gaa att ggt gca gat acc ttc agc cag ctg 1321
   Gln His Asn Arg Ile Trp Glu Ile Gly Ala Asp Thr Phe Ser Gln Leu
   385 390 395
   agt tcc ttg caa gct cta gat ctg agt tgg aat gcc atc cgt gcc att 1369
   Ser Ser Leu Gln Ala Leu Asp Leu Ser Trp Asn Ala Ile Arg Ala Ile
   400 405 410
   cac ccc gag gct ttc tca acc ctt cga tcc ttg gtt aag ctg gac ctg 1417
   His Pro Glu Ala Phe Ser Thr Leu Arg Ser Leu Val Lys Leu Asp Leu
   415 420 425
   act gac aac cag ctg acc aca ctg ccc ctg gct ggg ctg gga ggc ctg 1465
   Thr Asp Asn Gln Leu Thr Thr Leu Pro Leu Ala Gly Leu Gly Gly Leu
   430 435 440 445
   atg cac ctg aag ctc aag ggg aac ttg gcc ctg tct cag gcc ttc tcc 1513
   Met His Leu Lys Leu Lys Gly Asn Leu Ala Leu Ser Gln Ala Phe Ser
   450 455 460
   aag gac agt ttc ccc aaa ctg agg atc ctg gag gtg cct tac gcc tac 1561
   Lys Asp Ser Phe Pro Lys Leu Arg Ile Leu Glu Val Pro Tyr Ala Tyr
   465 470 475
   cag tgc tgt gcc tat ggc ctc tgt gcc aac ttc ttc aag acc tct ggg 1609
   Gln Cys Cys Ala Tyr Gly Leu Cys Ala Asn Phe Phe Lys Thr Ser Gly
   480 485 490
   cag tgg cag gcc gag gac ttt cat gca gag gaa gaa gag gca cca aag 1657
   Gln Trp Gln Ala Glu Asp Phe His Ala Glu Glu Glu Glu Ala Pro Lys
   495 500 505
   agg ccc ctg ggt ctc ctt gct gga caa gct gag aac cac tat gac cta 1705
   Arg Pro Leu Gly Leu Leu Ala Gly Gln Ala Glu Asn His Tyr Asp Leu
   510 515 520 525
   gac ctg gat gag ctc cag atg gag aca gag gac tca aag cca cac ccc 1753
   Asp Leu Asp Glu Leu Gln Met Glu Thr Glu Asp Ser Lys Pro His Pro
   530 535 540
   agt gtc cag tgc agc cct gtt cca ggc ccc ttc aag ccc tgc gag cac 1801
   Ser Val Gln Cys Ser Pro Val Pro Gly Pro Phe Lys Pro Cys Glu His
   545 550 555
   ctc ttt gag agc tgg ggc atc cgc ctt gct gtg tgg gcc atc gtg ctg 1849
   Leu Phe Glu Ser Trp Gly Ile Arg Leu Ala Val Trp Ala Ile Val Leu
   560 565 570
   ctc tcg gta ctc tgt aac ggg ctg gtg ctg ctt aca gtc ttt gct ggc 1897
   Leu Ser Val Leu Cys Asn Gly Leu Val Leu Leu Thr Val Phe Ala Gly
   575 580 585
   ggg ccc agc ccg ttg tct ccg gtc aag ctt gtg gtg ggt gcg atc gct 1945
   Gly Pro Ser Pro Leu Ser Pro Val Lys Leu Val Val Gly Ala Ile Ala
   590 595 600 605
   ggc gcc aac gcc cta acg ggc att tcc tgc ggc ctc ctg gcc tca gtg 1993
   Gly Ala Asn Ala Leu Thr Gly Ile Ser Cys Gly Leu Leu Ala Ser Val
   610 615 620
   gac gcc ttg acc ttc ggc cag ttc gcg gag tat gga gcc cgc tgg gag 2041
   Asp Ala Leu Thr Phe Gly Gln Phe Ala Glu Tyr Gly Ala Arg Trp Glu
   625 630 635
   acc ggg cta ggc tgc cag gct acg ggc ttc cta gcc gtc ctg ggg tcg 2089
   Thr Gly Leu Gly Cys Gln Ala Thr Gly Phe Leu Ala Val Leu Gly Ser
   640 645 650
   gag gcg tcg gtg ctg ctg ctc acg ctg gcg gcc gtg cag tgc agc gtc 2137
   Glu Ala Ser Val Leu Leu Leu Thr Leu Ala Ala Val Gln Cys Ser Val
   655 660 665
   tct gtg acc tgc gtc cgg gcc tac ggg aag gcg ccg tcg ccc ggt agc 2185
   Ser Val Thr Cys Val Arg Ala Tyr Gly Lys Ala Pro Ser Pro Gly Ser
   670 675 680 685
   gtc cgc gca ggc gca ctg gga tgc ttg gcg ctg gct ggg ctg gcc gcc 2233
   Val Arg Ala Gly Ala Leu Gly Cys Leu Ala Leu Ala Gly Leu Ala Ala
                   690                 695                 700
   gcg ctg ccg ctg gcc tcg gtg ggg gag tat ggt gcc tcc ccg ctc tgc      2281
   Ala Leu Pro Leu Ala Ser Val Gly Glu Tyr Gly Ala Ser Pro Leu Cys
               705                 710                 715
   ctg ccc tac gcc cct ccc gag ggc cgc ccg gcc gcc cta ggc ttc gcc      2329
   Leu Pro Tyr Ala Pro Pro Glu Gly Arg Pro Ala Ala Leu Gly Phe Ala
           720                 725                 730
   gta gcc ctg gtg atg atg aac tcg ctc tgc ttc ctc gtg gtg gcc ggt      2377
   Val Ala Leu Val Met Met Asn Ser Leu Cys Phe Leu Val Val Ala Gly
       735                 740                 745
   gcc tac atc aag ctc tac tgc gac ctg cct cgg ggt gac ttt gag gcc      2425
   Ala Tyr Ile Lys Leu Tyr Cys Asp Leu Pro Arg Gly Asp Phe Glu Ala
   750                 755                 760                 765
   gtg tgg gac tgc gcc atg gtg cgg cat gtg gcc tgg ctc atc ttt gca      2473
   Val Trp Asp Cys Ala Met Val Arg His Val Ala Trp Leu Ile Phe Ala
                   770                 775                 780
   gat ggc ctt ctc tac tgc ccc gtg gcc ttc ctc agc ttt gcc tcc atg      2521
   Asp Gly Leu Leu Tyr Cys Pro Val Ala Phe Leu Ser Phe Ala Ser Met
               785                 790                 795
   ctg ggc ctc ttc ccg gtc acc cct gag gct gtc aag tcg gtt ctt ctg      2569
   Leu Gly Leu Phe Pro Val Thr Pro Glu Ala Val Lys Ser Val Leu Leu
           800                 805                 810
   gtg gtg ttg cct ctg ccc gcc tgc ctg aac cca ctg ctc tac ctg ctc      2617
   Val Val Leu Pro Leu Pro Ala Cys Leu Asn Pro Leu Leu Tyr Leu Leu
       815                 820                 825
   ttc aac cct cac ttc cgg gaa gac ctt cgg agg ctc tgg ccg agc cct      2665
   Phe Asn Pro His Phe Arg Glu Asp Leu Arg Arg Leu Trp Pro Ser Pro
   830                 835                 840                 845
   cgg tcc cca ggg cct cta gct tat act gca gcc ggt gag ctg gag aag      2713
   Arg Ser Pro Gly Pro Leu Ala Tyr Thr Ala Ala Gly Glu Leu Glu Lys
                   850                 855                 860
   agc tcc tgc gac tcc acc caa gcc ctg gtg gct ttc tct gat gtg gat      2761
   Ser Ser Cys Asp Ser Thr Gln Ala Leu Val Ala Phe Ser Asp Val Asp
               865                 870                 875
   ctt att ctg gaa gct tct gag gct ggg cag cct cct ggg cta gag acc      2809
   Leu Ile Leu Glu Ala Ser Glu Ala Gly Gln Pro Pro Gly Leu Glu Thr
   880 885 890
   tat ggc ttc cct tca gtg acc ctc atc tcc cgt cat cag cca ggg gct 2857
   Tyr Gly Phe Pro Ser Val Thr Leu Ile Ser Arg His Gln Pro Gly Ala
   895 900 905
   acc agg ctg gag gga aac cat ttt gtg gag cct gat gga acc aag tta 2905
   Thr Arg Leu Glu Gly Asn His Phe Val Glu Pro Asp Gly Thr Lys Leu
   910 915 920 925
   ggg aac cca caa cct ccc atg aat gaa gaa ctg ctg ctg agg gca gag 2953
   Gly Asn Pro Gln Pro Pro Met Asn Glu Glu Leu Leu Leu Arg Ala Glu
   930 935 940
   gga gcc aca ctg gca gac tgt tct gca ggt gga gcc ctc tgg ccc tct 3001
   Gly Ala Thr Leu Ala Asp Cys Ser Ala Gly Gly Ala Leu Trp Pro Ser
   945 950 955
   ggc cct ctc ttt gcc tct cac ttg taa ataacccccc cccccaattt        3048
   Gly Pro Leu Phe Ala Ser His Leu
           960                 965
   gtcctccctc ctcccaatga tggctgctta taaaagaaag acaactacag ctccgcagca 3108
   gaatggccaa cgcccctggc tccattgttc accgtggcca taacaaggtg tgcttccagg 3168
   tcttgctttg tcttggcctt cagcttcacg gtcaccctgg gccttccctg tccaatccga 3228
   gtatgtatga cagagacctg ggaaatttac ttaggagaaa ggaggaaagc aaaagacagt 3288
   gaaggctgtg ggaggctgac agagttgcga ccagtgagtt acacagtgag cggggaggcc 3348
   tcacagagaa aggcactgga aggcaactac caaagacatt ggagtgtccc gcctacggcg 3408
   tgtggatagc atagaaaacg tgttctgcgt cccaccaatc ttgacctatg ctgtgccaaa 3468
   gtgcttccag ttaagatacg ctttggcaga cattgcatat tatgcccctt tcttagagtc 3528
   atggggtcat gttagtgtct aaaaggtcct aagaattagg accttttggc ttaatgatga 3588
   cagcagttaa acgtacttat ttgatgatag gtttctgttt gcttttcatg gaacgcttac 3648
   aaatctctcg cagaacttag cacgg 3673

<210> 4
<211> 965
<212> PRT
<213> Rattus norvegicus

<400> 4

Met His Ser Ser Pro Leu Leu Leu Ala Leu Cys Leu Cys Val Val Leu
1               5                   10                  15

Cys Ala Leu Ala Arg Ala Gly Ser Asp Pro Gln Pro Gly Pro Gly Arg
            20                  25                  30

Pro Ala Cys Pro Ala Pro Cys His Cys Gln Glu Asp Gly Ile Met Leu
        35                  40                  45

Ser Ala Asp Cys Ser Glu Leu Gly Leu Ser Glu Val Pro Ala Asp Leu
    50                  55                  60

Asp Pro Leu Thr Ala Tyr Leu Asp Leu Ser Met Asn Asn Leu Thr Glu
65                  70                  75                  80

Leu Gln Pro Gly Leu Phe His His Leu Arg Phe Leu Glu Glu Leu Arg
                85                  90                  95

Leu Ser Gly Asn His Leu Ser His Ile Pro Arg Gln Ala Phe Ser Gly
            100                 105                 110

Leu His Ser Leu Lys Ile Leu Met Leu Gln Ser Asn Gln Leu Arg Gly
        115                 120                 125

Ile Pro Ala Glu Ala Leu Trp Glu Leu Pro Ser Leu Gln Ser Leu Arg
    130                 135                 140

Leu Asp Ala Asn Leu Ile Ser Leu Val Pro Glu Arg Ser Phe Glu Gly
145                 150                 155                 160

Leu Ser Ser Leu Arg His Leu Trp Leu Asp Asp Asn Ala Leu Thr Glu
                165                 170                 175

Ile Pro Val Arg Ala Leu Asn Asn Leu Pro Ala Leu Gln Ala Met Thr
            180                 185                 190

Leu Ala Leu Asn Arg Ile Arg His Ile Pro Asp Tyr Ala Phe Gln Asn
        195                 200                 205

Leu Thr Ser Leu Val Val Leu His Leu His Asn Asn Arg Ile Gln His
    210                 215                 220

Val Gly Thr His Ser Phe Glu Gly Leu His Asn Leu Glu Thr Leu Asp
225                 230                 235                 240

Leu Asn Tyr Asn Glu Leu Gln Glu Phe Pro Val Ala Ile Arg Thr Leu
                245                 250                 255

Gly Arg Leu Gln Glu Leu Gly Phe His Asn Asn Asn Ile Lys Ala Ile
            260                 265                 270

Pro Glu Lys Ala Phe Met Gly Asn Pro Leu Leu Gln Thr Ile His Phe
        275                 280                 285

Tyr Asp Asn Pro Ile Gln Phe Val Gly Arg Ser Ala Phe Gln Tyr Leu
    290                 295                 300

Ser Lys Leu His Thr Leu Ser Leu Asn Gly Ala Thr Asp Ile Gln Glu
305                 310                 315                 320

Phe Pro Asp Leu Lys Gly Thr Thr Ser Leu Glu Ile Leu Thr Leu Thr
                325                 330                 335

Arg Ala Gly Ile Arg Leu Leu Pro Ala Gly Met Cys Gln Gln Leu Pro
            340                 345                 350

Arg Leu Arg Ile Leu Glu Leu Ser His Asn Gln Ile Glu Glu Leu Pro
        355                 360                 365

Ser Leu His Arg Cys Gln Lys Leu Glu Glu Ile Gly Leu Gln His Asn
    370                 375                 380

Arg Ile Trp Glu Ile Gly Ala Asp Thr Phe Ser Gln Leu Ser Ser Leu
385                 390                 395                 400

Gln Ala Leu Asp Leu Ser Trp Asn Ala Ile Arg Ala Ile His Pro Glu
                405                 410                 415

Ala Phe Ser Thr Leu Arg Ser Leu Val Lys Leu Asp Leu Thr Asp Asn
            420                 425                 430

Gln Leu Thr Thr Leu Pro Leu Ala Gly Leu Gly Gly Leu Met His Leu
        435                 440                 445

Lys Leu Lys Gly Asn Leu Ala Leu Ser Gln Ala Phe Ser Lys Asp Ser
    450                 455                 460

Phe Pro Lys Leu Arg Ile Leu Glu Val Pro Tyr Ala Tyr Gln Cys Cys
465                 470                 475                 480

Ala Tyr Gly Leu Cys Ala Asn Phe Phe Lys Thr Ser Gly Gln Trp Gln
                485                 490                 495

Ala Glu Asp Phe His Ala Glu Glu Glu Glu Ala Pro Lys Arg Pro Leu
            500                 505                 510

Gly Leu Leu Ala Gly Gln Ala Glu Asn His Tyr Asp Leu Asp Leu Asp
        515                 520                 525

Glu Leu Gln Met Glu Thr Glu Asp Ser Lys Pro His Pro Ser Val Gln
    530                 535                 540

Cys Ser Pro Val Pro Gly Pro Phe Lys Pro Cys Glu His Leu Phe Glu
545                 550                 555                 560

Ser Trp Gly Ile Arg Leu Ala Val Trp Ala Ile Val Leu Leu Ser Val
                565                 570                 575

Leu Cys Asn Gly Leu Val Leu Leu Thr Val Phe Ala Gly Gly Pro Ser
            580                 585                 590

Pro Leu Ser Pro Val Lys Leu Val Val Gly Ala Ile Ala Gly Ala Asn
        595                 600                 605

Ala Leu Thr Gly Ile Ser Cys Gly Leu Leu Ala Ser Val Asp Ala Leu
    610                 615                 620

Thr Phe Gly Gln Phe Ala Glu Tyr Gly Ala Arg Trp Glu Thr Gly Leu
625                 630                 635                 640

Gly Cys Gln Ala Thr Gly Phe Leu Ala Val Leu Gly Ser Glu Ala Ser
                645                 650                 655

Val Leu Leu Leu Thr Leu Ala Ala Val Gln Cys Ser Val Ser Val Thr
            660                 665                 670

Cys Val Arg Ala Tyr Gly Lys Ala Pro Ser Pro Gly Ser Val Arg Ala
        675                 680                 685

Gly Ala Leu Gly Cys Leu Ala Leu Ala Gly Leu Ala Ala Ala Leu Pro
    690                 695                 700

Leu Ala Ser Val Gly Glu Tyr Gly Ala Ser Pro Leu Cys Leu Pro Tyr
705                 710                 715                 720

Ala Pro Pro Glu Gly Arg Pro Ala Ala Leu Gly Phe Ala Val Ala Leu
                725                 730                 735

Val Met Met Asn Ser Leu Cys Phe Leu Val Val Ala Gly Ala Tyr Ile
            740                 745                 750

Lys Leu Tyr Cys Asp Leu Pro Arg Gly Asp Phe Glu Ala Val Trp Asp
        755                 760                 765

Cys Ala Met Val Arg His Val Ala Trp Leu Ile Phe Ala Asp Gly Leu

770                 775                 780

Leu Tyr Cys Pro Val Ala Phe Leu Ser Phe Ala Ser Met Leu Gly Leu
785                 790                 795                 800

Phe Pro Val Thr Pro Glu Ala Val Lys Ser Val Leu Leu Val Val Leu
                805                 810                 815

Pro Leu Pro Ala Cys Leu Asn Pro Leu Leu Tyr Leu Leu Phe Asn Pro
            820                 825                 830

His Phe Arg Glu Asp Leu Arg Arg Leu Trp Pro Ser Pro Arg Ser Pro
        835                 840                 845

Gly Pro Leu Ala Tyr Thr Ala Ala Gly Glu Leu Glu Lys Ser Ser Cys
    850                 855                 860

Asp Ser Thr Gln Ala Leu Val Ala Phe Ser Asp Val Asp Leu Ile Leu
865                 870                 875                 880

Glu Ala Ser Glu Ala Gly Gln Pro Pro Gly Leu Glu Thr Tyr Gly Phe
                885                 890                 895

Pro Ser Val Thr Leu Ile Ser Arg His Gln Pro Gly Ala Thr Arg Leu
            900                 905                 910

Glu Gly Asn His Phe Val Glu Pro Asp Gly Thr Lys Leu Gly Asn Pro
        915                 920                 925

Gln Pro Pro Met Asn Glu Glu Leu Leu Leu Arg Ala Glu Gly Ala Thr
    930                 935                 940

Leu Ala Asp Cys Ser Ala Gly Gly Ala Leu Trp Pro Ser Gly Pro Leu
945                 950                 955                 960

Phe Ala Ser His Leu
                965

<210> 5
<211> 3458
<212> DNA
<213> Homo sapiens

<220>
<221> CDS
<222> (1)..(2904)

<400> 5
atg ccc agc ccg ccg ggg ctc cgg gcg cta tgg ctt tgc gcc gcg ctg 48
Met Pro Ser Pro Pro Gly Leu Arg Ala Leu Trp Leu Cys Ala Ala Leu
1               5                   10                  15

tgc gct tcc cgg agg gcc ggc ggc gcc ccc cag ccc ggc ccg ggg ccc 96
Cys Ala Ser Arg Arg Ala Gly Gly Ala Pro Gln Pro Gly Pro Gly Pro
            20                  25                  30

acc gcc tgc ccg gcc ccc tgc cac tgc cag gag gac ggc atc atg ctg 144
Thr Ala Cys Pro Ala Pro Cys His Cys Gln Glu Asp Gly Ile Met Leu
        35                  40                  45

tct gcc gac tgc tct gag ctc ggg ctg tcc gcc gtt ccg ggg gac ctg 192
Ser Ala Asp Cys Ser Glu Leu Gly Leu Ser Ala Val Pro Gly Asp Leu
    50                  55                  60

gac ccc ctg acg gct tac ctg gac ctc agc atg aac aac ctc aca gag 240
Asp Pro Leu Thr Ala Tyr Leu Asp Leu Ser Met Asn Asn Leu Thr Glu
65                  70                  75                  80

ctt cag cct ggc ctc ttc cac cac ctg cgc ttc ttg gag gag ctg cgt 288
Leu Gln Pro Gly Leu Phe His His Leu Arg Phe Leu Glu Glu Leu Arg
                85                  90                  95

ctc tct ggg aac cat ctc tca cac atc cca gga caa gca ttc tct ggt 336
Leu Ser Gly Asn His Leu Ser His Ile Pro Gly Gln Ala Phe Ser Gly
            100                 105                 110

ctc tac agc ctg aaa atc ctg atg ctg cag aac aat cag ctg gga gga 384
Leu Tyr Ser Leu Lys Ile Leu Met Leu Gln Asn Asn Gln Leu Gly Gly
        115                 120                 125

atc ccc gca gag gcg ctg tgg gag ctg ccg agc ctg cag tcg ctg cgc 432
Ile Pro Ala Glu Ala Leu Trp Glu Leu Pro Ser Leu Gln Ser Leu Arg
    130                 135                 140

cta gat gcc aac ctc atc tcc ctg gtc ccg gag agg agc ttt gag ggg 480
Leu Asp Ala Asn Leu Ile Ser Leu Val Pro Glu Arg Ser Phe Glu Gly
145                 150                 155                 160

ctg tcc tcc ctc cgc cac ctc tgg ctg gac gac aat gca ctc acg gag 528
Leu Ser Ser Leu Arg His Leu Trp Leu Asp Asp Asn Ala Leu Thr Glu
                165                 170                 175

atc cct gtc agg gcc ctc aac aac ctc cct gcc ctg cag gcc atg acc 576
Ile Pro Val Arg Ala Leu Asn Asn Leu Pro Ala Leu Gln Ala Met Thr
            180                 185                 190

ctg gcc ctc aac cgc atc agc cac atc ccc gac tac gcg ttc cag aat 624
Leu Ala Leu Asn Arg Ile Ser His Ile Pro Asp Tyr Ala Phe Gln Asn
        195                 200                 205

ctc acc agc ctt gtg gtg ctg cat ttg cat aac aac cgc atc cag cat 672
Leu Thr Ser Leu Val Val Leu His Leu His Asn Asn Arg Ile Gln His
    210                 215                 220

ctg ggg acc cac agc ttc gag ggg ctg cac aat ctg gag aca cta gac 720
Leu Gly Thr His Ser Phe Glu Gly Leu His Asn Leu Glu Thr Leu Asp
225                 230                 235                 240

ctg aat tat aac aag ctg cag gag ttc cct gtg gcc atc cgg acc ctg 768
Leu Asn Tyr Asn Lys Leu Gln Glu Phe Pro Val Ala Ile Arg Thr Leu
                245                 250                 255

ggc aga ctg cag gaa ctg ggg ttc cat aac aac aac atc aag gcc atc 816
Gly Arg Leu Gln Glu Leu Gly Phe His Asn Asn Asn Ile Lys Ala Ile
            260                 265                 270

cca gaa aag gcc ttc atg ggg aac cct ctg cta cag acg ata cac ttt 864
Pro Glu Lys Ala Phe Met Gly Asn Pro Leu Leu Gln Thr Ile His Phe
        275                 280                 285

tat gat aac cca atc cag ttt gtg gga aga tcg gca ttc cag tac ctg 912
Tyr Asp Asn Pro Ile Gln Phe Val Gly Arg Ser Ala Phe Gln Tyr Leu
    290                 295                 300

cct aaa ctc cac aca cta tct ctg aat ggt gcc atg gac atc cag gag 960
Pro Lys Leu His Thr Leu Ser Leu Asn Gly Ala Met Asp Ile Gln Glu
305                 310                 315                 320

ttt cca gat ctc aaa ggc acc acc agc ctg gag atc ctg acc ctg acc 1008
Phe Pro Asp Leu Lys Gly Thr Thr Ser Leu Glu Ile Leu Thr Leu Thr
                325                 330                 335

cgc gca ggc atc cgg ctg ctc cca tcg ggg atg tgc caa cag ctg ccc 1056

Arg Ala Gly Ile Arg Leu Leu Pro Ser Gly Met Cys Gln Gln Leu Pro
            340                 345                 350

agg ctc cga gtc ctg gaa ctg tct cac aat caa att gag gag ctg ccc 1104
Arg Leu Arg Val Leu Glu Leu Ser His Asn Gln Ile Glu Glu Leu Pro
        355                 360                 365

agc ctg cac agg tgt cag aaa ttg gag gaa atc ggc ctc caa cac aac 1152
Ser Leu His Arg Cys Gln Lys Leu Glu Glu Ile Gly Leu Gln His Asn
    370                 375                 380

cgc atc tgg gaa att gga gct gac acc ttc agc cag ctg agc tcc ctg 1200
Arg Ile Trp Glu Ile Gly Ala Asp Thr Phe Ser Gln Leu Ser Ser Leu
385                 390                 395                 400

caa gcc ctg gat ctt agc tgg aac gcc atc cgg tcc atc cac ccc gag 1248
Gln Ala Leu Asp Leu Ser Trp Asn Ala Ile Arg Ser Ile His Pro Glu
                405                 410                 415

gcc ttc tcc acc ctg cac tcc ctg gtc aag ctg gac ctg aca gac aac 1296
Ala Phe Ser Thr Leu His Ser Leu Val Lys Leu Asp Leu Thr Asp Asn
            420                 425                 430

cag ctg acc aca ctg ccc ctg gct gga ctt ggg ggc ttg atg cat ctg 1344
Gln Leu Thr Thr Leu Pro Leu Ala Gly Leu Gly Gly Leu Met His Leu
        435                 440                 445

aag ctc aaa ggg aac ctt gct ctc tcc cag gcc ttc tcc aag gac agt 1392
Lys Leu Lys Gly Asn Leu Ala Leu Ser Gln Ala Phe Ser Lys Asp Ser
    450                 455                 460

ttc cca aaa ctg agg atc ctg gag gtg cct tat gcc tac cag tgc tgt 1440
Phe Pro Lys Leu Arg Ile Leu Glu Val Pro Tyr Ala Tyr Gln Cys Cys
465                 470                 475                 480

ccc tat ggg atg tgt gcc agc ttc ttc aag gcc tct ggg cag tgg gag 1488
Pro Tyr Gly Met Cys Ala Ser Phe Phe Lys Ala Ser Gly Gln Trp Glu
                485                 490                 495

gct gaa gac ctt cac ctt gat gat gag gag tct tca aaa agg ccc ctg 1536
Ala Glu Asp Leu His Leu Asp Asp Glu Glu Ser Ser Lys Arg Pro Leu
            500                 505                 510

ggc ctc ctt gcc aga caa gca gag aac cac tat gac cag gac ctg gat 1584
Gly Leu Leu Ala Arg Gln Ala Glu Asn His Tyr Asp Gln Asp Leu Asp
        515                 520                 525

gag ctc cag ctg gag atg gag gac tca aag cca cac ccc agt gtc cag 1632
Glu Leu Gln Leu Glu Met Glu Asp Ser Lys Pro His Pro Ser Val Gln

    530                 535                 540

tgt agc cct act cca ggc ccc ttc aag ccc tgt gag tac ctc ttt gaa 1680
Cys Ser Pro Thr Pro Gly Pro Phe Lys Pro Cys Glu Tyr Leu Phe Glu
545                 550                 555                 560

agc tgg ggc atc cgc ctg gcc gtg tgg gcc atc gtg ttg ctc tcc gtg 1728
Ser Trp Gly Ile Arg Leu Ala Val Trp Ala Ile Val Leu Leu Ser Val
                565                 570                 575

ctc tgc aat gga ctg gtg ctg ctg acc gtg ttc gct ggc ggg cct gtc 1776
Leu Cys Asn Gly Leu Val Leu Leu Thr Val Phe Ala Gly Gly Pro Val
            580                 585                 590

ccc ctg ccc ccg gtc aag ttt gtg gta ggt gcg att gca ggc gcc aac 1824
Pro Leu Pro Pro Val Lys Phe Val Val Gly Ala Ile Ala Gly Ala Asn
        595                 600                 605

acc ttg act ggc att tcc tgt ggc ctt cta gcc tca gtc gat gcc ctg 1872
Thr Leu Thr Gly Ile Ser Cys Gly Leu Leu Ala Ser Val Asp Ala Leu
    610                 615                 620

acc ttt ggt cag ttc tct gag tac gga gcc cgc tgg gag acg ggg cta 1920
Thr Phe Gly Gln Phe Ser Glu Tyr Gly Ala Arg Trp Glu Thr Gly Leu
625                 630                 635                 640

ggc tgc cgg gcc act ggc ttc ctg gca gta ctt ggg tcg gag gca tcg 1968
Gly Cys Arg Ala Thr Gly Phe Leu Ala Val Leu Gly Ser Glu Ala Ser
                645                 650                 655

gtg ctg ctg ctc act ctg gcc gca gtg cag tgc agc gtc tcc gtc tcc 2016
Val Leu Leu Leu Thr Leu Ala Ala Val Gln Cys Ser Val Ser Val Ser
            660                 665                 670

tgt gtc cgg gcc tat ggg aag tcc ccc tcc ctg ggc agc gtt cga gca 2064
Cys Val Arg Ala Tyr Gly Lys Ser Pro Ser Leu Gly Ser Val Arg Ala
        675                 680                 685

ggg gtc cta ggc tgc ctg gca ctg gca ggg ctg gcc gcc gcg ctg ccc 2112
Gly Val Leu Gly Cys Leu Ala Leu Ala Gly Leu Ala Ala Ala Leu Pro
    690                 695                 700

ctg gcc tca gtg gga gaa tac ggg gcc tcc cca ctc tgc ctg ccc tac 2160
Leu Ala Ser Val Gly Glu Tyr Gly Ala Ser Pro Leu Cys Leu Pro Tyr
705                 710                 715                 720

gcg cca cct gag ggt cag cca gca gcc ctg ggc ttc acc gtg gcc ctg 2208
Ala Pro Pro Glu Gly Gln Pro Ala Ala Leu Gly Phe Thr Val Ala Leu
                725                 730                 735

gtg atg atg aac tcc ttc tgt ttc ctg gtc gtg gcc ggt gcc tac atc 2256
Val Met Met Asn Ser Phe Cys Phe Leu Val Val Ala Gly Ala Tyr Ile
            740                 745                 750

aaa ctg tac tgt gac ctg ccg cgg ggc gac ttt gag gcc gtg tgg gac 2304
Lys Leu Tyr Cys Asp Leu Pro Arg Gly Asp Phe Glu Ala Val Trp Asp
        755                 760                 765

tgc gcc atg gtg agg cac gtg gcc tgg ctc atc ttc gca gac ggg ctc 2352
Cys Ala Met Val Arg His Val Ala Trp Leu Ile Phe Ala Asp Gly Leu
    770                 775                 780

ctc tac tgt ccc gtg gcc ttc ctc agc ttt gcc tcc atg ctg ggc ctc 2400
Leu Tyr Cys Pro Val Ala Phe Leu Ser Phe Ala Ser Met Leu Gly Leu
785                 790                 795                 800

ttc cct gtc acg ccc gag gcc gtc aag tct gtc ctg ctg gtg gtg ctg 2448
Phe Pro Val Thr Pro Glu Ala Val Lys Ser Val Leu Leu Val Val Leu
                805                 810                 815

ccc ctg cct gcc tgc ctc aac cca ctg ctg tac ctg ctc ttc aac ccc 2496
Pro Leu Pro Ala Cys Leu Asn Pro Leu Leu Tyr Leu Leu Phe Asn Pro
            820                 825                 830

cac ttc cgg gat gac ctt cgg cgg ctt cgg ccc cgc gca ggg gac tca 2544
His Phe Arg Asp Asp Leu Arg Arg Leu Arg Pro Arg Ala Gly Asp Ser
        835                 840                 845

ggg ccc cta gcc tat gct gcg gcc ggg gag ctg gag aag agc tcc tgt 2592
Gly Pro Leu Ala Tyr Ala Ala Ala Gly Glu Leu Glu Lys Ser Ser Cys
    850                 855                 860

gat tct acc cag gcc ctg gta gcc ttc tct gat gtg gat ctc att ctg 2640
Asp Ser Thr Gln Ala Leu Val Ala Phe Ser Asp Val Asp Leu Ile Leu
865                 870                 875                 880

gaa gct tct gaa gct ggg cgg ccc cct ggg ctg gag acc tat ggc ttc 2688
Glu Ala Ser Glu Ala Gly Arg Pro Pro Gly Leu Glu Thr Tyr Gly Phe
                885                 890                 895

ccc tca gtg acc ctc atc tcc tgt cag cag cca ggg gcc ccc agg ctg 2736
Pro Ser Val Thr Leu Ile Ser Cys Gln Gln Pro Gly Ala Pro Arg Leu
            900                 905                 910

gag ggc agc cat tgt gta gag cca gag ggg aac cac ttt ggg aac ccc 2784
Glu Gly Ser His Cys Val Glu Pro Glu Gly Asn His Phe Gly Asn Pro
        915                 920                 925

caa ccc tcc atg gat gga gaa ctg ctg ctg agg gca gag gga tct acg     2832
Gln Pro Ser Met Asp Gly Glu Leu Leu Leu Arg Ala Glu Gly Ser Thr
    930                 935                 940

cca gca ggt gga ggc ttg tca ggg ggt ggc ggc ttt cag ccc tct ggc     2880
Pro Ala Gly Gly Gly Leu Ser Gly Gly Gly Gly Phe Gln Pro Ser Gly
945                 950                 955                 960

ttg gcc ttt gct tca cac gtg taa atatccctcc ccattcttct cttcccctct     2934
Leu Ala Phe Ala Ser His Val
                965

cttccctttc ctctctcccc ctcggtgaat gatggctgct tctaaaacaa atacaaccaa     2994

aactcagcag tgtgatctat agcaggatgg cccagtccct ggctccactg atcacctctc     3054

tcctgtgacc atcaccaacg ggtgcctctt ggcctggctt tcccttggcc ttcctcagct     3114

tcaccttgat actgggcctc ttccttgtca tgtctgaagc tgtggaccag agacctggac     3174

ttttgtctgc ttaagggaaa tgagggaagt aaagacagtg aaggggtgga gggttgatca     3234

gggcacagtg gacagggaga cctcacagag aaaggcctgg aaggtgattt cccgtgtgac     3294

tcatggatag gatacaaaat gtgttccatg taccattaat cttgacatat gccatgcata     3354

aagacttcct attaaaataa gctttggaag agattacaca tgatgtcttt ttcttagaga     3414

ttcacagtgc atgttagtgt aataaagaga taagtcctac agta                      3458

<210>  6
<211>  967
<212>  PRT
<213>  Homo sapiens

<400>  6

Met Pro Ser Pro Pro Gly Leu Arg Ala Leu Trp Leu Cys Ala Ala Leu
1               5                   10                  15

Cys Ala Ser Arg Arg Ala Gly Gly Ala Pro Gln Pro Gly Pro Gly Pro
            20                  25                  30

Thr Ala Cys Pro Ala Pro Cys His Cys Gln Glu Asp Gly Ile Met Leu
        35                  40                  45

Ser Ala Asp Cys Ser Glu Leu Gly Leu Ser Ala Val Pro Gly Asp Leu
    50                  55                  60

Asp Pro Leu Thr Ala Tyr Leu Asp Leu Ser Met Asn Asn Leu Thr Glu
65                  70                  75                  80

Leu Gln Pro Gly Leu Phe His His Leu Arg Phe Leu Glu Glu Leu Arg
                85                  90                  95

Leu Ser Gly Asn His Leu Ser His Ile Pro Gly Gln Ala Phe Ser Gly
            100                 105                 110

Leu Tyr Ser Leu Lys Ile Leu Met Leu Gln Asn Asn Gln Leu Gly Gly
        115                 120                 125

Ile Pro Ala Glu Ala Leu Trp Glu Leu Pro Ser Leu Gln Ser Leu Arg
    130                 135                 140

Leu Asp Ala Asn Leu Ile Ser Leu Val Pro Glu Arg Ser Phe Glu Gly
145                 150                 155                 160

Leu Ser Ser Leu Arg His Leu Trp Leu Asp Asp Asn Ala Leu Thr Glu
                165                 170                 175

Ile Pro Val Arg Ala Leu Asn Asn Leu Pro Ala Leu Gln Ala Met Thr
            180                 185                 190

Leu Ala Leu Asn Arg Ile Ser His Ile Pro Asp Tyr Ala Phe Gln Asn
        195                 200                 205

Leu Thr Ser Leu Val Val Leu His Leu His Asn Asn Arg Ile Gln His
    210                 215                 220

Leu Gly Thr His Ser Phe Glu Gly Leu His Asn Leu Glu Thr Leu Asp
225                 230                 235                 240

Leu Asn Tyr Asn Lys Leu Gln Glu Phe Pro Val Ala Ile Arg Thr Leu
                245                 250                 255

Gly Arg Leu Gln Glu Leu Gly Phe His Asn Asn Asn Ile Lys Ala Ile
            260                 265                 270

Pro Glu Lys Ala Phe Met Gly Asn Pro Leu Leu Gln Thr Ile His Phe
        275                 280                 285

Tyr Asp Asn Pro Ile Gln Phe Val Gly Arg Ser Ala Phe Gln Tyr Leu
    290                 295                 300

Pro Lys Leu His Thr Leu Ser Leu Asn Gly Ala Met Asp Ile Gln Glu
305                 310                 315                 320

Phe Pro Asp Leu Lys Gly Thr Thr Ser Leu Glu Ile Leu Thr Leu Thr
                325                 330                 335

Arg Ala Gly Ile Arg Leu Leu Pro Ser Gly Met Cys Gln Gln Leu Pro
            340                 345                 350

Arg Leu Arg Val Leu Glu Leu Ser His Asn Gln Ile Glu Glu Leu Pro
        355                 360                 365

Ser Leu His Arg Cys Gln Lys Leu Glu Glu Ile Gly Leu Gln His Asn
    370                 375                 380

Arg Ile Trp Glu Ile Gly Ala Asp Thr Phe Ser Gln Leu Ser Ser Leu
385                 390                 395                 400

Gln Ala Leu Asp Leu Ser Trp Asn Ala Ile Arg Ser Ile His Pro Glu
                405                 410                 415

Ala Phe Ser Thr Leu His Ser Leu Val Lys Leu Asp Leu Thr Asp Asn
            420                 425                 430

Gln Leu Thr Thr Leu Pro Leu Ala Gly Leu Gly Gly Leu Met His Leu

435                 440                 445

Lys Leu Lys Gly Asn Leu Ala Leu Ser Gln Ala Phe Ser Lys Asp Ser
    450                 455                 460

Phe Pro Lys Leu Arg Ile Leu Glu Val Pro Tyr Ala Tyr Gln Cys Cys
465                 470                 475                 480

Pro Tyr Gly Met Cys Ala Ser Phe Phe Lys Ala Ser Gly Gln Trp Glu
                485                 490                 495

Ala Glu Asp Leu His Leu Asp Asp Glu Glu Ser Ser Lys Arg Pro Leu
            500                 505                 510

Gly Leu Leu Ala Arg Gln Ala Glu Asn His Tyr Asp Gln Asp Leu Asp
        515                 520                 525

Glu Leu Gln Leu Glu Met Glu Asp Ser Lys Pro His Pro Ser Val Gln
    530                 535                 540

Cys Ser Pro Thr Pro Gly Pro Phe Lys Pro Cys Glu Tyr Leu Phe Glu
545                 550                 555                 560

Ser Trp Gly Ile Arg Leu Ala Val Trp Ala Ile Val Leu Leu Ser Val
                565                 570                 575

Leu Cys Asn Gly Leu Val Leu Leu Thr Val Phe Ala Gly Gly Pro Val
            580                 585                 590

Pro Leu Pro Pro Val Lys Phe Val Val Gly Ala Ile Ala Gly Ala Asn
        595                 600                 605

Thr Leu Thr Gly Ile Ser Cys Gly Leu Leu Ala Ser Val Asp Ala Leu
    610                 615                 620

Thr Phe Gly Gln Phe Ser Glu Tyr Gly Ala Arg Trp Glu Thr Gly Leu
625                 630                 635                 640

Gly Cys Arg Ala Thr Gly Phe Leu Ala Val Leu Gly Ser Glu Ala Ser
                645                 650                 655

Val Leu Leu Leu Thr Leu Ala Ala Val Gln Cys Ser Val Ser Val Ser
            660                 665                 670

Cys Val Arg Ala Tyr Gly Lys Ser Pro Ser Leu Gly Ser Val Arg Ala
        675                 680                 685

Gly Val Leu Gly Cys Leu Ala Leu Ala Gly Leu Ala Ala Ala Leu Pro
    690                 695                 700

Leu Ala Ser Val Gly Glu Tyr Gly Ala Ser Pro Leu Cys Leu Pro Tyr
705                 710                 715                 720

Ala Pro Pro Glu Gly Gln Pro Ala Ala Leu Gly Phe Thr Val Ala Leu
                725                 730                 735

Val Met Met Asn Ser Phe Cys Phe Leu Val Val Ala Gly Ala Tyr Ile
            740                 745                 750

Lys Leu Tyr Cys Asp Leu Pro Arg Gly Asp Phe Glu Ala Val Trp Asp
        755                 760                 765

Cys Ala Met Val Arg His Val Ala Trp Leu Ile Phe Ala Asp Gly Leu
    770                 775                 780

Leu Tyr Cys Pro Val Ala Phe Leu Ser Phe Ala Ser Met Leu Gly Leu
785                 790                 795                 800

Phe Pro Val Thr Pro Glu Ala Val Lys Ser Val Leu Leu Val Val Leu
                805                 810                 815

Pro Leu Pro Ala Cys Leu Asn Pro Leu Leu Tyr Leu Leu Phe Asn Pro
            820                 825                 830

His Phe Arg Asp Asp Leu Arg Arg Leu Arg Pro Arg Ala Gly Asp Ser
        835                 840                 845

Gly Pro Leu Ala Tyr Ala Ala Ala Gly Glu Leu Glu Lys Ser Ser Cys
    850                 855                 860

Asp Ser Thr Gln Ala Leu Val Ala Phe Ser Asp Val Asp Leu Ile Leu
865                 870                 875                 880

Glu Ala Ser Glu Ala Gly Arg Pro Pro Gly Leu Glu Thr Tyr Gly Phe
                885                 890                 895

Pro Ser Val Thr Leu Ile Ser Cys Gln Gln Pro Gly Ala Pro Arg Leu
            900                 905                 910

Glu Gly Ser His Cys Val Glu Pro Glu Gly Asn His Phe Gly Asn Pro
        915                 920                 925

Gln Pro Ser Met Asp Gly Glu Leu Leu Leu Arg Ala Glu Gly Ser Thr
    930                 935                 940

Pro Ala Gly Gly Gly Leu Ser Gly Gly Gly Gly Phe Gln Pro Ser Gly
945                 950                 955                 960

Leu Ala Phe Ala Ser His Val
                965

<210> 7
<211> 543
<212> PRT
<213> Artificial

<220>
<223> synthetic peptide

<400> 7

Ala Pro Gln Pro Gly Pro Gly Pro Thr Ala Cys Pro Ala Pro Cys His
1               5                   10                  15

Cys Gln Glu Asp Gly Ile Met Leu Ser Ala Asp Cys Ser Glu Leu Gly
            20                  25                  30

Leu Ser Ala Val Pro Gly Asp Leu Asp Pro Leu Thr Ala Tyr Leu Asp
        35                  40                  45

Leu Ser Met Asn Asn Leu Thr Glu Leu Gln Pro Gly Leu Phe His His
    50                  55                  60

Leu Arg Phe Leu Glu Glu Leu Arg Leu Ser Gly Asn His Leu Ser His
65                  70                  75                  80

Ile Pro Gly Gln Ala Phe Ser Gly Leu Tyr Ser Leu Lys Ile Leu Met
                85                  90                  95

Leu Gln Asn Asn Gln Leu Gly Gly Ile Pro Ala Glu Ala Leu Trp Glu
            100                 105                 110

Leu Pro Ser Leu Gln Ser Leu Arg Leu Asp Ala Asn Leu Ile Ser Leu
        115                 120                 125

Val Pro Glu Arg Ser Phe Glu Gly Leu Ser Ser Leu Arg His Leu Trp
    130                 135                 140

Leu Asp Asp Asn Ala Leu Thr Glu Ile Pro Val Arg Ala Leu Asn Asn
145                 150                 155                 160

Leu Pro Ala Leu Gln Ala Met Thr Leu Ala Leu Asn Arg Ile Ser His
                165                 170                 175

Ile Pro Asp Tyr Ala Phe Gln Asn Leu Thr Ser Leu Val Val Leu His
            180                 185                 190

Leu His Asn Asn Arg Ile Gln His Leu Gly Thr His Ser Phe Glu Gly
        195                 200                 205

Leu His Asn Leu Glu Thr Leu Asp Leu Asn Tyr Asn Lys Leu Gln Glu
    210                 215                 220

Phe Pro Val Ala Ile Arg Thr Leu Gly Arg Leu Gln Glu Leu Gly Phe
225                 230                 235                 240

His Asn Asn Asn Ile Lys Ala Ile Pro Glu Lys Ala Phe Met Gly Asn
                245                 250                 255

Pro Leu Leu Gln Thr Ile His Phe Tyr Asp Asn Pro Ile Gln Phe Val
            260                 265                 270

Gly Arg Ser Ala Phe Gln Tyr Leu Pro Lys Leu His Thr Leu Ser Leu
        275                 280                 285

Asn Gly Ala Met Asp Ile Gln Glu Phe Pro Asp Leu Lys Gly Thr Thr
    290                 295                 300

Ser Leu Glu Ile Leu Thr Leu Thr Arg Ala Gly Ile Arg Leu Leu Pro
305                 310                 315                 320

Ser Gly Met Cys Gln Gln Leu Pro Arg Leu Arg Val Leu Glu Leu Ser
                325                 330                 335

His Asn Gln Ile Glu Glu Leu Pro Ser Leu His Arg Cys Gln Lys Leu
            340                 345                 350

Glu Glu Ile Gly Leu Gln His Asn Arg Ile Trp Glu Ile Gly Ala Asp
        355                 360                 365

Thr Phe Ser Gln Leu Ser Ser Leu Gln Ala Leu Asp Leu Ser Trp Asn
    370                 375                 380

Ala Ile Arg Ser Ile His Pro Glu Ala Phe Ser Thr Leu His Ser Leu
385                 390                 395                 400

Val Lys Leu Asp Leu Thr Asp Asn Gln Leu Thr Thr Leu Pro Leu Ala
                405                 410                 415

Gly Leu Gly Gly Leu Met His Leu Lys Leu Lys Gly Asn Leu Ala Leu
            420                 425                 430

Ser Gln Ala Phe Ser Lys Asp Ser Phe Pro Lys Leu Arg Ile Leu Glu
        435                 440                 445

Val Pro Tyr Ala Tyr Gln Cys Cys Pro Tyr Gly Met Cys Ala Ser Phe
    450                 455                 460

Phe Lys Ala Ser Gly Gln Trp Glu Ala Glu Asp Leu His Leu Asp Asp
465                 470                 475                 480

Glu Glu Ser Ser Lys Arg Pro Leu Gly Leu Leu Ala Arg Gln Ala Glu
                485                 490                 495

Asn His Tyr Asp Gln Asp Leu Asp Glu Leu Gln Leu Glu Met Glu Asp
            500                 505                 510

Ser Lys Pro His Pro Ser Val Gln Cys Ser Pro Thr Pro Gly Pro Phe
        515                 520                 525

Lys Pro Cys Glu Tyr Leu Phe Glu Ser Trp Gly Ile Arg Leu Ala
    530                 535                 540

<210>  8
<211>  477
<212>  PRT
<213>  Artificial

<220>
<223>  synthetic peptide

<400>  8

Phe Leu Glu Glu Leu Arg Leu Ser Gly Asn His Leu Ser His Ile Pro
1               5                   10                  15

Gly Gln Ala Phe Ser Gly Leu Tyr Ser Leu Lys Ile Leu Met Leu Gln
            20                  25                  30

Asn Asn Gln Leu Gly Gly Ile Pro Ala Glu Ala Leu Trp Glu Leu Pro
        35                  40                  45

Ser Leu Gln Ser Leu Arg Leu Asp Ala Asn Leu Ile Ser Leu Val Pro
    50                  55                  60

Glu Arg Ser Phe Glu Gly Leu Ser Ser Leu Arg His Leu Trp Leu Asp
65                  70                  75                  80

Asp Asn Ala Leu Thr Glu Ile Pro Val Arg Ala Leu Asn Asn Leu Pro
                85                  90                  95

Ala Leu Gln Ala Met Thr Leu Ala Leu Asn Arg Ile Ser His Ile Pro
            100                 105                 110

Asp Tyr Ala Phe Gln Asn Leu Thr Ser Leu Val Val Leu His Leu His
        115                 120                 125

Asn Asn Arg Ile Gln His Leu Gly Thr His Ser Phe Glu Gly Leu His
    130                 135                 140

Asn Leu Glu Thr Leu Asp Leu Asn Tyr Asn Lys Leu Gln Glu Phe Pro
145                 150                 155                 160

Val Ala Ile Arg Thr Leu Gly Arg Leu Gln Glu Leu Gly Phe His Asn
                165                 170                 175

Asn Asn Ile Lys Ala Ile Pro Glu Lys Ala Phe Met Gly Asn Pro Leu
            180                 185                 190

Leu Gln Thr Ile His Phe Tyr Asp Asn Pro Ile Gln Phe Val Gly Arg
        195                 200                 205

Ser Ala Phe Gln Tyr Leu Pro Lys Leu His Thr Leu Ser Leu Asn Gly
    210                 215                 220

Ala Met Asp Ile Gln Glu Phe Pro Asp Leu Lys Gly Thr Thr Ser Leu
225                 230                 235                 240

Glu Ile Leu Thr Leu Thr Arg Ala Gly Ile Arg Leu Leu Pro Ser Gly
                245                 250                 255

Met Cys Gln Gln Leu Pro Arg Leu Arg Val Leu Glu Leu Ser His Asn
            260                 265                 270

Gln Ile Glu Glu Leu Pro Ser Leu His Arg Cys Gln Lys Leu Glu Glu
        275                 280                 285

Ile Gly Leu Gln His Asn Arg Ile Trp Glu Ile Gly Ala Asp Thr Phe
    290                 295                 300

Ser Gln Leu Ser Ser Leu Gln Ala Leu Asp Leu Ser Trp Asn Ala Ile
305                 310                 315                 320

Arg Ser Ile His Pro Glu Ala Phe Ser Thr Leu His Ser Leu Val Lys
                325                 330                 335

Leu Asp Leu Thr Asp Asn Gln Leu Thr Thr Leu Pro Leu Ala Gly Leu
            340                 345                 350

Gly Gly Leu Met His Leu Lys Leu Lys Gly Asn Leu Ala Leu Ser Gln
        355                 360                 365

Ala Phe Ser Lys Asp Ser Phe Pro Lys Leu Arg Ile Leu Glu Val Pro
    370                 375                 380

Tyr Ala Tyr Gln Cys Cys Pro Tyr Gly Met Cys Ala Ser Phe Phe Lys
385                 390                 395                 400

Ala Ser Gly Gln Trp Glu Ala Glu Asp Leu His Leu Asp Asp Glu Glu

405                    410                    415

Ser Ser Lys Arg Pro Leu Gly Leu Leu Ala Arg Gln Ala Glu Asn His
            420                    425                    430

Tyr Asp Gln Asp Leu Asp Glu Leu Gln Leu Glu Met Glu Asp Ser Lys
        435                    440                    445

Pro His Pro Ser Val Gln Cys Ser Pro Thr Pro Gly Pro Phe Lys Pro
    450                    455                    460

Cys Glu Tyr Leu Phe Glu Ser Trp Gly Ile Arg Leu Ala
465                    470                    475

<210>  9
<211>  249
<212>  PRT
<213>  Artificial

<220>
<223>  synthetic peptide

<400>  9

Gln Glu Phe Pro Asp Leu Lys Gly Thr Thr Ser Leu Glu Ile Leu Thr
1               5                   10                  15

Leu Thr Arg Ala Gly Ile Arg Leu Leu Pro Ser Gly Met Cys Gln Gln
            20                  25                  30

Leu Pro Arg Leu Arg Val Leu Glu Leu Ser His Asn Gln Ile Glu Glu
        35                  40                  45

Leu Pro Ser Leu His Arg Cys Gln Lys Leu Glu Glu Ile Gly Leu Gln
    50                  55                  60

His Asn Arg Ile Trp Glu Ile Gly Ala Asp Thr Phe Ser Gln Leu Ser
65                  70                  75                  80

Ser Leu Gln Ala Leu Asp Leu Ser Trp Asn Ala Ile Arg Ser Ile His
                85                  90                  95

Pro Glu Ala Phe Ser Thr Leu His Ser Leu Val Lys Leu Asp Leu Thr
            100                 105                 110

Asp Asn Gln Leu Thr Thr Leu Pro Leu Ala Gly Leu Gly Gly Leu Met
        115                 120                 125

His Leu Lys Leu Lys Gly Asn Leu Ala Leu Ser Gln Ala Phe Ser Lys
    130                 135                 140

Asp Ser Phe Pro Lys Leu Arg Ile Leu Glu Val Pro Tyr Ala Tyr Gln
145                 150                 155                 160

Cys Cys Pro Tyr Gly Met Cys Ala Ser Phe Phe Lys Ala Ser Gly Gln
                165                 170                 175

Trp Glu Ala Glu Asp Leu His Leu Asp Asp Glu Glu Ser Ser Lys Arg
            180                 185                 190

Pro Leu Gly Leu Leu Ala Arg Gln Ala Glu Asn His Tyr Asp Gln Asp
        195                 200                 205

Leu Asp Glu Leu Gln Leu Glu Met Glu Asp Ser Lys Pro His Pro Ser
    210                 215                 220

Val Gln Cys Ser Pro Thr Pro Gly Pro Phe Lys Pro Cys Glu Tyr Leu
225                 230                 235                 240

Phe Glu Ser Trp Gly Ile Arg Leu Ala
                245

<210>  10
<211>  124
<212>  PRT
<213>  Artificial

<220>
<223> synthetic peptide

<400> 10

Gly Leu Met His Leu Lys Leu Lys Gly Asn Leu Ala Leu Ser Gln Ala
1               5                   10                  15

Phe Ser Lys Asp Ser Phe Pro Lys Leu Arg Ile Leu Glu Val Pro Tyr
            20                  25                  30

Ala Tyr Gln Cys Cys Pro Tyr Gly Met Cys Ala Ser Phe Phe Lys Ala
        35                  40                  45

Ser Gly Gln Trp Glu Ala Glu Asp Leu His Leu Asp Asp Glu Glu Ser
    50                  55                  60

Ser Lys Arg Pro Leu Gly Leu Leu Ala Arg Gln Ala Glu Asn His Tyr
65                  70                  75                  80

Asp Gln Asp Leu Asp Glu Leu Gln Leu Glu Met Glu Asp Ser Lys Pro
                85                  90                  95

His Pro Ser Val Gln Cys Ser Pro Thr Pro Gly Pro Phe Lys Pro Cys
            100                 105                 110

Glu Tyr Leu Phe Glu Ser Trp Gly Ile Arg Leu Ala
        115                 120

<210> 11
<211> 828
<212> PRT
<213> Artificial

<220>
<223> synthetic peptide

<400> 11

Met Arg Leu Glu Gly Glu Gly Arg Ser Ala Arg Ala Gly Gln Asn Leu
1               5                   10                  15

Ser Arg Ala Gly Ser Ala Arg Arg Gly Ala Pro Arg Asp Leu Ser Met
            20                  25                  30

Asn Asn Leu Thr Glu Leu Gln Pro Gly Leu Phe His His Leu Arg Phe
        35                  40                  45

Leu Glu Glu Leu Arg Leu Ser Gly Asn His Leu Ser His Ile Pro Gly
    50                  55                  60

Gln Ala Phe Ser Gly Leu Tyr Ser Leu Lys Ile Leu Met Leu Gln Asn
65                  70                  75                  80

Asn Gln Leu Gly Gly Ile Pro Ala Glu Ala Leu Trp Glu Leu Pro Ser
                85                  90                  95

Leu Gln Ser Leu Asp Leu Asn Tyr Asn Lys Leu Gln Glu Phe Pro Val
            100                 105                 110

Ala Ile Arg Thr Leu Gly Arg Leu Gln Glu Leu Gly Phe His Asn Asn
        115                 120                 125

Asn Ile Lys Ala Ile Pro Glu Lys Ala Phe Met Gly Asn Pro Leu Leu
    130                 135                 140

Gln Thr Ile His Phe Tyr Asp Asn Pro Ile Gln Phe Val Gly Arg Ser
145                 150                 155                 160

Ala Phe Gln Tyr Leu Pro Lys Leu His Thr Leu Ser Leu Asn Gly Ala
                165                 170                 175

Met Asp Ile Gln Glu Phe Pro Asp Leu Lys Gly Thr Thr Ser Leu Glu
            180                 185                 190

Ile Leu Thr Leu Thr Arg Ala Gly Ile Arg Leu Leu Pro Ser Gly Met
        195                 200                 205

Cys Gln Gln Leu Pro Arg Leu Arg Val Leu Glu Leu Ser His Asn Gln
    210                 215                 220

Ile Glu Glu Leu Pro Ser Leu His Arg Cys Gln Lys Leu Glu Glu Ile
225                 230                 235                 240

Gly Leu Gln His Asn Arg Ile Trp Glu Ile Gly Ala Asp Thr Phe Ser
                245                 250                 255

Gln Leu Ser Ser Leu Gln Ala Leu Asp Leu Ser Trp Asn Ala Ile Arg
            260                 265                 270

Ser Ile His Pro Glu Ala Phe Ser Thr Leu His Ser Leu Val Lys Leu
        275                 280                 285

Asp Leu Thr Asp Asn Gln Leu Thr Thr Leu Pro Leu Ala Gly Leu Gly
    290                 295                 300

Gly Leu Met His Leu Lys Leu Lys Gly Asn Leu Ala Leu Ser Gln Ala
305                 310                 315                 320

Phe Ser Lys Asp Ser Phe Pro Lys Leu Arg Ile Leu Glu Val Pro Tyr
                325                 330                 335

Ala Tyr Gln Cys Cys Pro Tyr Gly Met Cys Ala Ser Phe Phe Lys Ala
            340                 345                 350

Ser Gly Gln Trp Glu Ala Glu Asp Leu His Leu Asp Asp Glu Glu Ser
        355                 360                 365

Ser Lys Arg Pro Leu Gly Leu Leu Ala Arg Gln Ala Glu Asn His Tyr
    370                 375                 380

Asp Gln Asp Leu Asp Glu Leu Gln Leu Glu Met Glu Asp Ser Lys Pro
385                 390                 395                 400

His Pro Ser Val Gln Cys Ser Pro Thr Pro Gly Pro Phe Lys Pro Cys
                405                 410                 415

Glu Tyr Leu Phe Glu Ser Trp Gly Ile Arg Leu Ala Val Trp Ala Ile
            420                 425                 430

Val Leu Leu Ser Val Leu Cys Asn Gly Leu Val Leu Leu Thr Val Phe
        435                 440                 445

Ala Gly Gly Pro Val Pro Leu Pro Pro Val Lys Phe Val Val Gly Ala
    450                 455                 460

Ile Ala Gly Ala Asn Thr Leu Thr Gly Ile Ser Cys Gly Leu Leu Ala
465                 470                 475                 480

Ser Val Asp Ala Leu Thr Phe Gly Gln Phe Ser Glu Tyr Gly Ala Arg
                485                 490                 495

Trp Glu Thr Gly Leu Gly Cys Arg Ala Thr Gly Phe Leu Ala Val Leu
            500                 505                 510

Gly Ser Glu Ala Ser Val Leu Leu Leu Thr Leu Ala Ala Val Gln Cys
        515                 520                 525

Ser Val Ser Val Ser Cys Val Arg Ala Tyr Gly Lys Ser Pro Ser Leu
    530                 535                 540

Gly Ser Val Arg Ala Gly Val Leu Gly Cys Leu Ala Leu Ala Gly Leu
545                 550                 555                 560

Ala Ala Ala Leu Pro Leu Ala Ser Val Gly Glu Tyr Gly Ala Ser Pro
                565                 570                 575

Leu Cys Leu Pro Tyr Ala Pro Pro Glu Gly Gln Pro Ala Ala Leu Gly
            580                 585                 590

Phe Thr Val Ala Leu Val Met Met Asn Ser Phe Cys Phe Leu Val Val

        595                 600                 605

Ala Gly Ala Tyr Ile Lys Leu Tyr Cys Asp Leu Pro Arg Gly Asp Phe
    610                 615                 620

Glu Ala Val Trp Asp Cys Ala Met Val Arg His Val Ala Trp Leu Ile
625                 630                 635                 640

Phe Ala Asp Gly Leu Leu Tyr Cys Pro Val Ala Phe Leu Ser Phe Ala
                645                 650                 655

Ser Met Leu Gly Leu Phe Pro Val Thr Pro Glu Ala Val Lys Ser Val
            660                 665                 670

Leu Leu Val Val Leu Pro Leu Pro Ala Cys Leu Asn Pro Leu Leu Tyr
        675                 680                 685

Leu Leu Phe Asn Pro His Phe Arg Asp Asp Leu Arg Arg Leu Arg Pro
    690                 695                 700

Arg Ala Gly Asp Ser Gly Pro Leu Ala Tyr Ala Ala Ala Gly Glu Leu
705                 710                 715                 720

Glu Lys Ser Ser Cys Asp Ser Thr Gln Ala Leu Val Ala Phe Ser Asp
                725                 730                 735

Val Asp Leu Ile Leu Glu Ala Ser Glu Ala Gly Arg Pro Pro Gly Leu
            740                 745                 750

Glu Thr Tyr Gly Phe Pro Ser Val Thr Leu Ile Ser Cys Gln Gln Pro
        755                 760                 765

Gly Ala Pro Arg Leu Glu Gly Ser His Cys Val Glu Pro Glu Gly Asn
    770                 775                 780

His Phe Gly Asn Pro Gln Pro Ser Met Asp Gly Glu Leu Leu Leu Arg
785                 790                 795                 800

Ala Glu Gly Ser Thr Pro Ala Gly Gly Gly Leu Ser Gly Gly Gly Gly
                805                 810                 815

Phe Gln Pro Ser Gly Leu Ala Phe Ala Ser His Val
            820                 825

<210>  12
<211>  915
<212>  PRT
<213>  Artificial

<220>
<223>  synthetic peptide

<400>  12

Met Gly Arg Pro Arg Leu Thr Leu Val Cys Gln Val Ser Ile Ile Ile
1               5                   10                  15

Ser Ala Arg Asp Leu Ser Met Asn Asn Leu Thr Glu Leu Gln Pro Gly
            20                  25                  30

Leu Phe His His Leu Arg Phe Leu Glu Glu Leu Arg Leu Ser Gly Asn
        35                  40                  45

His Leu Ser His Ile Pro Gly Gln Ala Phe Ser Gly Leu Tyr Ser Leu
    50                  55                  60

Lys Ile Leu Met Leu Gln Asn Asn Gln Leu Gly Gly Ile Pro Ala Glu
65                  70                  75                  80

Ala Leu Trp Glu Leu Pro Ser Leu Gln Ser Leu Arg Leu Asp Ala Asn
                85                  90                  95

Leu Ile Ser Leu Val Pro Glu Arg Ser Phe Glu Gly Leu Ser Ser Leu
            100                 105                 110

Arg His Leu Trp Leu Asp Asp Asn Ala Leu Thr Glu Ile Pro Val Arg

115                 120                 125

Ala Leu Asn Asn Leu Pro Ala Leu Gln Ala Met Thr Leu Ala Leu Asn
    130                 135                 140

Arg Ile Ser His Ile Pro Asp Tyr Ala Phe Gln Asn Leu Thr Ser Leu
145                 150                 155                 160

Val Val Leu His Leu His Asn Asn Arg Ile Gln His Leu Gly Thr His
                165                 170                 175

Ser Phe Glu Gly Leu His Asn Leu Glu Thr Leu Asp Leu Asn Tyr Asn
            180                 185                 190

Lys Leu Gln Glu Phe Pro Val Ala Ile Arg Thr Leu Gly Arg Leu Gln
        195                 200                 205

Glu Leu Gly Phe His Asn Asn Asn Ile Lys Ala Ile Pro Glu Lys Ala
    210                 215                 220

Phe Met Gly Asn Pro Leu Leu Gln Thr Ile His Phe Tyr Asp Asn Pro
225                 230                 235                 240

Ile Gln Phe Val Gly Arg Ser Ala Phe Gln Tyr Leu Pro Lys Leu His
                245                 250                 255

Thr Leu Ser Leu Asn Gly Ala Met Asp Ile Gln Glu Phe Pro Asp Leu
            260                 265                 270

Lys Gly Thr Thr Ser Leu Glu Ile Leu Thr Leu Thr Arg Ala Gly Ile
        275                 280                 285

Arg Leu Leu Pro Ser Gly Met Cys Gln Gln Leu Pro Arg Leu Arg Val
    290                 295                 300

Leu Glu Leu Ser His Asn Gln Ile Glu Glu Leu Pro Ser Leu His Arg
305                 310                 315                 320

Cys Gln Lys Leu Glu Glu Ile Gly Leu Gln His Asn Arg Ile Trp Glu
                325                 330                 335

Ile Gly Ala Asp Thr Phe Ser Gln Leu Ser Ser Leu Gln Ala Leu Asp
            340                 345                 350

Leu Ser Trp Asn Ala Ile Arg Ser Ile His Pro Glu Ala Phe Ser Thr
        355                 360                 365

Leu His Ser Leu Val Lys Leu Asp Leu Thr Asp Asn Gln Leu Thr Thr
    370                 375                 380

Leu Pro Leu Ala Gly Leu Gly Gly Leu Met His Leu Lys Leu Lys Gly
385                 390                 395                 400

Asn Leu Ala Leu Ser Gln Ala Phe Ser Lys Asp Ser Phe Pro Lys Leu
                405                 410                 415

Arg Ile Leu Glu Val Pro Tyr Ala Tyr Gln Cys Cys Pro Tyr Gly Met
            420                 425                 430

Cys Ala Ser Phe Phe Lys Ala Ser Gly Gln Trp Glu Ala Glu Asp Leu
        435                 440                 445

His Leu Asp Asp Glu Glu Ser Ser Lys Arg Pro Leu Gly Leu Leu Ala
    450                 455                 460

Arg Gln Ala Glu Asn His Tyr Asp Gln Asp Leu Asp Glu Leu Gln Leu
465                 470                 475                 480

Glu Met Glu Asp Ser Lys Pro His Pro Ser Val Gln Cys Ser Pro Thr
                485                 490                 495

Pro Gly Pro Phe Lys Pro Cys Glu Tyr Leu Phe Glu Ser Trp Gly Ile
            500                 505                 510

Arg Leu Ala Val Trp Ala Ile Val Leu Leu Ser Val Leu Cys Asn Gly
        515                 520                 525

Leu Val Leu Leu Thr Val Phe Ala Gly Gly Pro Val Pro Leu Pro Pro
    530                 535                 540

Val Lys Phe Val Val Gly Ala Ile Ala Gly Ala Asn Thr Leu Thr Gly
545                 550                 555                 560

Ile Ser Cys Gly Leu Leu Ala Ser Val Asp Ala Leu Thr Phe Gly Gln
                565                 570                 575

Phe Ser Glu Tyr Gly Ala Arg Trp Glu Thr Gly Leu Gly Cys Arg Ala
            580                 585                 590

Thr Gly Phe Leu Ala Val Leu Gly Ser Glu Ala Ser Val Leu Leu Leu
        595                 600                 605

Thr Leu Ala Ala Val Gln Cys Ser Val Ser Val Ser Cys Val Arg Ala
    610                 615                 620

Tyr Gly Lys Ser Pro Ser Leu Gly Ser Val Arg Ala Gly Val Leu Gly
625                 630                 635                 640

Cys Leu Ala Leu Ala Gly Leu Ala Ala Ala Leu Pro Leu Ala Ser Val
                645                 650                 655

Gly Glu Tyr Gly Ala Ser Pro Leu Cys Leu Pro Tyr Ala Pro Pro Glu
            660                 665                 670

Gly Gln Pro Ala Ala Leu Gly Phe Thr Val Ala Leu Val Met Met Asn
        675                 680                 685

Ser Phe Cys Phe Leu Val Val Ala Gly Ala Tyr Ile Lys Leu Tyr Cys
    690                 695                 700

Asp Leu Pro Arg Gly Asp Phe Glu Ala Val Trp Asp Cys Ala Met Val
705                 710                 715                 720

Arg His Val Ala Trp Leu Ile Phe Ala Asp Gly Leu Leu Tyr Cys Pro
                725                 730                 735

Val Ala Phe Leu Ser Phe Ala Ser Met Leu Gly Leu Phe Pro Val Thr
            740                 745                 750

Pro Glu Ala Val Lys Ser Val Leu Leu Val Val Leu Pro Leu Pro Ala
        755                 760                 765

Cys Leu Asn Pro Leu Leu Tyr Leu Leu Phe Asn Pro His Phe Arg Asp
    770                 775                 780

Asp Leu Arg Arg Leu Arg Pro Arg Ala Gly Asp Ser Gly Pro Leu Ala
785                 790                 795                 800

Tyr Ala Ala Ala Gly Glu Leu Glu Lys Ser Ser Cys Asp Ser Thr Gln
                805                 810                 815

Ala Leu Val Ala Phe Ser Asp Val Asp Leu Ile Leu Glu Ala Ser Glu
            820                 825                 830

Ala Gly Arg Pro Pro Gly Leu Glu Thr Tyr Gly Phe Pro Ser Val Thr
        835                 840                 845

Leu Ile Ser Cys Gln Gln Pro Gly Ala Pro Arg Leu Glu Gly Ser His
    850                 855                 860

Cys Val Glu Pro Glu Gly Asn His Phe Gly Asn Pro Gln Pro Ser Met
865                 870                 875                 880

Asp Gly Glu Leu Leu Leu Arg Ala Glu Gly Ser Thr Pro Ala Gly Gly
                885                 890                 895

Gly Leu Ser Gly Gly Gly Gly Phe Gln Pro Ser Gly Leu Ala Phe Ala

900                 905                 910

Ser His Val
        915

<210>  13
<211>  747
<212>  DNA
<213>  Artificial

<220>
<223>  synthetic DNA

<400>  13
caggagtttc cagatctcaa aggcaccacc agcctggaga tcctgaccct gacccgcgca      60

ggcatccggc tgctcccatc ggggatgtgc caacagctgc ccaggctccg agtcctggaa     120

ctgtctcaca atcaaattga ggagctgccc agcctgcaca ggtgtcagaa attggaggaa     180

atcggcctcc aacacaaccg catctgggaa attggagctg acaccttcag ccagctgagc     240

tccctgcaag ccctggatct tagctggaac gccatccggt ccatccaccc cgaggccttc     300

tccaccctgc actccctggt caagctggac ctgacagaca accagctgac cacactgccc     360

ctggctggac ttgggggctt gatgcatctg aagctcaaag ggaaccttgc tctctcccag     420

gccttctcca aggacagttt cccaaaactg aggatcctgg aggtgcctta tgcctaccag     480

tgctgtccct atgggatgtg tgccagcttc ttcaaggcct ctgggcagtg ggaggctgaa     540

gaccttcacc ttgatgatga ggagtcttca aaaaggcccc tgggcctcct tgccagacaa     600

gcagagaacc actatgacca ggacctggat gagctccagc tggagatgga ggactcaaag     660

ccacacccca gtgtccagtg tagccctact ccaggcccct tcaagccctg tgagtacctc     720

tttgaaagct ggggcatccg cctggcc                                         747

<210>  14
<211>  37
<212>  DNA
<213>  Artificial

<220>

<223>  synthetic DNA

<400>  14
tttgaattcg atgcaggagt ttccagatct caaaggc                                37

<210>  15
<211>  33
<212>  DNA
<213>  Artificial

<220>
<223>  synthetic DNA

<400>  15
tttgcggccg ccacgtgtga agcaaaggcc aag                                    33

<210>  16
<211>  45
<212>  DNA
<213>  Artificial

<220>
<223>  synthetic DNA

<400>  16
ctaatacgac tcactatagg gcaagcagtg gtatcaacgc agagt                       45

<210>  17
<211>  22
<212>  DNA
<213>  Artificial

<220>
<223>  synthetic DNA

<400>  17
ctaatacgac tcactatagg gc                                                22

<210>  18
<211>  34
<212>  DNA
<213>  Artificial

<220>
<223>  synthetic DNA

<400> 18
ccaggggcca gtggatagac agatgggggt gtcg 34

<210> 19
<211> 26
<212> DNA
<213> Artificial

<220>
<223> synthetic DNA

<400> 19
gcacctccag atgttaactg ctcact 26

<210> 20
<211> 20
<212> DNA
<213> Artificial

<220>
<223> synthetic DNA

<400> 20
gtcacgacgt tgtaaaacga 20

<210> 21
<211> 20
<212> DNA
<213> Artificial

<220>
<223> synthetic DNA

<400> 21
atttaggtga cactatagaa 20

<210> 22
<211> 421
<212> DNA
<213> Artificial

<220>
<223> synthetic DNA

<220>
<221> CDS
<222> (1)..(420)

<400> 22
atg gaa agg cac tgg atc ttt ctc ttc ctg ttg tca gta act gca ggt 48
Met Glu Arg His Trp Ile Phe Leu Phe Leu Leu Ser Val Thr Ala Gly
1               5                   10                  15

gtc cac tcc cag gtc cag ctg cag cag tct gca gct gaa ctg gca aga 96
Val His Ser Gln Val Gln Leu Gln Gln Ser Ala Ala Glu Leu Ala Arg
            20                  25                  30

cct ggg gcc tca gtg aag atg tcc tgc aag gct tct ggc tac acc ttt 144
Pro Gly Ala Ser Val Lys Met Ser Cys Lys Ala Ser Gly Tyr Thr Phe
        35                  40                  45

acc acc tac acg atg cac tgg gta aaa cag agg cct gga cag ggt ctg 192
Thr Thr Tyr Thr Met His Trp Val Lys Gln Arg Pro Gly Gln Gly Leu
    50                  55                  60

gaa tgg att gga tac att aat cct aac aat gga tat act gaa ttc aat 240
Glu Trp Ile Gly Tyr Ile Asn Pro Asn Asn Gly Tyr Thr Glu Phe Asn
65                  70                  75                  80

cag aag ttc aag gac aag acc aca ttg act gca gac aaa tcc tcc agc 288
Gln Lys Phe Lys Asp Lys Thr Thr Leu Thr Ala Asp Lys Ser Ser Ser
                85                  90                  95

aca gcc tac atg caa ctg agc agc ctg aca tct gag gac tct gcg gtc 336
Thr Ala Tyr Met Gln Leu Ser Ser Leu Thr Ser Glu Asp Ser Ala Val
            100                 105                 110

tat tac tgt gca aga agg gat tac tat aga ttt tcg acg ggg ttt gct 384
Tyr Tyr Cys Ala Arg Arg Asp Tyr Tyr Arg Phe Ser Thr Gly Phe Ala
        115                 120                 125

tac tgg ggc caa ggg act ctg gtc act gtc tct gca g 421
Tyr Trp Gly Gln Gly Thr Leu Val Thr Val Ser Ala
    130                 135                 140


<210> 23
<211> 140
<212> PRT
<213> Artificial

<220>

<223>  synthetic peptide

<400>  23

Met Glu Arg His Trp Ile Phe Leu Phe Leu Leu Ser Val Thr Ala Gly
1               5                   10                  15

Val His Ser Gln Val Gln Leu Gln Gln Ser Ala Ala Glu Leu Ala Arg
            20                  25                  30

Pro Gly Ala Ser Val Lys Met Ser Cys Lys Ala Ser Gly Tyr Thr Phe
        35                  40                  45

Thr Thr Tyr Thr Met His Trp Val Lys Gln Arg Pro Gly Gln Gly Leu
    50                  55                  60

Glu Trp Ile Gly Tyr Ile Asn Pro Asn Asn Gly Tyr Thr Glu Phe Asn
65                  70                  75                  80

Gln Lys Phe Lys Asp Lys Thr Thr Leu Thr Ala Asp Lys Ser Ser Ser
                85                  90                  95

Thr Ala Tyr Met Gln Leu Ser Ser Leu Thr Ser Glu Asp Ser Ala Val
            100                 105                 110

Tyr Tyr Cys Ala Arg Arg Asp Tyr Tyr Arg Phe Ser Thr Gly Phe Ala
        115                 120                 125

Tyr Trp Gly Gln Gly Thr Leu Val Thr Val Ser Ala
    130                 135                 140

<210>  24
<211>  394
<212>  DNA
<213>  Artificial

<220>
<223>  synthetic DNA

<220>
<221> CDS
<222> (1)..(393)

<400> 24
atg aag ttg cct gtt agg ctg ttg gtg ctg atg ttc tgg att cct gct 48
Met Lys Leu Pro Val Arg Leu Leu Val Leu Met Phe Trp Ile Pro Ala
1               5                   10                  15

tcc agc agt gat gtt gtg atg acc caa act cca ctc tcc ctg cct gtc 96
Ser Ser Ser Asp Val Val Met Thr Gln Thr Pro Leu Ser Leu Pro Val
            20                  25                  30

agt ctt gga gat caa gcc tcc atc tct tgc aga tct agt cag agc ctt 144
Ser Leu Gly Asp Gln Ala Ser Ile Ser Cys Arg Ser Ser Gln Ser Leu
        35                  40                  45

gta cac act aat gga aac acc tat tta cat tgg tac ctg cag aag cca 192
Val His Thr Asn Gly Asn Thr Tyr Leu His Trp Tyr Leu Gln Lys Pro
    50                  55                  60

ggc cag tct ccg aag ctc ctg atc tac aaa gtt tcc aac cga ttt tct 240
Gly Gln Ser Pro Lys Leu Leu Ile Tyr Lys Val Ser Asn Arg Phe Ser
65                  70                  75                  80

ggg gtc cca gac agg ttc agt ggc agt gga tca ggg aca gat ttc aca 288
Gly Val Pro Asp Arg Phe Ser Gly Ser Gly Ser Gly Thr Asp Phe Thr
                85                  90                  95

ctc aag atc agc aga gtg gag gct gag gat ctg gga gtt tat ttc tgc 336
Leu Lys Ile Ser Arg Val Glu Ala Glu Asp Leu Gly Val Tyr Phe Cys
            100                 105                 110

tct caa agt aca cat gtt ccg tac acg ttc gga ggg ggg acc aag ctg 384
Ser Gln Ser Thr His Val Pro Tyr Thr Phe Gly Gly Gly Thr Lys Leu
        115                 120                 125

gaa ata aaa c 394
Glu Ile Lys
    130

<210> 25
<211> 131
<212> PRT
<213> Artificial

<220>
<223> synthetic peptide

<400>  25

Met Lys Leu Pro Val Arg Leu Leu Val Leu Met Phe Trp Ile Pro Ala
1               5                   10                  15

Ser Ser Ser Asp Val Val Met Thr Gln Thr Pro Leu Ser Leu Pro Val
            20                  25                  30

Ser Leu Gly Asp Gln Ala Ser Ile Ser Cys Arg Ser Ser Gln Ser Leu
        35                  40                  45

Val His Thr Asn Gly Asn Thr Tyr Leu His Trp Tyr Leu Gln Lys Pro
    50                  55                  60

Gly Gln Ser Pro Lys Leu Leu Ile Tyr Lys Val Ser Asn Arg Phe Ser
65                  70                  75                  80

Gly Val Pro Asp Arg Phe Ser Gly Ser Gly Ser Gly Thr Asp Phe Thr
                85                  90                  95

Leu Lys Ile Ser Arg Val Glu Ala Glu Asp Leu Gly Val Tyr Phe Cys
            100                 105                 110

Ser Gln Ser Thr His Val Pro Tyr Thr Phe Gly Gly Gly Thr Lys Leu
        115                 120                 125

Glu Ile Lys
    130

<210>  26
<211>  24
<212>  DNA
<213>  Artificial

<220>
<223>  synthetic DNA

<220>

<221>  CDS
<222>  (1)..(24)

<400>  26
ggc tac acc ttt acc acc tac acg                                          24
Gly Tyr Thr Phe Thr Thr Tyr Thr
1               5


<210>  27
<211>  8
<212>  PRT
<213>  Artificial

<220>
<223>  synthetic peptide

<400>  27

Gly Tyr Thr Phe Thr Thr Tyr Thr
1               5


<210>  28
<211>  24
<212>  DNA
<213>  Artificial

<220>
<223>  synthetic DNA


<220>
<221>  CDS
<222>  (1)..(24)

<400>  28
att aat cct aac aat gga tat act                                          24
Ile Asn Pro Asn Asn Gly Tyr Thr
1               5


<210>  29
<211>  8
<212>  PRT
<213>  Artificial

<220>
<223>  synthetic peptide

<400>  29

Ile Asn Pro Asn Asn Gly Tyr Thr
1               5

<210>  30
<211>  42
<212>  DNA
<213>  Artificial

<220>
<223>  synthetic DNA

<220>
<221>  CDS
<222>  (1)..(42)

<400>  30
gca aga agg gat tac tat aga ttt tcg acg ggg ttt gct tac                42
Ala Arg Arg Asp Tyr Tyr Arg Phe Ser Thr Gly Phe Ala Tyr
1               5                   10

<210>  31
<211>  14
<212>  PRT
<213>  Artificial

<220>
<223>  synthetic peptide

<400>  31

Ala Arg Arg Asp Tyr Tyr Arg Phe Ser Thr Gly Phe Ala Tyr
1               5                   10

<210>  32
<211>  33
<212>  DNA
<213>  Artificial

<220>
<223>  synthetic DNA

<220>
<221> CDS
<222> (1)..(33)

<400> 32
cag agc ctt gta cac act aat gga aac acc tat 33
Gln Ser Leu Val His Thr Asn Gly Asn Thr Tyr
1               5                   10

<210> 33
<211> 11
<212> PRT
<213> Artificial

<220>
<223> synthetic peptide

<400> 33

Gln Ser Leu Val His Thr Asn Gly Asn Thr Tyr
1               5                   10

<210> 34
<211> 9
<212> DNA
<213> Artificial

<220>
<223> synthetic DNA

<400> 34
aaagtttcc 9

<210> 35
<211> 3
<212> PRT
<213> Artificial

<220>
<223> synthetic peptide

<400> 35

Lys Val Ser
1

<210> 36
<211> 27
<212> DNA
<213> Artificial

<220>
<223> synthetic DNA


<220>
<221> CDS
<222> (1)..(27)

<400> 36
tct caa agt aca cat gtt ccg tac acg 27
Ser Gln Ser Thr His Val Pro Tyr Thr
1               5


<210> 37
<211> 9
<212> PRT
<213> Artificial

<220>
<223> synthetic peptide

<400> 37

Ser Gln Ser Thr His Val Pro Tyr Thr
1               5


<210> 38
<211> 801
<212> DNA
<213> Artificial

<220>
<223> synthetic DNA


<220>
<221> CDS
<222> (1)..(801)

<400> 38
cag ttc ccc aat ctt aca gga act gtc cac ctg gaa agt ctg act ttg 48

Gln Phe Pro Asn Leu Thr Gly Thr Val His Leu Glu Ser Leu Thr Leu
1               5                   10                  15

aca ggt aca aag ata agc agc ata cct aat aat ttg tgt caa gaa caa      96
Thr Gly Thr Lys Ile Ser Ser Ile Pro Asn Asn Leu Cys Gln Glu Gln
            20                  25                  30

aag atg ctt agg act ttg gac ttg tct tac aat aat ata aga gac ctt      144
Lys Met Leu Arg Thr Leu Asp Leu Ser Tyr Asn Asn Ile Arg Asp Leu
        35                  40                  45

cca agt ttt aat ggt tgc cat gct ctg gaa gaa att tct tta cag cgt      192
Pro Ser Phe Asn Gly Cys His Ala Leu Glu Glu Ile Ser Leu Gln Arg
    50                  55                  60

aat caa atc tac caa ata aag gaa ggc acc ttt caa ggc ctg ata tct      240
Asn Gln Ile Tyr Gln Ile Lys Glu Gly Thr Phe Gln Gly Leu Ile Ser
65                  70                  75                  80

cta agg att cta gat ctg agt aga aac ctg ata cat gaa att cac agt      288
Leu Arg Ile Leu Asp Leu Ser Arg Asn Leu Ile His Glu Ile His Ser
                85                  90                  95

aga gct ttt gcc aca ctt ggg cca ata act aac cta gat gta agt ttc      336
Arg Ala Phe Ala Thr Leu Gly Pro Ile Thr Asn Leu Asp Val Ser Phe
            100                 105                 110

aat gaa tta act tcc ttt cct acg gaa ggc ctg aat ggg cta aat caa      384
Asn Glu Leu Thr Ser Phe Pro Thr Glu Gly Leu Asn Gly Leu Asn Gln
        115                 120                 125

ctg aaa ctt gtg ggc aac ttc aag ctg aaa gaa gcc tta gca gca aaa      432
Leu Lys Leu Val Gly Asn Phe Lys Leu Lys Glu Ala Leu Ala Ala Lys
    130                 135                 140

gac ttt gtt aac ctc agg tct tta tca gta cca tat gct tat cag tgc      480
Asp Phe Val Asn Leu Arg Ser Leu Ser Val Pro Tyr Ala Tyr Gln Cys
145                 150                 155                 160

tgt gca ttt tgg ggt tgt gac tct tat gca aat tta aac aca gaa gat      528
Cys Ala Phe Trp Gly Cys Asp Ser Tyr Ala Asn Leu Asn Thr Glu Asp
                165                 170                 175

aac agc ctc cag gac cac agt gtg gca cag gag aaa ggt act gct gat      576
Asn Ser Leu Gln Asp His Ser Val Ala Gln Glu Lys Gly Thr Ala Asp
            180                 185                 190

gca gca aat gtc aca agc act ctt gaa aat gaa gaa cat agt caa ata      624
Ala Ala Asn Val Thr Ser Thr Leu Glu Asn Glu Glu His Ser Gln Ile

195                    200                    205

att atc cat tgt aca cct tca aca ggt gct ttt aag ccc tgt gaa tat          672
Ile Ile His Cys Thr Pro Ser Thr Gly Ala Phe Lys Pro Cys Glu Tyr
    210                 215                 220

tta ctg gga agc tgg atg att cgt ctt act gtg tgg ttc att ttc ttg          720
Leu Leu Gly Ser Trp Met Ile Arg Leu Thr Val Trp Phe Ile Phe Leu
225                 230                 235                 240

gtt gca tta ttt ttc aac ctg ctt gtt att tta aca aca ttt gca tct          768
Val Ala Leu Phe Phe Asn Leu Leu Val Ile Leu Thr Thr Phe Ala Ser
                245                 250                 255

tgt aca tca ctg cct tcg tcc aaa ttg ttt ata                              801
Cys Thr Ser Leu Pro Ser Ser Lys Leu Phe Ile
            260                 265

<210>  39
<211>  267
<212>  PRT
<213>  Artificial

<220>
<223>  synthetic peptide

<400>  39

Gln Phe Pro Asn Leu Thr Gly Thr Val His Leu Glu Ser Leu Thr Leu
1               5                   10                  15

Thr Gly Thr Lys Ile Ser Ser Ile Pro Asn Asn Leu Cys Gln Glu Gln
            20                  25                  30

Lys Met Leu Arg Thr Leu Asp Leu Ser Tyr Asn Asn Ile Arg Asp Leu
        35                  40                  45

Pro Ser Phe Asn Gly Cys His Ala Leu Glu Glu Ile Ser Leu Gln Arg
    50                  55                  60

Asn Gln Ile Tyr Gln Ile Lys Glu Gly Thr Phe Gln Gly Leu Ile Ser
65                  70                  75                  80

Leu Arg Ile Leu Asp Leu Ser Arg Asn Leu Ile His Glu Ile His Ser
                85                  90                  95

Arg Ala Phe Ala Thr Leu Gly Pro Ile Thr Asn Leu Asp Val Ser Phe
            100                 105                 110

Asn Glu Leu Thr Ser Phe Pro Thr Glu Gly Leu Asn Gly Leu Asn Gln
        115                 120                 125

Leu Lys Leu Val Gly Asn Phe Lys Leu Lys Glu Ala Leu Ala Ala Lys
    130                 135                 140

Asp Phe Val Asn Leu Arg Ser Leu Ser Val Pro Tyr Ala Tyr Gln Cys
145                 150                 155                 160

Cys Ala Phe Trp Gly Cys Asp Ser Tyr Ala Asn Leu Asn Thr Glu Asp
                165                 170                 175

Asn Ser Leu Gln Asp His Ser Val Ala Gln Glu Lys Gly Thr Ala Asp
            180                 185                 190

Ala Ala Asn Val Thr Ser Thr Leu Glu Asn Glu Glu His Ser Gln Ile
        195                 200                 205

Ile Ile His Cys Thr Pro Ser Thr Gly Ala Phe Lys Pro Cys Glu Tyr
    210                 215                 220

Leu Leu Gly Ser Trp Met Ile Arg Leu Thr Val Trp Phe Ile Phe Leu
225                 230                 235                 240

Val Ala Leu Phe Phe Asn Leu Leu Val Ile Leu Thr Thr Phe Ala Ser
                245                 250                 255

Cys Thr Ser Leu Pro Ser Ser Lys Leu Phe Ile
            260                 265

<210>  40

<211> 735
<212> DNA
<213> Artificial

<220>
<223> synthetic DNA

<220>
<221> CDS
<222> (1)..(735)

<400> 40
act gaa ttt cct gat tta act gga act gca aac ctg gag agt ctg act 48
Thr Glu Phe Pro Asp Leu Thr Gly Thr Ala Asn Leu Glu Ser Leu Thr
1               5                   10                  15

tta act gga gca cag atc tca tct ctt cct caa acc gtc tgc aat cag 96
Leu Thr Gly Ala Gln Ile Ser Ser Leu Pro Gln Thr Val Cys Asn Gln
            20                  25                  30

tta cct aat ctc caa gtg cta gat ctg tct tac aac cta tta gaa gat 144
Leu Pro Asn Leu Gln Val Leu Asp Leu Ser Tyr Asn Leu Leu Glu Asp
        35                  40                  45

tta ccc agt ttt tca gtc tgc caa aag ctt cag aaa att gac cta aga 192
Leu Pro Ser Phe Ser Val Cys Gln Lys Leu Gln Lys Ile Asp Leu Arg
    50                  55                  60

cat aat gaa atc tac gaa att aaa gtt gac act ttc cag cag ttg ctt 240
His Asn Glu Ile Tyr Glu Ile Lys Val Asp Thr Phe Gln Gln Leu Leu
65                  70                  75                  80

agc ctc cga tcg ctg aat ttg gct tgg aac aaa att gct att att cac 288
Ser Leu Arg Ser Leu Asn Leu Ala Trp Asn Lys Ile Ala Ile Ile His
                85                  90                  95

ccc aat gca ttt tcc act ttg cca tcc cta ata aag ctg gac cta tcg 336
Pro Asn Ala Phe Ser Thr Leu Pro Ser Leu Ile Lys Leu Asp Leu Ser
            100                 105                 110

tcc aac ctc ctg tcg tct ttt cct ata act ggg tta cat ggt tta act 384
Ser Asn Leu Leu Ser Ser Phe Pro Ile Thr Gly Leu His Gly Leu Thr
        115                 120                 125

cac tta aaa tta aca gga aat cat gcc tta cag agc ttg ata tca tct 432
His Leu Lys Leu Thr Gly Asn His Ala Leu Gln Ser Leu Ile Ser Ser
    130                 135                 140

gaa aac ttt cca gaa ctc aag gtt ata gaa atg cct tat gct tac cag     480
Glu Asn Phe Pro Glu Leu Lys Val Ile Glu Met Pro Tyr Ala Tyr Gln
145                 150                 155                 160

tgc tgt gca ttt gga gtg tgt gag aat gcc tat aag att tct aat caa     528
Cys Cys Ala Phe Gly Val Cys Glu Asn Ala Tyr Lys Ile Ser Asn Gln
                165                 170                 175

tgg aat aaa ggt gac aac agc agt atg gac gac ctt cat aag aaa gat     576
Trp Asn Lys Gly Asp Asn Ser Ser Met Asp Asp Leu His Lys Lys Asp
            180                 185                 190

gct gga atg ttt cag gct caa gat gaa cgt gac ctt gaa gat ttc ctg     624
Ala Gly Met Phe Gln Ala Gln Asp Glu Arg Asp Leu Glu Asp Phe Leu
        195                 200                 205

ctt gac ttt gag gaa gac ctg aaa gcc ctt cat tca gtg cag tgt tca     672
Leu Asp Phe Glu Glu Asp Leu Lys Ala Leu His Ser Val Gln Cys Ser
    210                 215                 220

cct tcc cca ggc ccc ttc aaa ccc tgt gaa cac ctg ctt gat ggc tgg     720
Pro Ser Pro Gly Pro Phe Lys Pro Cys Glu His Leu Leu Asp Gly Trp
225                 230                 235                 240

ctg atc aga att gga                                                 735
Leu Ile Arg Ile Gly
                245

<210>  41
<211>  245
<212>  PRT
<213>  Artificial

<220>
<223>  synthetic peptide

<400>  41

Thr Glu Phe Pro Asp Leu Thr Gly Thr Ala Asn Leu Glu Ser Leu Thr
1               5                   10                  15

Leu Thr Gly Ala Gln Ile Ser Ser Leu Pro Gln Thr Val Cys Asn Gln
            20                  25                  30

Leu Pro Asn Leu Gln Val Leu Asp Leu Ser Tyr Asn Leu Leu Glu Asp
        35                  40                  45

Leu Pro Ser Phe Ser Val Cys Gln Lys Leu Gln Lys Ile Asp Leu Arg
    50                  55                  60

His Asn Glu Ile Tyr Glu Ile Lys Val Asp Thr Phe Gln Gln Leu Leu
65                  70                  75                  80

Ser Leu Arg Ser Leu Asn Leu Ala Trp Asn Lys Ile Ala Ile Ile His
                85                  90                  95

Pro Asn Ala Phe Ser Thr Leu Pro Ser Leu Ile Lys Leu Asp Leu Ser
            100                 105                 110

Ser Asn Leu Leu Ser Ser Phe Pro Ile Thr Gly Leu His Gly Leu Thr
        115                 120                 125

His Leu Lys Leu Thr Gly Asn His Ala Leu Gln Ser Leu Ile Ser Ser
    130                 135                 140

Glu Asn Phe Pro Glu Leu Lys Val Ile Glu Met Pro Tyr Ala Tyr Gln
145                 150                 155                 160

Cys Cys Ala Phe Gly Val Cys Glu Asn Ala Tyr Lys Ile Ser Asn Gln
                165                 170                 175

Trp Asn Lys Gly Asp Asn Ser Ser Met Asp Asp Leu His Lys Lys Asp
            180                 185                 190

Ala Gly Met Phe Gln Ala Gln Asp Glu Arg Asp Leu Glu Asp Phe Leu
        195                 200                 205

Leu Asp Phe Glu Glu Asp Leu Lys Ala Leu His Ser Val Gln Cys Ser
    210                 215                 220

Pro Ser Pro Gly Pro Phe Lys Pro Cys Glu His Leu Leu Asp Gly Trp
225                 230                 235                 240

Leu Ile Arg Ile Gly
                245

<210>  42
<211>  30
<212>  DNA
<213>  Artificial

<220>
<223>  synthetic DNA

<400>  42
cgtgaattcg cagcagttcc ccaatcttac                                        30

<210>  43
<211>  30
<212>  DNA
<213>  Artificial

<220>
<223>  synthetic DNA

<400>  43
cgcaaagctt agtaagacga atcatccagc                                        30